# EUROPEAN PATENT APPLICATION

(11) **EP 3 835 787 A1**
(43) Date of publication of application: **16.06.2021**
(21) Application number: 20207809.3
(22) Date of filing: 16.02.2017
(51) Int. Cl.: G01N 33/564

(54) **ANTI-CXC CHEMOKINE RECEPTOR ANTIBODIES AND C-X-C CHEMOKINES FOR THE PROGNOSIS OF ACUTE GRAFT-VERSUS-HOST DISEASE**

(30) Priority: 16.02.2016 EP 16156011; 07.10.2016 EP 16192784
(62) Divisional of application: 17705868.2
(71) Applicant: CellTrend GmbH, 14943 Luckenwalde (DE); Ruprecht-Karls-Universität Heidelberg, 69117 Heidelberg (DE)
(72) Inventor: HEIDECKE, Harald, 14169 Berlin (DE); SCHULZE-FORSTER, Kai, 12207 Berlin (DE); LUFT, Thomas, 69126 Heidelberg (DE)
(74) Representative: Vossius & Partner Patentanwälte Rechtsanwälte mbB

(57) **Abstract**

The invention relates to the use of (auto)antibodies against the C-X-C chemokine receptor type 3 (CXCR3) as a prognostic marker in subjects with acute graft-versus-host disease (GVHD), particularly in subjects with high grade acute GVHD. The invention also relates to the use of C-X-C chemokines selected from the group consisting of CXCL4, CXCL9, CXCL10 and CXCL11, preferably CXCL9, as a prognostic marker in subjects with acute GVHD, particularly in subjects with high grade acute GVHD

SEQUENCE LISTING

<110> CellTrend GmbH
      Ruprecht-Karls-Universitaet Heidelberg

<120> Anti-CXC chemokine receptor antibodies and C-X-C chemokines for the
diagnosis of autoimmune disease and graft-versus-host disease

<130> X3319 EP/1 BLN

<150> EP 17 70 5868.2
<151> 2017-02-16

<150> PCT/EP2017/053524
<151> 2017-02-16

<150> EP 16 19 2784.3
<151> 2016-10-07

<150> EP 16 15 6011.5
<151> 2016-02-16

<160> 11

<170> BiSSAP 1.3

<210> 1
<211> 368
<212> PRT
<213> Homo sapiens

<220>
<223> CXCR3-A (also referred to as CXCR3 isoform 1) (NCBI-Database as
      of February 13, 2015: NP_001136269)

<400> 1
Met Val Leu Glu Val Ser Asp His Gln Val Leu Asn Asp Ala Glu Val
1               5                   10                  15
Ala Ala Leu Leu Glu Asn Phe Ser Ser Ser Tyr Asp Tyr Gly Glu Asn
            20                  25                  30
Glu Ser Asp Ser Cys Cys Thr Ser Pro Pro Cys Pro Gln Asp Phe Ser
        35                  40                  45
Leu Asn Phe Asp Arg Ala Phe Leu Pro Ala Leu Tyr Ser Leu Leu Phe
    50                  55                  60
Leu Leu Gly Leu Leu Gly Asn Gly Ala Val Ala Ala Val Leu Leu Ser
65                  70                  75                  80
Arg Arg Thr Ala Leu Ser Ser Thr Asp Thr Phe Leu Leu His Leu Ala
                85                  90                  95
Val Ala Asp Thr Leu Leu Val Leu Thr Leu Pro Leu Trp Ala Val Asp
            100                 105                 110
Ala Ala Val Gln Trp Val Phe Gly Ser Gly Leu Cys Lys Val Ala Gly
        115                 120                 125
Ala Leu Phe Asn Ile Asn Phe Tyr Ala Gly Ala Leu Leu Leu Ala Cys
    130                 135                 140
Ile Ser Phe Asp Arg Tyr Leu Asn Ile Val His Ala Thr Gln Leu Tyr
145                 150                 155                 160
Arg Arg Gly Pro Pro Ala Arg Val Thr Leu Thr Cys Leu Ala Val Trp
                165                 170                 175
Gly Leu Cys Leu Leu Phe Ala Leu Pro Asp Phe Ile Phe Leu Ser Ala
            180                 185                 190
His His Asp Glu Arg Leu Asn Ala Thr His Cys Gln Tyr Asn Phe Pro
        195                 200                 205
Gln Val Gly Arg Thr Ala Leu Arg Val Leu Gln Leu Val Ala Gly Phe

210                 215                 220
Leu Leu Pro Leu Leu Val Met Ala Tyr Cys Tyr Ala His Ile Leu Ala
225                 230                 235                 240
Val Leu Leu Val Ser Arg Gly Gln Arg Arg Leu Arg Ala Met Arg Leu
                245                 250                 255
Val Val Val Val Val Val Ala Phe Ala Leu Cys Trp Thr Pro Tyr His
            260                 265                 270
Leu Val Val Leu Val Asp Ile Leu Met Asp Leu Gly Ala Leu Ala Arg
        275                 280                 285
Asn Cys Gly Arg Glu Ser Arg Val Asp Val Ala Lys Ser Val Thr Ser
    290                 295                 300
Gly Leu Gly Tyr Met His Cys Cys Leu Asn Pro Leu Leu Tyr Ala Phe
305                 310                 315                 320
Val Gly Val Lys Phe Arg Glu Arg Met Trp Met Leu Leu Leu Arg Leu
                325                 330                 335
Gly Cys Pro Asn Gln Arg Gly Leu Gln Arg Gln Pro Ser Ser Ser Arg
            340                 345                 350
Arg Asp Ser Ser Trp Ser Glu Thr Ser Glu Ala Ser Tyr Ser Gly Leu
        355                 360                 365

<210> 2
<211> 415
<212> PRT
<213> Homo sapiens

<220>
<223> CXCR3-B (also referred to as CXCR3 isoform 2) (NCBI-Database as
      of February 13, 2015: NP_001495)

<400> 2
Met Glu Leu Arg Lys Tyr Gly Pro Gly Arg Leu Ala Gly Thr Val Ile
1               5                   10                  15
Gly Gly Ala Ala Gln Ser Lys Ser Gln Thr Lys Ser Asp Ser Ile Thr
            20                  25                  30
Lys Glu Phe Leu Pro Gly Leu Tyr Thr Ala Pro Ser Ser Pro Phe Pro
        35                  40                  45
Pro Ser Gln Val Ser Asp His Gln Val Leu Asn Asp Ala Glu Val Ala
    50                  55                  60
Ala Leu Leu Glu Asn Phe Ser Ser Ser Tyr Asp Tyr Gly Glu Asn Glu
65                  70                  75                  80
Ser Asp Ser Cys Cys Thr Ser Pro Pro Cys Pro Gln Asp Phe Ser Leu
                85                  90                  95
Asn Phe Asp Arg Ala Phe Leu Pro Ala Leu Tyr Ser Leu Leu Phe Leu
            100                 105                 110
Leu Gly Leu Leu Gly Asn Gly Ala Val Ala Ala Val Leu Leu Ser Arg
        115                 120                 125
Arg Thr Ala Leu Ser Ser Thr Asp Thr Phe Leu Leu His Leu Ala Val
    130                 135                 140
Ala Asp Thr Leu Leu Val Leu Thr Leu Pro Leu Trp Ala Val Asp Ala
145                 150                 155                 160
Ala Val Gln Trp Val Phe Gly Ser Gly Leu Cys Lys Val Ala Gly Ala
                165                 170                 175
Leu Phe Asn Ile Asn Phe Tyr Ala Gly Ala Leu Leu Leu Ala Cys Ile
            180                 185                 190
Ser Phe Asp Arg Tyr Leu Asn Ile Val His Ala Thr Gln Leu Tyr Arg
        195                 200                 205
Arg Gly Pro Pro Ala Arg Val Thr Leu Thr Cys Leu Ala Val Trp Gly
    210                 215                 220
Leu Cys Leu Leu Phe Ala Leu Pro Asp Phe Ile Phe Leu Ser Ala His
225                 230                 235                 240
His Asp Glu Arg Leu Asn Ala Thr His Cys Gln Tyr Asn Phe Pro Gln

245                 250                 255
Val Gly Arg Thr Ala Leu Arg Val Leu Gln Leu Val Ala Gly Phe Leu
            260                 265                 270
Leu Pro Leu Leu Val Met Ala Tyr Cys Tyr Ala His Ile Leu Ala Val
        275                 280                 285
Leu Leu Val Ser Arg Gly Gln Arg Arg Leu Arg Ala Met Arg Leu Val
    290                 295                 300
Val Val Val Val Val Ala Phe Ala Leu Cys Trp Thr Pro Tyr His Leu
305                 310                 315                 320
Val Val Leu Val Asp Ile Leu Met Asp Leu Gly Ala Leu Ala Arg Asn
                325                 330                 335
Cys Gly Arg Glu Ser Arg Val Asp Val Ala Lys Ser Val Thr Ser Gly
            340                 345                 350
Leu Gly Tyr Met His Cys Cys Leu Asn Pro Leu Leu Tyr Ala Phe Val
        355                 360                 365
Gly Val Lys Phe Arg Glu Arg Met Trp Met Leu Leu Leu Arg Leu Gly
    370                 375                 380
Cys Pro Asn Gln Arg Gly Leu Gln Arg Gln Pro Ser Ser Ser Arg Arg
385                 390                 395                 400
Asp Ser Ser Trp Ser Glu Thr Ser Glu Ala Ser Tyr Ser Gly Leu
                405                 410                 415

<210> 3
<211> 356
<212> PRT
<213> Homo sapiens

<220>
<223> CXCR4a (isoform a; NCBI-Database as of February 13, 2015:
      NP_001008540)

<400> 3
Met Ser Ile Pro Leu Pro Leu Leu Gln Ile Tyr Thr Ser Asp Asn Tyr
1               5                   10                  15
Thr Glu Glu Met Gly Ser Gly Asp Tyr Asp Ser Met Lys Glu Pro Cys
            20                  25                  30
Phe Arg Glu Glu Asn Ala Asn Phe Asn Lys Ile Phe Leu Pro Thr Ile
        35                  40                  45
Tyr Ser Ile Ile Phe Leu Thr Gly Ile Val Gly Asn Gly Leu Val Ile
    50                  55                  60
Leu Val Met Gly Tyr Gln Lys Lys Leu Arg Ser Met Thr Asp Lys Tyr
65                  70                  75                  80
Arg Leu His Leu Ser Val Ala Asp Leu Leu Phe Val Ile Thr Leu Pro
                85                  90                  95
Phe Trp Ala Val Asp Ala Val Ala Asn Trp Tyr Phe Gly Asn Phe Leu
            100                 105                 110
Cys Lys Ala Val His Val Ile Tyr Thr Val Asn Leu Tyr Ser Ser Val
        115                 120                 125
Leu Ile Leu Ala Phe Ile Ser Leu Asp Arg Tyr Leu Ala Ile Val His
    130                 135                 140
Ala Thr Asn Ser Gln Arg Pro Arg Lys Leu Leu Ala Glu Lys Val Val
145                 150                 155                 160
Tyr Val Gly Val Trp Ile Pro Ala Leu Leu Leu Thr Ile Pro Asp Phe
                165                 170                 175
Ile Phe Ala Asn Val Ser Glu Ala Asp Asp Arg Tyr Ile Cys Asp Arg
            180                 185                 190
Phe Tyr Pro Asn Asp Leu Trp Val Val Val Phe Gln Phe Gln His Ile
        195                 200                 205
Met Val Gly Leu Ile Leu Pro Gly Ile Val Ile Leu Ser Cys Tyr Cys
    210                 215                 220
Ile Ile Ile Ser Lys Leu Ser His Ser Lys Gly His Gln Lys Arg Lys
225                 230                 235                 240
Ala Leu Lys Thr Thr Val Ile Leu Ile Leu Ala Phe Phe Ala Cys Trp

245                 250                 255
Leu Pro Tyr Tyr Ile Gly Ile Ser Ile Asp Ser Phe Ile Leu Leu Glu
            260                 265                 270
Ile Ile Lys Gln Gly Cys Glu Phe Glu Asn Thr Val His Lys Trp Ile
        275                 280                 285
Ser Ile Thr Glu Ala Leu Ala Phe Phe His Cys Cys Leu Asn Pro Ile
    290                 295                 300
Leu Tyr Ala Phe Leu Gly Ala Lys Phe Lys Thr Ser Ala Gln His Ala
305                 310                 315                 320
Leu Thr Ser Val Ser Arg Gly Ser Ser Leu Lys Ile Leu Ser Lys Gly
                325                 330                 335
Lys Arg Gly Gly His Ser Ser Val Ser Thr Glu Ser Glu Ser Ser Ser
            340                 345                 350
Phe His Ser Ser
        355

<210> 4
<211> 352
<212> PRT
<213> Homo sapiens

<220>
<223> CXCR4b (isoform b; NCBI-Database as of January 5, 2016:
      NP_003458)

<400> 4
Met Glu Gly Ile Ser Ile Tyr Thr Ser Asp Asn Tyr Thr Glu Glu Met
1               5                   10                  15
Gly Ser Gly Asp Tyr Asp Ser Met Lys Glu Pro Cys Phe Arg Glu Glu
            20                  25                  30
Asn Ala Asn Phe Asn Lys Ile Phe Leu Pro Thr Ile Tyr Ser Ile Ile
        35                  40                  45
Phe Leu Thr Gly Ile Val Gly Asn Gly Leu Val Ile Leu Val Met Gly
    50                  55                  60
Tyr Gln Lys Lys Leu Arg Ser Met Thr Asp Lys Tyr Arg Leu His Leu
65                  70                  75                  80
Ser Val Ala Asp Leu Leu Phe Val Ile Thr Leu Pro Phe Trp Ala Val
                85                  90                  95
Asp Ala Val Ala Asn Trp Tyr Phe Gly Asn Phe Leu Cys Lys Ala Val
            100                 105                 110
His Val Ile Tyr Thr Val Asn Leu Tyr Ser Ser Val Leu Ile Leu Ala
        115                 120                 125
Phe Ile Ser Leu Asp Arg Tyr Leu Ala Ile Val His Ala Thr Asn Ser
    130                 135                 140
Gln Arg Pro Arg Lys Leu Leu Ala Glu Lys Val Val Tyr Val Gly Val
145                 150                 155                 160
Trp Ile Pro Ala Leu Leu Leu Thr Ile Pro Asp Phe Ile Phe Ala Asn
                165                 170                 175
Val Ser Glu Ala Asp Asp Arg Tyr Ile Cys Asp Arg Phe Tyr Pro Asn
            180                 185                 190
Asp Leu Trp Val Val Val Phe Gln Phe Gln His Ile Met Val Gly Leu
        195                 200                 205
Ile Leu Pro Gly Ile Val Ile Leu Ser Cys Tyr Cys Ile Ile Ile Ser
    210                 215                 220
Lys Leu Ser His Ser Lys Gly His Gln Lys Arg Lys Ala Leu Lys Thr
225                 230                 235                 240
Thr Val Ile Leu Ile Leu Ala Phe Phe Ala Cys Trp Leu Pro Tyr Tyr
                245                 250                 255
Ile Gly Ile Ser Ile Asp Ser Phe Ile Leu Leu Glu Ile Ile Lys Gln
            260                 265                 270
Gly Cys Glu Phe Glu Asn Thr Val His Lys Trp Ile Ser Ile Thr Glu
        275                 280                 285
Ala Leu Ala Phe Phe His Cys Cys Leu Asn Pro Ile Leu Tyr Ala Phe

290                 295                 300
Leu Gly Ala Lys Phe Lys Thr Ser Ala Gln His Ala Leu Thr Ser Val
305                 310                 315                 320
Ser Arg Gly Ser Ser Leu Lys Ile Leu Ser Lys Gly Lys Arg Gly Gly
                325                 330                 335
His Ser Ser Val Ser Thr Glu Ser Glu Ser Ser Ser Phe His Ser Ser
            340                 345                 350

<210> 5
<211> 100
<212> PRT
<213> Homo sapiens

<220>
<223> preferred antigenic fragment of CXCR3-A (SEQ ID NO:1)

<400> 5
Ser Gly Leu Cys Lys Val Ala Gly Ala Leu Phe Asn Ile Asn Phe Tyr
1               5                   10                  15
Ala Gly Ala Leu Leu Leu Ala Cys Ile Ser Phe Asp Arg Tyr Leu Asn
            20                  25                  30
Ile Val His Ala Thr Gln Leu Tyr Arg Arg Gly Pro Pro Ala Arg Val
        35                  40                  45
Thr Leu Thr Cys Leu Ala Val Asx Gly Leu Cys Leu Leu Phe Ala Leu
    50                  55                  60
Pro Asp Phe Ile Phe Leu Ser Ala His His Asp Glu Arg Leu Asn Ala
65                  70                  75                  80
Thr His Cys Gln Tyr Asn Phe Pro Gln Val Gly Arg Thr Ala Leu Arg
                85                  90                  95
Val Leu Gln Leu
            100

<210> 6
<211> 46
<212> PRT
<213> Homo sapiens

<220>
<223> preferred antigenic fragment of CXCR4b (SEQ ID NO:4)

<400> 6
Met Glu Gly Ile Ser Ile Tyr Thr Ser Asp Asn Tyr Thr Glu Glu Met
1               5                   10                  15
Gly Ser Gly Asp Tyr Asp Ser Met Lys Glu Pro Cys Phe Arg Glu Glu
            20                  25                  30
Asn Ala Asn Phe Asn Lys Ile Phe Leu Pro Thr Ile Tyr Ser
        35                  40                  45

<210> 7
<211> 101
<212> PRT
<213> Homo sapiens

<220>
<223> CXCL4

<400> 7
Met Ser Ser Ala Ala Gly Phe Cys Ala Ser Arg Pro Gly Leu Leu Phe
1               5                   10                  15
Leu Gly Leu Leu Leu Leu Pro Leu Val Val Ala Phe Ala Ser Ala Glu
            20                  25                  30

Ala Glu Glu Asp Gly Asp Leu Gln Cys Leu Cys Val Lys Thr Thr Ser
        35                  40                  45
Gln Val Arg Pro Arg His Ile Thr Ser Leu Glu Val Ile Lys Ala Gly
    50                  55                  60
Pro His Cys Pro Thr Ala Gln Leu Ile Ala Thr Leu Lys Asn Gly Arg
65                  70                  75                  80
Lys Ile Cys Leu Asp Leu Gln Ala Pro Leu Tyr Lys Lys Ile Ile Lys
                85                  90                  95
Lys Leu Leu Glu Ser
            100

<210> 8
<211> 125
<212> PRT
<213> Homo sapiens

<220>
<223> CXCL9

<400> 8
Met Lys Lys Ser Gly Val Leu Phe Leu Leu Gly Ile Ile Leu Leu Val
1               5                   10                  15
Leu Ile Gly Val Gln Gly Thr Pro Val Val Arg Lys Gly Arg Cys Ser
            20                  25                  30
Cys Ile Ser Thr Asn Gln Gly Thr Ile His Leu Gln Ser Leu Lys Asp
        35                  40                  45
Leu Lys Gln Phe Ala Pro Ser Pro Ser Cys Glu Lys Ile Glu Ile Ile
    50                  55                  60
Ala Thr Leu Lys Asn Gly Val Gln Thr Cys Leu Asn Pro Asp Ser Ala
65                  70                  75                  80
Asp Val Lys Glu Leu Ile Lys Lys Trp Glu Lys Gln Val Ser Gln Lys
                85                  90                  95
Lys Lys Gln Lys Asn Gly Lys Lys His Gln Lys Lys Lys Val Leu Lys
            100                 105                 110
Val Arg Lys Ser Gln Arg Ser Arg Gln Lys Lys Thr Thr
        115                 120                 125

<210> 9
<211> 98
<212> PRT
<213> Homo sapiens

<220>
<223> CXCL10

<400> 9
Met Asn Gln Thr Ala Ile Leu Ile Cys Cys Leu Ile Phe Leu Thr Leu
1               5                   10                  15
Ser Gly Ile Gln Gly Val Pro Leu Ser Arg Thr Val Arg Cys Thr Cys
            20                  25                  30
Ile Ser Ile Ser Asn Gln Pro Val Asn Pro Arg Ser Leu Glu Lys Leu
        35                  40                  45
Glu Ile Ile Pro Ala Ser Gln Phe Cys Pro Arg Val Glu Ile Ile Ala
    50                  55                  60
Thr Met Lys Lys Lys Gly Glu Lys Arg Cys Leu Asn Pro Glu Ser Lys
65                  70                  75                  80
Ala Ile Lys Asn Leu Leu Lys Ala Val Ser Lys Glu Arg Ser Lys Arg
                85                  90                  95
Ser Pro


<210> 10
<211> 94

<212> PRT
<213> Homo sapiens

<220>
<223> CXCL11 (isoform 1)

<400> 10
Met Ser Val Lys Gly Met Ala Ile Ala Leu Ala Val Ile Leu Cys Ala
1               5                   10                  15
Thr Val Val Gln Gly Phe Pro Met Phe Lys Arg Gly Arg Cys Leu Cys
            20                  25                  30
Ile Gly Pro Gly Val Lys Ala Val Lys Val Ala Asp Ile Glu Lys Ala
        35                  40                  45
Ser Ile Met Tyr Pro Ser Asn Asn Cys Asp Lys Ile Glu Val Ile Ile
    50                  55                  60
Thr Leu Lys Glu Asn Lys Gly Gln Arg Cys Leu Asn Pro Lys Ser Lys
65                  70                  75                  80
Gln Ala Arg Leu Ile Ile Lys Lys Val Glu Arg Lys Asn Phe
                85                  90

<210> 11
<211> 106
<212> PRT
<213> Homo sapiens

<220>
<223> CXCL11 (isoform 2)

<400> 11
Met Ser Val Lys Gly Met Ala Ile Ala Leu Ala Val Ile Leu Cys Ala
1               5                   10                  15
Thr Val Val Gln Gly Phe Pro Met Phe Lys Arg Gly Arg Cys Leu Cys
            20                  25                  30
Ile Gly Pro Gly Val Lys Ala Val Lys Val Ala Asp Ile Glu Lys Ala
        35                  40                  45
Ser Ile Met Tyr Pro Ser Asn Asn Cys Asp Lys Ile Glu Val Ile Ile
    50                  55                  60
Thr Leu Lys Glu Asn Lys Gly Gln Arg Cys Leu Asn Pro Lys Ser Lys
65                  70                  75                  80
Gln Ala Arg Leu Ile Ile Lys Leu Lys Glu Arg Ile Phe Lys Asn Ile
                85                  90                  95
Lys Thr Tyr Glu Val Leu Glu Lys Ser Ile
            100                 105

## Description

### Field of the Invention

The present invention relates to the field of medicine, particularly to diagnosis. More particularly the present invention relates to the diagnosis of an autoimmune disease, preferably a connective tissue autoimmune disease using the detection of autoimmune antibodies directed against a CXC chemokine receptor. The present invention further relates to the prognosis for patients with acute graft-versus-host disease (GVHD) after allogeneic hematopoietic stem cell transplantation (HSCT).

### Background of the Invention

Autoimmune diseases arise from an abnormal immune response of the body against substances and tissues normally present in the body (autoimmunity). This may be restricted to certain organs (e.g. in autoimmune thyroiditis) or involve a particular tissue in different places (e.g. Goodpasture's disease which may affect the basement membrane in both the lung and the kidney). The treatment of autoimmune diseases is typically with immunosuppression-medication that decreases the immune response (Cotsapas C, Hafler DA (2013). "Immune-mediated disease genetics: the shared basis of pathogenesis". Trends in Immunology 34 (1): 22-26). A large number of autoimmune diseases are recognized. A major understanding of the underlying pathophysiology of autoimmune diseases has been the application of genome wide association scans that have identified a striking degree of genetic sharing among the autoimmune diseases (Cotsapas C, Hafler DA (2013). "Immune-mediated disease genetics: the shared basis of pathogenesis". Trends in Immunology 34 (1): 22-26). For a disease to be regarded as an autoimmune disease it needs to answer to Witebsky's postulates (first formulated by Ernst Witebsky and colleagues in 1957 and modified in 1994) (Witebsky E, Rose NR, Terplan K, Paine JR, Egan RW (1957). "Chronic thyroiditis and autoimmunization". J. Am. Med. Assoc. 164 (13): 1439-47; and Rose NR, Bona C (September 1993). "Defining criteria for autoimmune diseases (Witebsky's postulates revisited)". Immunol. Today 14 (9): 426-30): 1. Direct evidence from transfer of pathogenic antibody or pathogenic T cells; 2. Indirect evidence based on reproduction of the autoimmune disease in experimental animals; 3. Circumstantial evidence from clinical clues, and 4. Genetic architecture clustering with other autoimmune diseases. It has been estimated that autoimmune diseases are among the top ten leading causes of death among women in all age groups up to 65 years (Walsh, SJ; Rau, LM (September 2000). "Autoimmune diseases: a leading cause of death among young and middle-aged women in the United States.". American journal of public health 90 (9): 1463-1466). A substantial part of the population suffers from these diseases, which are often chronic, debilitating, and life-threatening.

Autoimmune connective tissue diseases (CTDs) are also referred to as systemic autoimmune diseases. The autoimmune CTDs may have both genetic and environmental causes. Genetic factors may create a predisposition towards developing these autoimmune diseases. They are characterized as a group by the presence of spontaneous over-activity of the immune system that results in the production of extra antibodies into the circulation. The classic collagen vascular diseases have a "classic" presentation with typical findings that doctors can recognize during an examination. Each also has "classic" blood test abnormalities and abnormal antibody patterns. However, each of these diseases can evolve slowly or rapidly from very subtile abnormalities before demonstrating the classic features that help in the diagnosis. The classic collagen vascular diseases include systemic lupus erythematosus (SLE), rheumatoid arthritis, xcleroderma, Sjögren's syndrome, mixed connective tissue disease, systemic sclerosis (SSc); dermatomyositis (DM), polymyositis (PM), anti-synthetase syndrome, and psoriatic arthritis.

Rheumatoid arthritis is a systemic disorder in which immune cells attack and inflame the membrane around joints. It also can affect the heart, lungs, and eyes. Of the estimated 2.1 million Americans with rheumatoid arthritis, approximately 1.5 million (71 percent) are women.

Scleroderma refers to an activation of immune cells that produces scar tissue in the skin, internal organs, and small blood vessels. It affects women three times more often than men overall, but increases to a rate 15 times greater for women during childbearing years, and appears to be more common among black women.

Sjögren's syndrome, also called Sjögren's disease, is a chronic, slowly progressing inability to secrete saliva and tears. It can occur alone or in combination with rheumatoid arthritis, scleroderma, or systemic lupus erythematosus. Nine out of 10 cases occur in women, most often at or around mid-life.

Mixed connective-tissue disease (MCTD) is a disorder in which features of various connective-tissue diseases (CTDs) such as systemic lupus erythematosus (SLE); systemic sclerosis (SSc); dermatomyositis (DM); polymyositis (PM); anti-synthetase syndrome; and, occasionally, Sjögren syndrome can coexist and overlap. The course of the disease is chronic and usually milder than other CTDs. In most cases, MCTD is considered an intermediate stage of a disease that eventually becomes either SLE or Scleroderma.

Systemic lupus erythematosus, also known as SLE or lupus, is a systemic autoimmune disease (or autoimmune connective tissue disease (collagenosis)) in which the body's immune system mistakenly attacks healthy tissue. When the immune system is functioning normally, it produces antibodies that protect against pathogens such as viruses and bacteria. Lupus is characterized by the presence of antibodies against a person's own proteins; these are most commonly anti-nuclear antibodies, which are routinely used for diagnosis. These antibodies lead to inflammation. Although the underlying cause of autoimmune diseases is unknown, most believe that lupus results from both genetic and environmental stimuli. SLE is an inflammation of the connective tissues, SLE can afflict every organ system. It is up to nine times more common in women than men and strikes black women three times as often as white women. The condition is aggravated by sunlight.

There are several kinds of lupus. The most common type is systemic lupus erythematosus (SLE) affecting many internal organs in the body. SLE most often harms the heart, joints, skin, lungs, blood vessels, liver, kidneys, and nervous system. The course of the disease is unpredictable, with periods of illness (called flares or active disease) alternating with remissions (inactive disease). The disease occurs nine times more often in women than in men, especially in women in child-bearing years ages 15 to 35, and is also more common in those of non-European descent ("Harrison's Internal Medicine, 17th ed. Chapter 313. Systemic Lupus Erythematosus"; and Anisur Rahman and David A. Isenberg (February 28, 2008). "Review Article: Systemic Lupus Erythematosus". N Engl J Med 358 (9): 929-939). The ANA is the most sensitive screening test for evaluation, whereas anti-Sm (anti-Smith) is the most specific. The dsDNA (double-stranded DNA) antibody is also fairly specific and often fluctuates with disease activity; as such, the dsDNA titre is sometimes useful to monitor disease flares or response to treatment. Until now there is no common diagnostic test providing good sensitivity and specificity.

While there is no cure for SLE, it is treated with immunosuppression, mainly with cyclophosphamide, corticosteroids and other immunosuppressants. The goal of these treatments is to keep symptoms under control. SLE can be fatal. The leading cause of death is from cardiovascular disease due to accelerated atherosclerosis. Survival for people with SLE in the United States, Canada, and Europe has risen to approximately 95% at five years, 90% at 10 years, and 78% at 20 years.

Even though being clinically well characterized, there is a need for an improved diagnosis of lupus. Furthermore, there is a need to differentiate between active disease stadium and inactive disease stadium, e.g. to judge whether a immunosuppressant is to be given or not.

Acute GVHD is a complication of allogeneic stem cell transplantation (alloSCT) occurring in up to 40% of patients. It targets skin, liver and bowels, and in most cases it responds to escalation of immunosuppressive therapy.

However, severe acute GVHD of the intestines have a propensity to progress despite of the application of high dose steroids and other immunosuppressive drugs. This "refractory GVHD" has an extremely high mortality rate and stands for a major proportion of non-relapse mortality after alloSCT.

Evidence exists that a major variable defining risk of death from acute GVHD is endothelial cell distress. This complication can be predicted already prior to allogeneic stem cell transplantation (named endothelial vulnerability). Concomitant statin prophylaxis appears to reduce the risk of endothelial complications after allogeneic stem cell transplantation in the cohort of patients with endothelial vulnerability markers. However, a significant proportion of patients die from acute GVHD without a noticeable endothelial vulnerability. Similarly, refractory acute GVHD and death from acute GVHD remains a major problem even in patients taking statins. This suggests that a second mechanism must exist leading to non-response of GVHD patients to immunosuppressive therapy.

### Summary of the Invention

The invention relates to a method for diagnosis of an autoimmune disease in a subject wherein, presence or absence of an anti-CXC chemokine receptor (CXCR) antibody is detected in a sample from the subject diagnosed, and wherein the presence of an anti-CXCR antibody is indicative of the disease.

Absence and presence may be determined relative to a control. In such an embodiment the method may comprise the determination of the level of anti-CXCR antibodies in a sample of the subject to be diagnosed and comparing the determined level to a control level. The inventors interestingly found that the presence or increased levels of CXCR antibodies correlate with the presence of an autoimmune disease in a subject. In case the level is compared to a control, an increase of CXCR antibody level as compared to the control level denotes presence of a CXCR antibody in the sample and hence is indicative of the disease to be diagnosed, i.e. an autoimmune disease. Hence, in one embodiment of the method for diagnosis an autoimmune disease the present invention the presence or absence of the anti-CXCR antibody is detected by the following steps: (i) determining the level of anti-CXCR antibodies in a sample of the subject to be diagnosed; and (ii) comparing the determined level to an anti-CXCR antibody control level derived from a subject without an autoimmune disease; wherein an increased level of anti-CXCR antibody in the sample of the subject to be diagnosed as compared to the anti-CXCR antibody control level denotes the presence of an anti-CXCR antibody. The skilled person will acknowledge that when performing the method of the present invention the presence and absence may be visualized only when the sample contains the desired antibodies above a certain value, e.g. the control level, depending on the used assay.

When using an immunoassay like an ELISA the immunoassay may be adjusted so that the assay shows a "positive" result indicating the presence of the antibody only when the level is above the threshold, e.g. the control level. Such modifications regarding the sensibility of an immunoassay are known to the skilled person and are outlined herein.

Furthermore, the inventors unexpectedly found that the level of anti-CXCR antibodies in samples of a subject correlates with the degree of disease activity of an autoimmune disease in said subject, i.e. the higher the level of anti-CXCR antibodies in samples of a subject, the more active the autoimmune disease is in said subject. Even though subdivisions of different activity levels of autoimmune diseases may be desirable, it is preferred to differentiate between inactive and active autoimmune diseases, as for example medication is often indicated only in case the disease is active. Hence, the present invention also relates to a method for determining disease activity of an autoimmune disease comprising the steps of: (i) determining the level of anti-CXCR antibodies in a sample from a subject to be diagnosed, (ii) comparing the determined level in the sample to either one or both of a first and second anti-CXCR antibody control level, a) wherein the first anti-CXCR antibody control level is derived from subjects suffering an inactive autoimmune disease; and b) wherein the second anti-CXCR antibody control level derived from subjects suffering an active autoimmune disease; wherein a increased level in the sample from the subject to be diagnosed as compared to the first anti-CXCR antibody control level and/or an equal level as compared to the second anti-CXCR antibody control level is indicative of the presence of an active autoimmune disease in the subject to be diagnosed, and wherein an decreased level in the sample from the subject to be diagnosed as compared to the second anti-CXCR antibody control level and/or an equal level as compared to the first anti-CXCR antibody control level is indicative of the presence of an inactive autoimmune disease.

The inventors for the first time show the presence of auto-antibodies directed against CXC chemokine receptors (CXCR). The furthermore provide for the first time an assay for detecting such antibodies in samples from a subject. Hence, the present invention also relates to an immunoassay method for detecting an anti-CXC chemokine receptor (CXCR) antibody in a sample from a subject, comprising the steps of (a) contacting the sample suspected of comprising an anti-CXCR antibody with a CXC chemokine receptor (CXCR) or an antigenic peptide fragment thereof under conditions allowing for the formation of a complex between the anti- CXCR antibody with CXCR or the peptide fragment thereof; (b) detecting the complex.

The inventors for the first time demonstrate the presence of anti-CXCR antibodies in samples of a subject and their diagnostic predictive value. Such antibodies can be detected using the receptors or immunogenic peptides thereof. The invention therefore relates to a kit for detecting an anti-CXCR antibody comprising a CXCR or an immunogenic peptide thereof.

Such CXCR or immunogenic peptides thereof may be part of a diagnostic kit. Hence, the also encompassed by the present invention is the use of a kit in for the diagnosis of an autoimmune disease, wherein the kit comprises a CXCR or an immunogenic peptide thereof. Likewise, the present invention also relates to the use of a CXCR or an immunogenic peptide thereof for the diagnosis of an autoimmune disease.

Further, the invention relates to an immunosuppressant for use in the treatment of an autoimmune disease in a subject, wherein the immunosuppressant is administered to the subject upon determination of the presence of an anti-CXCR antibod in a sample of said subject. Preferably presence of the antibodies was performed as outlined for the methods of diagnosis of an autoimmune disease or an active autoimmune disease according to the present invention. The disease is preferably systemic lupus erythematosus. The immunosuppressant being preferably selected from the group consisting of cyclophosphamides, glucocorticoids, and Rituximab.
In common approach for treatment of autoimmune diseases is the isolation of auto-antibodies from the blood of the patient suffering from the disease. To this end, blood is isolated from the subject, the auto-antibodies are removed from the isolated blood, and the isolated blood is reinjected into the patient. Such approach is also known as plasmapheresis. As the inventors for the first time showed the correlation of the presence of auto-antibodies directed against CXCR with the presence of an autoimmune disease in a subject, it is apparently plausible to the skilled person that the removal of the antibodies from the blood of the patient will ameliorate the disease. Hence, the invention also relates to a method for the removal of anti-CXCR antibodies from isolated blood, (i) wherein in a first step the presence or absence of an anti-CXCR antibody is determined in a blood sample from a subject to be diagnosed for an autoimmune disease; and (ii) wherein, upon determining the presence of an anti-CXCR antibody the antibody is removed from isolated blood of the subject. The blood may optionally been reinjected into the subject once the antibody are removed.

The invention in another aspect relates to the use of (auto)antibodies against the C-X-C chemokine receptor type 3 (CXCR3) as a prognostic marker in subjects with acute graft-versus-host disease (GVHD), particularly in subjects with high grade acute GVHD. The invention also relates to the use of C-X-C chemokines selected from the group consisting of CXCL4, CXCL9, CXCL10 and CXCL11, preferably CXCL9, as a prognostic marker in subjects with acute GVHD, particularly in subjects with high grade acute GVHD.

The inventors for the first time show that the level of these prognostic markers in samples of the subjects correlate with the outcome of the acute GVHD and therefore, inter alia, allow a more focused treatment of this disease and prevention of a severe outcome.

In particular, the present invention relates to a method for the prognosis of acute graft-versus host-disease (GVHD) in a subject comprising the determination of the level of an antibody against the C-X-C chemokine receptor type 3 (CXCR3) and/or the level of a C-X-C chemokine selected from the group consisting of CXCL4, CXCL9, CXCL10 and CXCL11, preferably CXCL9, in a sample of a bodily fluid of said subject.

The invention further pertains to a method for the prognosis of acute graft-versus host-disease (GVHD) in a subject, wherein said method comprises
(a)
   (i) determining the level of an antibody against the C-X-C chemokine receptor type 3 (CXCR3) in a sample of a bodily fluid of said subject and
   (ii) comparing the determined level of the anti-CXCR3 antibody to a control level of anti-CXCR3 antibody, and/or
(b)
   (i) determining the level of a C-X-C chemokine selected from the group consisting of CXCL4, CXCL9, CXCL10 and CXCL11, preferably CXCL9, in a sample of a bodily fluid of said subject and
   (ii) comparing the determined level of said C-X-C chemokine to a control level of said C-X-C chemokine,
wherein a decreased level of said anti-CXCR3 antibody with respect to the anti-CXCR3 antibody control level and/or an increased level of said C-X-C chemokine with respect to the C-X-C chemokine control level in said sample is indicative for an adverse outcome for said subject.

Accordingly, the present invention also relates to the use of CXCR3 or an immunogenic peptide thereof for the prognosis of acute GVHD, preferably high grade acute GVHD. The invention also pertains to the use of an anti-C-X-C chemokine antibody, preferably an anti-CXCL9 antibody, for the prognosis of acute GVHD, preferably high grade acute GVHD. The use of corresponding kits is also part of the invention.

In a further aspect, the present invention also relates to a method for treating acute GVHD, preferably high grade GVHD, in a subject, wherein the subject is treated upon detecting a decreased level of anti-CXCR3 antibody with respect to an anti-CXCR3 antibody control level and/or an increased level of a C-X-C chemokine, preferably CXCL9, with respect to a C-X-C chemokine control level, preferably a CXCL9 control level, in a sample of a bodily fluid of said subject. In this context, medical uses of immunosuppressants, C-X-C chemokine antagonists and/or CXCR3 antagonists, preferably anti-CXCR3 antibodies, for the treatment of subjects with acute GVHD with a predicted adverse outcome (according to the prognostic methods of the invention) are also part of the invention.

The methods of the invention are preferably immunoassays such as ELISAs and the like.

### Figure Legends

**Figure 1****:** Standard curve of anti-CXCR3 autoantibody ELISA. Details see Example 1.
**Figure 2****:** Standard curve of anti-CXCR4 autoantibody ELISA. Details see Example 1.
**Figure 3****:** Distribution of anti-CXCR3 antibodies in SLE patients compared to normal (healthy) donors (Normalspender) as box-plot (**A**) and dot-density plot (**B**).
**Figure 4****:** Distribution of anti-CXCR4 antibodies in SLE patients compared to normal (healthy) donors (Normalspender) as box-plot (**A**) and dot-density plot (**B**).
**Figure 5****:** Distribution of antibodies in SLE collective with differentiation of BILAG2004-categories compared with normal (healthy) donors (NS) figured as boxplot for anti-CXCR3 antibodies (**A**) and anti-CXCR4 antibodies (**B**).
**Figure 6****:** Non-relapse mortality (NRM) of patients suffering from acute high grade GVHD (n=103). Serum levels of CXCL9 (MIG) were measured at onset of acute GVHD. NRM of patients with CXCL9-levels exceeding the median (679 pg/ml) was significantly higher than for patients with low CXCL9 levels. allo-TPL = alloSCT.
**Figure 7****:** Non-relapse mortality (NRM) of patients suffering from acute high grade GVHD (n=103). Serum levels of anti-CXCR3 autoantibodies were measured at onset of acute GVHD. NRM of patients with serum concentrations of anti-CXCR3 autoantibodies exceeding the median (2.74 U/ml) was significantly lower than for patients with high concentrations of anti-CXCR3 autoantibodies. allo-TPL = alloSCT.
**Figure 8****:** Non-relapse mortality (NRM) of patients suffering from acute high grade GVHD (n=103). Serum levels of CXCL9 (MIG) and anti-CXCR3 autoantibodies were measured at onset of acute GVHD. Three groups were defined according to CXCL9/anti-CXCR3 concentrations: 1. CXCL9>median and anti-CXCR3<median (high risk patients), 2. CXCL9<median and anti-CXCR3>median (low risk patients), 3 all others. allo-TPL = alloSCT.

### Detailed Description of the Invention

The inventors unexpectedly found that the presence of auto-antibodies directed against one or more CXCR in samples of a subject are indicative of the presence of an autoimmune disease in said subject. The inventors thereby provide a new and beneficial method for the diagnosis of an autoimmune disease. The invention hence relates to a method for diagnosis an autoimmune disease in a subject wherein, presence or absence of an anti-CXC chemokine receptor (CXCR) antibody is detected in a sample from the subject diagnosed, and wherein the presence of an anti-CXCR antibody is indicative of the disease.

The presence of antibodies in a sample may be performed using different approaches. It may in some cases be desirable to compare a determined level to a control level and attribute increased levels of antibodies in the sample of the subject to be diagnosed as compared to the control level to the presence of antibodies in the sample. Hence, in one embodiment of the method for diagnosis an autoimmune disease the present invention the presence or absence of the anti-CXCR antibody is detected by the following steps: (i) determining the level of anti-CXCR antibodies in a sample of the subject to be diagnosed; and (ii) comparing the determined level to an anti-CXCR antibody control level derived from a subject without an autoimmune disease; wherein an increased level of anti-CXCR antibody in the sample of the subject to be diagnosed as compared to the anti-CXCR antibody control level denotes the presence of an anti-CXCR antibody. The comparison of the determined level to the control level may be conducted in different ways. The assay used may for instance show a "positive" result for the presence of an anti-CXCR antibody only if the determined level is above a certain threshold. Such thresholds may for instance be ratios. Control levels may be determined using mean values of a plurality of reference samples, e.g. healthy subjects. However, levels may naturally vary around the so determined mean value. Hence, it may be desirable to set a threshold compared to the control level which denotes the presence of an anti-CXCR antibody in the sample. In one embodiment a difference of +/- 10 % is attributed to "equal" level, as further defined herein. Hence, in one embodiment of the method to the present invention a level in the sample of the subject to be diagnosed of more than 1.1 fold as compared to the control level from subjects without an autoimmune disease denotes the presence of an anti-CXCR antibody in the sample of the subject to be diagnosed, preferably a level in the sample of the subject to be diagnosed of more than 1.8 fold as compared to the control level from subjects without an autoimmune disease denotes the presence of an anti-CXCR antibody in the sample of the subject to be diagnosed, more preferably a level in the sample of the subject to be diagnosed of more than 2 fold as compared to the control level from subjects without an autoimmune disease denotes the presence of an anti-CXCR antibody in the sample of the subject to be diagnosed.

As outlined herein, the comparison is used to determine the "presence" of an auto-antibody in a sample, while the presence of the autoantibody itself is indicative of the presence of an autoimmune disease in the subject. Hence, the diagnosis may be directly performed using the comparison. As outlined herein, the determination of the presence or absence may be conducted by comparing the level of antibodies directed against a CXC chemokine receptor in the sample of the subject to be diagnosed to control levels, e.g. from healthy subjects. Determined levels above the control level are then denoted to the presence of an anti-CXCR antibody in the sample, while levels equal or lower than the control level are denoted to the absence of an anti-CXCR antibody in the sample. Hence, the invention also relates to a method for diagnosis an autoimmune disease comprising the following steps: (i) determining the level of anti-CXCR antibodies in a sample of the subject to be diagnosed; and (ii) comparing the determined level to an anti-CXCR antibody control level derived from a subject without an autoimmune disease; wherein an increased level of anti-CXCR antibody in the sample of the subject to be diagnosed as compared to the anti-CXCR antibody control level is indicative of the disease, preferably for the presence of the disease. The outlined ratio may directly be used to indicate the presence of the autoimmune disease. Hence, in one embodiment of the method to the present invention a level in the sample of the subject to be diagnosed of more than 1.1 fold as compared to the control level from subjects without an autoimmune disease is indicative of the presence of an autoimmune disease in the subject to be diagnosed, preferably a level in the sample of the subject to be diagnosed of more than 1.8 fold as compared to the control level from subjects without an autoimmune disease is indicative of the presence of an autoimmune disease in the subject to be diagnosed, more preferably a level in the sample of the subject to be diagnosed of more than 2 fold as compared to the control level from subjects without an autoimmune disease is indicative of the presence of an autoimmune disease in the subject to be diagnosed.

The control levels as used herein refer to control levels of anti-CXCR antibodies derived from the defined control group. It will be readily understood by the skilled person that the control levels from subjects having the desired disease, being healthy or the desired disease activity as defined in the methods and to which the determined levels are compared to, are not necessarily determined in parallel but may be represented by previously determined levels. Nevertheless, control levels may be determined in parallel. When referring to control levels from subjects having an active or inactive autoimmune disease, the autoimmune disease is preferably an autoimmune disease of connective tissue or graft-versus-host disease (GvHD), preferably selected from the group consisting of systemic lupus erythematosus (SLE), rheumatoid arthritis, scleroderma, Sjögren's syndrome, mixed connective tissue disease, systemic sclerosis (SSc); dermatomyositis (DM), polymyositis (PM), anti-synthetase syndrome, and psoriatic arthritis, more preferably systemic lupus erythematosus. The GvHD is preferably a chronic GvHD. The skilled person with the disclosure of the present invention and his knowledge is able to determine such control levels, as will be outlined herein below. Hence, the control levels of the present invention may be previously defined thresholds. Preferred thresholds are also disclosed herein but are not limiting the invention. Furthermore, it will be acknowledged by the skilled person that control levels are, like the levels to be determined in the patient to be diagnosed or treated, determined in samples of the recited subjects having the desired disease or symptoms or being healthy (not suffering from a autoimmune disease, preferably not suffering from the autoimmune disease to be diagnosed). Preferably, the sample is the same kind of sample as the sample of the person to be diagnosed, e.g. when the sample of the latter is serum, the control levels are preferably determined in serum samples derived from the control subjects. However, as outlined the control levels according to the present invention may also be predetermined control levels which are also integrated into the assay used. For example the assay may be designed in a way to give only positive results for the determination of the anti-CXCR antibody if the concentration of anti-CXCR antibodies in the sample to be analysed is above a certain level, e.g. increased as compared to the respective control.

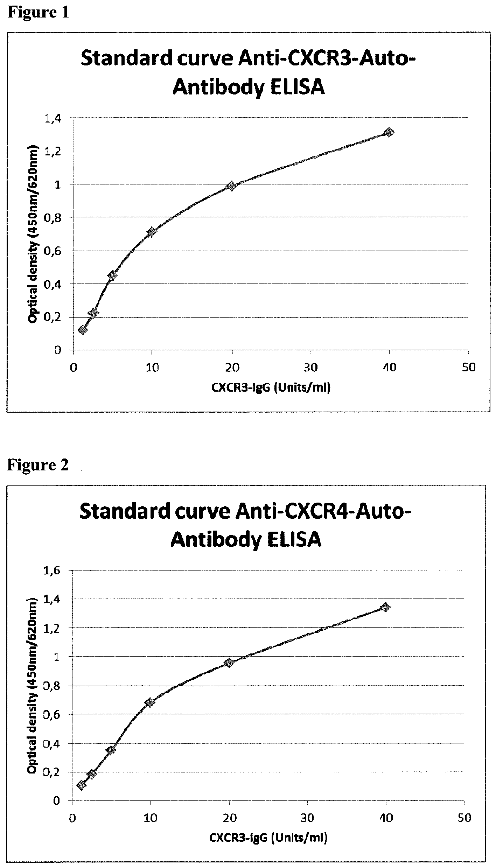

As outlined herein, the levels of anti-CXCR antibodies in samples of the patient to be diagnosed and treated or to be treated are compared with the control groups as defined herein. However, in one embodiment the levels are compared to fixed values, i.e. thresholds under or over which a certain diagnosis, or prognosis of response is given. To this end, unit-standards may be applied. The present inventors set out such standard for the anti-CXCR antibody, both anti-CXCR3 and anti-CXCR4 antibodies, using serum samples from patients suffering from systemic sclerosis. Systemic sclerosis patients are known to have high levels of autoimmune antibodies in general. Hence, the inventors took a serum sample of a systemic sclerosis patient. However, it will be acknowledged by the skilled person that also other samples may be taken to set a different standard, e.g. samples of healthy subjects. Nevertheless, the principle of generating a standard (units) is the same in any case and is exemplified herein using serum samples of systemic sclerosis patients. In the context of the present invention "units/ml", unless specified otherwise, refers to the concentration of antibodies standardised as exemplified herein. Hence, in one embodiment of the present invention 40 units/ml refers to a dilution of 1:50 of a serum sample of systemic sclerosis patients. The serum sample may be derived from a single patient or of a cohort of a plurality of patients. The present inventors found that the concentration of anti-CXCR antibodies in samples of systemic sclerosis do not differ by more than about 10 %, showing such standard being reproducible. In one preferred embodiment the standard for the concentrations of the autoimmune antibodies is generated in the following way: a serum sample of a systemic sclerosis patient (or a larger cohort) is diluted (a) 1:50 for standard point 40 Units/ml, (b) 1:100 for standard point 20 Units/ml, (c) 1:200 for standard point 10 Units/ml, (d) 1:400 for standard point 5 Units/ml, (e) 1:800 for standard point 2.5Units/ml, (f) 1:1600 for standard point 1.25 Units/ml. These standards are then used for the immunoassay chosen, e.g. ELISA, and then correlated with the respective read-out value, e.g. for ELISA optical density at 450nm/ optical density at 620 nm. A typical standard curve for an anti-CXCR3 and anti-CXCR 4 auto-antibody ELISA is shown in Figures 1 and 2, respectively. Nevertheless, the skilled person will readily understand that it may also be possible to standardize the levels of anti-CXCR antibodies using different samples, e.g. samples of healthy subjects or patients with an autoimmune disease.

As also outlined herein, levels of anti-CXCR antibodies in samples of patient/subjects may be measured in terms of units/ml (U/ml). In a preferred embodiment of the method for diagnosing according to the present invention a level of anti-CXCR antibodies in the sample of the patient to be diagnosed of more than 3 units/ml is indicative of the autoimmune disease preferably a level of more than 3.5 units/ml, even more preferably of more than 5 units/ml. As outlined herein, the skilled person is aware of methods for determining units/ml of an antibody.

However, in a preferred embodiment the units according to the present invention are determined using the unit-standards or unit solutions as outlined in greater detail herein.

Furthermore, the inventors unexpectedly found that the level of anti-CXCR antibodies in samples of a subject correlates with the degree of disease activity of an autoimmune disease in said subject, i.e. the higher the level of anti-CXCR antibodies in samples of a subject, the more active the autoimmune disease is in said subject. Even though subdivisions of different activity levels of autoimmune diseases may be desirable, it is preferred to differentiate between inactive and active autoimmune diseases, as for example medication is often indicated only in case the disease is active. Hence, the present invention also relates to a method for determining disease activity of an autoimmune disease comprising the steps of: (i) determining the level of anti-CXCR antibodies in a sample from a subject to be diagnosed, (ii) comparing the determined level in the sample to either one or both of a first and second anti-CXCR antibody control level, a) wherein the first anti-CXCR antibody control level is derived from subjects suffering an inactive autoimmune disease; and b) wherein the second anti-CXCR antibody control level derived from subjects suffering an active autoimmune disease; wherein a increased level in the sample from the subject to be diagnosed as compared to the first anti-CXCR antibody control level and/or an equal level as compared to the second anti-CXCR antibody control level is indicative of the presence of an active autoimmune disease in the subject to be diagnosed, and wherein an decreased level in the sample from the subject to be diagnosed as compared to the second anti-CXCR antibody control level and/or an equal level as compared to the first anti-CXCR antibody control level is indicative of the presence of an inactive autoimmune disease.

Further, the skilled person with the disclosure of the present application and in particular the data presented in the examples will acknowledge that the present invention also provides for a method of diagnosis the grade of an autoimmune disease, e.g. SLE. A scoring nowadays is performed using the BILAG-2004 activity index as published (see Isenberg DA et al. BILAG 2004. Development and initial validation of an updated version of the British Isles Lupus Assessment Group's disease activity index for patients with systemic lupus erythematous. Rheumatology. 2005; 44:902-906; Yee CS, et al. Revised British Isles Lupus Assessment Group 2004 index: a reliable tool for assessment of systemic lupus erythematosus activity. Arthritis Rheum. 2006; 54:3300-3305; and Yee CS, et al. British Isles Lupus Assessment Group 2004 index is valid for assessment of disease activity in systemic lupus erythematosus. Arthritis Rheum. 2007; 56:4113-4119; all of which are incorporated herein by reference). The scoring implies a rating into mild, moderate and severe activity. The inventors that the higher the levels of the anti-CXCR3 or anti-CXCR4 antibodies the more severe the activity of the autoimmune disease. Hence, the method for determining disease activity of an autoimmune disease is preferably a method for determining the activity status of an autoimmune disease comprising the steps of: (i) determining the level of anti-CXCR antibodies in a sample from a subject to be diagnosed, (ii) comparing the determined level in the sample to either one or two or all three of a first, second and third anti-CXCR antibody control level, a) wherein the first anti-CXCR antibody control level is derived from subjects suffering an autoimmune disease with mild activity; and b) wherein the second anti-CXCR antibody control level derived from subjects suffering an autoimmune disease with moderate activity; and c) wherein the third anti-CXCR antibody control level derived from subjects suffering an autoimmune disease with severe activity; wherein an equal level in the sample from the subject to be diagnosed as compared to the first anti-CXCR antibody control level and/or an decreased level as compared to the second and/or third anti-CXCR antibody control level is indicative of the presence of an active autoimmune disease with mild activity in the subject to be diagnosed, and wherein an increased level in the sample from the subject to be diagnosed as compared to the first anti-CXCR antibody control level and a decreased level as compared to the third anti-CXCR antibody control level; and/or an equal level as compared to the second anti-CXCR antibody control level is indicative of the presence of an active autoimmune disease with moderate activity; and wherein an equal or increased level in the sample from the subject to be diagnosed as compared to the third anti-CXCR antibody control level and/or an increased level as compared to the first and/or second anti-CXCR antibody control level is indicative of the presence of an active autoimmune disease with severe activity in the subject to be diagnosed. Preferred anti-CXCR antibodies are anti-CXCR3 or anti-CXCR4 antibodies. The autoimmune disease is preferably SLE. The definition of the activity status of SLE is preferably as disclosed in above references for BILAG2004.

"Equal" level in context with the present invention means that the levels differ by not more than ± 10 %, preferably by not more than ± 5 %, more preferably by not more than ± 2 %."Decreased" or "increased" level in the context of the present invention mean that the levels differ by more than 10%, preferably by more than 15 %, preferably more than 20 %.

The autoimmune disease according to the present invention is preferably an autoimmune disease of connective tissue (CTD). The autoimmune disease of connective tissue is preferably selected from the group consisting of systemic lupus erythematosus (SLE), rheumatoid arthritis, scleroderma, Sjögren's syndrome, mixed connective tissue disease, systemic sclerosis (SSc); dermatomyositis (DM), polymyositis (PM), anti-synthetase syndrome, graft versus host disease (GvHD), preferably a chronic GvHD (cGvHD) and psoriatic arthritis, more preferably systemic lupus erythematosus.

In the context of the present invention, the levels of the anti-CXCR antibodies a may be analyzed in a number of fashions well known to a person skilled in the art. For example, each assay result obtained may be compared to a "normal" value, or a value indicating a particular disease or outcome. A particular diagnosis/prognosis may depend upon the comparison of each assay result to such a value, which may be referred to as a diagnostic or prognostic "threshold". In certain embodiments, assays for one or more diagnostic or prognostic indicators are correlated to a condition or disease by merely the presence or absence of the indicator(s) in the assay. For example, an assay can be designed so that a positive signal only occurs above a particular threshold concentration of interest, and below which concentration the assay provides no signal above background.

The sensitivity and specificity of a diagnostic and/or prognostic test depends on more than just the analytical "quality" of the test, they also depend on the definition of what constitutes an abnormal result. In practice, Receiver Operating Characteristic curves (ROC curves), are typically calculated by plotting the value of a variable versus its relative frequency in "normal" (i.e. apparently healthy individuals not having fibrosis or graft rejection, e.g. with operational tolerance) and "disease" populations. For any particular marker, a distribution of marker levels for subjects with and without a disease will likely overlap. Under such conditions, a test does not absolutely distinguish normal from disease with 100% accuracy, and the area of overlap indicates where the test cannot distinguish normal from disease. A threshold is selected, below which the test is considered to be abnormal and above which the test is considered to be normal. The area under the ROC curve is a measure of the probability that the perceived measurement will allow correct identification of a condition. ROC curves can be used even when test results don't necessarily give an accurate number. As long as one can rank results, one can create a ROC curve. For example, results of a test on "disease" samples might be ranked according to degree (e.g. 1=low, 2=normal, and 3=high). This ranking can be correlated to results in the "normal" population, and a ROC curve created. These methods are well known in the art; *see,* e.g., Hanley et al. 1982. Radiology 143: 29-36*.* Preferably, a threshold is selected to provide a ROC curve area of greater than about 0.5, more preferably greater than about 0.7, still more preferably greater than about 0.8, even more preferably greater than about 0.85, and most preferably greater than about 0.9. The term "about" in this context refers to +/-5% of a given measurement.

The horizontal axis of the ROC curve represents (1-specificity), which increases with the rate of false positives. The vertical axis of the curve represents sensitivity, which increases with the rate of true positives. Thus, for a particular cut-off selected, the value of (1-specificity) may be determined, and a corresponding sensitivity may be obtained. The area under the ROC curve is a measure of the probability that the measured marker level will allow correct identification of a disease or condition. Thus, the area under the ROC curve can be used to determine the effectiveness of the test.

In other embodiments, a positive likelihood ratio, negative likelihood ratio, odds ratio, or hazard ratio is used as a measure of a test's ability to predict risk or diagnose a disease. In the case of a positive likelihood ratio, a value of 1 indicates that a positive result is equally likely among subjects in both the "diseased" and "control" groups; a value greater than 1 indicates that a positive result is more likely in the diseased group; and a value less than 1 indicates that a positive result is more likely in the control group. In the case of a negative likelihood ratio, a value of 1 indicates that a negative result is equally likely among subjects in both the "diseased" and "control" groups; a value greater than 1 indicates that a negative result is more likely in the test group; and a value less than 1 indicates that a negative result is more likely in the control group.

In the case of an odds ratio, a value of 1 indicates that a positive result is equally likely among subjects in both the "diseased" and "control" groups; a value greater than 1 indicates that a positive result is more likely in the diseased group; and a value less than 1 indicates that a positive result is more likely in the control group.

In the case of a hazard ratio, a value of 1 indicates that the relative risk of an endpoint (e.g., death) is equal in both the "diseased" and "control" groups; a value greater than 1 indicates that the risk is greater in the diseased group; and a value less than 1 indicates that the risk is greater in the control group.

The skilled artisan will understand that associating a diagnostic or prognostic indicator, with a diagnosis or with a prognostic risk of a future clinical outcome is a statistical analysis. For example, a marker level of lower than X may signal that a patient is more likely to suffer from an adverse outcome than patients with a level more than or equal to X, as determined by a level of statistical significance. Additionally, a change in marker concentration from baseline levels may be reflective of patient prognosis, and the degree of change in marker level may be related to the severity or activity of the disease. Statistical significance is often determined by comparing two or more populations, and determining a confidence interval and/or a p value; *see,* e.g., Dowdy and Wearden, Statistics for Research, John Wiley & Sons, New York, 1983. Preferred confidence intervals of the invention are 90%, 95%, 97.5%, 98%, 99%, 99.5%, 99.9% and 99.99%, while preferred p values are 0.1, 0.05, 0.025, 0.02, 0.01, 0.005, 0.001, and 0.0001.

Suitable threshold levels for the stratification of subjects into different groups (categories) have to be determined for each particular combination of anti-CXCR antibodies, disease and/or medication. This can e.g. be done by grouping a reference population of patients according to their level of anti-CXCR antibodies into certain quantiles, e.g. quartiles, quintiles or even according to suitable percentiles. Preferably the the presence of an autoimmune disease according to the invention is indicated at anti-CXCR antioby levels above the 75^{th} percentile of a normal (healthy) population, more prerably above the 80^{th} percentile, more preferably above the 90^{th} percentile, even more preferred above the 95^{th} percentile. For each of the quantiles or groups above and below certain percentiles, hazard ratios can be calculated comparing the risk for an adverse outcome, i.e. an "active disease" or a "need for administration of an immunosuppressant", e.g. in terms of survival rate/mortality, between those patients who have received a certain medication and those who did not, or in terms of presence and absence of graft intolerance in patients. In such a scenario, a hazard ratio (HR) above 1 indicates a higher risk for an adverse outcome for the patients who have received a treatment than for patients who did not. A HR below 1 indicates beneficial effects of a certain treatment in the group of patients. A HR around 1 (e.g. +/- 0.1) indicates no elevated risk but also no benefit from medication for the particular group of patients. By comparison of the HR between certain quantiles of patients with each other and with the HR of the overall population of patients, it is possible to identify those quantiles of patients who have an elevated risk and those who benefit from medication and thereby stratify subjects according to the present invention.

In some cases presence of the autoimmune disease or active autoimmune disease, respectively, will affect patients with low levels (e.g. in the fifth quintile) of anti-CXCR antibodies, while in other cases only patients with high levels of anti-CXCR antibodies will be affected (e.g. in the first quintile). However, with the above explanations, a skilled person is able to identify those groups of patients having the disease or active disease. Exemplarily, some combinations of antibodies and medications are listed for several diseases in the appended examples. In another embodiment of the invention, the diagnosis, the differential diagnosis, the assessment of the need for an immunosuppressant (when active disease is present) or the monitoring of the need for a immunosuppressant are determined by relating the patient's individual level of marker peptide to certain percentiles (e.g. 75^{th}, 80^{th}, 90^{th}, 95^{th} or 97.5^{th} percentile; preferably 95^{th}) of a healthy population.

Kaplan-Meier estimators may be used for the assessment or prediction of the outcome or risk (e.g. diagnosis, disease activity) of a subject.

Furthermore, in the methods of the present invention further parameters of the subject may be considered as well for diagnosis, differential diagnosis, assessment of the need for an immunosuppressant, therapy monitoring etc. Such parameters in a multivariate model may include gender, age, histological evaluation, and other markers. A Cox-Proportional-Hazard regression predicts the dependent variable based on one or more independent variables. These predictors can either be measures (as e.g. level of a biomarker) or categorical data. The skilled person is aware of the fact that diagnostic markers only give a certain degree of sensitivity and specificity. He knows that different further parameters might be considered in order to increase both. Nevertheless, the present invention provides a new and superior marker for diagnosis, disease activity, assessment of the need for an immunosuppressant, therapy monitoring as outlined herein in great detail. In the context of the methods of the invention and particularly the immunoassays of the invention, the presence of one or more further diagnostic markers for the autoimmune disease is detected in the sample. For example, in a diagnostic method of the present invention levels of ANA detected in addition. Antinuclear antibody (ANA) testing and anti-extractable nuclear antigen (anti-ENA) form the mainstay of serologic testing for SLE. ANA screening yields positive results in many connective tissue disorders and other autoimmune diseases, and may occur in normal individuals.

In the methods according to the present invention the presence of anti-CXCR antibodies in a sample or the determination of the level of CXCR antibodies is preferably performed using immune assays. Preferably the anti-CXCR antibody is detected using an immunoassay comprising the steps of (a) contacting the sample with a CXC chemokine receptor (CXCR) or an antigenic peptide fragment thereof under conditions allowing for the formation of a complex between anti-CXCR antibodies with CXCR or the antigenic peptide fragment thereof; (b) detecting the complex.

In the methods of the present invention, the anti-CXCR antibody is preferably detected in an immunoassay. Suitable immunoassays may be selected from the group consisting of immunoprecipitation, enzyme immunoassay (EIA)), enzyme-linked immunosorbent assay (ELISA), radioimmunoassay (RIA), fluorescent immunoassay, a chemiluminescent assay, an agglutination assay, nephelometric assay, turbidimetric assay, a Western Blot, a competitive immunoassay, a noncompetitive immunoassay, a homogeneous immunoassay a heterogeneous immunoassay, a bioassay and a reporter assay such as a luciferase assay. Preferably herein the immunoassay is an enzyme linked immunosorbent assay (ELISA).

The immunoassays can be homogenous or heterogeneous assays, competitive and non-competitive assays. In a particularly preferred embodiment, the assay is in the form of a sandwich assay, which is a non-competitive immunoassay, wherein the anti-CXCR antibody (i.e. the "analyte") to be detected and/or quantified is allowed to bind to an immobilized CXCR protein or immunogenic peptide fragment thereof and to a secondary antibody or other detection means. The CXCR protein or immunogenic fragment thereof (i.e. a peptide), may e.g., be bound to a solid phase, e.g. a bead, a surface of a well or other container, a chip or a strip, and the secondary antibody detection mean is an antibody or detection mean which is labeled, e.g. with a dye, with a radioisotope, or a reactive or catalytically active moiety such as a peroxidase, e.g. horseradish peroxidase. The amount of labeled antibody bound to the analyte is then measured by an appropriate method. The general composition and procedures involved with "sandwich assays" are well-established and known to the skilled person (The Immunoassay Handbook, Ed. David Wild, Elsevier LTD, Oxford; 3rd ed. (May 2005), ISBN-13: 978-0080445267*;* Hultschig C et al., Curr Opin Chem Biol. 2006 Feb;10(1):4-10*. PMID: 16376134,* incorporated herein by reference). Sandwich immunoassays can for example be designed as one-step assays or as two-step assays.

In the methods according to the present invention the CXCR or the immunogenic peptide fragment thereof used in the methods are in one embodiment immobilized on a surface.

The skilled artisan will understand that for detection of the so bound anti-CXCR antibody to be detected a secondary antibody or detection mean, e.g. scaffolds, may be advantageous as outlined herein. The secondary antibody preferably binds anti-CXCR antibodies in a specific manner. The detection of the complex between anti-CXCR antibodies and the CXCR protein or peptide fragment is in one embodiment performed using a secondary antibody or detection mean against the Fc portion of the anti-CXCR antibody. Furthermore, if it is desirable to determine only a certain type of antibodies, the skilled person is aware of type specific secondary antibodies or detection means, e.g. anti-IgG antibodies, or even subtype specific antibodies, e.g. IgG1 antibodies or the like. In the context of the present invention the anti-CXCR antibody may particularly be selected from the group of IgA-antibody, IgG-antibody and IgM-antibody, preferably an IgG antibody, e.g. IgG1, IgG2, IgG3 and IgG4. In one embodiment of the present invention the anti-CXCR antibody is an IgG-antibody, preferably detecting all subtypes of IgG.

The term "secondary antibody" comprises monoclonal and polyclonal antibodies and binding fragments thereof, in particular Fc-fragments as well as so called "single-chain-antibodies" (Bird R. E. et al (1988) Science 242:423-426), chimeric, humanized, in particular CDR-grafted antibodies, and dia- or tetrabodies (Holliger P. et al (1993) Proc. Natl. Acad. Sci. U.S.A. 90:6444-8). Also comprised are immunoglobulin like proteins that are selected through techniques including, for example, phage display to specifically bind to the molecule of interest contained in a sample. In this context the term "specific binding" refers to antibodies raised against the molecule of interest or a fragment thereof. An antibody is considered to be specific, if its affinity towards the molecule of interest or the aforementioned fragment thereof is at least 50-fold higher, preferably 100-fold higher, more preferably at least 1000-fold higher than towards other molecules comprised in a sample containing the molecule of interest. It is well known in the art how to make antibodies and to select antibodies with a given specificity.

The detectable label may for example be based on fluorescence or chemiluminescence. The labelling system comprises rare earth cryptates or rare earth chelates in combination with a fluorescence dye or chemiluminescence dye, in particular a dye of the cyanine type. In the context of the present invention, fluorescence based assays comprise the use of dyes, which may for instance be selected from the group comprising FAM (5-or 6-carboxyfluorescein), VIC, NED, Fluorescein, Fluoresceinisothiocyanate (FITC), IRD-700/800, Cyanine dyes, such as CY3, CY5, CY3.5, CY5.5, Cy7, Xanthen, 6-Carboxy-2',4',7',4,7-hexachlorofluorescein (HEX), TET, 6-Carboxy-4',5'-dichloro-2',7'-dimethodyfluorescein (JOE), N,N,N',N'-Tetramethyl-6-carboxyrhodamine (TAMRA), 6-Carboxy-X-rhodamine (ROX), 5-Carboxyrhodamine-6G (R6G5), 6-carboxyrhodamine-6G (RG6), Rhodamine, Rhodamine Green, Rhodamine Red, Rhodamine 110, BODIPY dyes, such as BODIPY TMR, Oregon Green, Coumarines such as Umbelliferone, Benzimides, such as Hoechst 33258; Phenanthridines, such as Texas Red, Yakima Yellow, Alexa Fluor, PET, Ethidiumbromide, Acridinium dyes, Carbazol dyes, Phenoxazine dyes, Porphyrine dyes, Polymethin dyes, and the like.

In the context of the present invention, chemiluminescence based assays comprise the use of dyes, based on the physical principles described for chemiluminescent materials in Kirk-Othmer, Encyclopedia of chemical technology, 4th ed., executive editor, J. I. Kroschwitz; editor, M. Howe-Grant, John Wiley & Sons, 1993, vol. 15. p. 518-562*,* incorporated herein by reference, including citations on pages 551-562. Preferred chemiluminescent dyes are acridiniumesters.

In a preferred embodiment of the present invetnion the immunoassay is selected from the group of immunoprecipitation, enzyme immunoassay (EIA), radioimmunoassay (RIA) or fluorescencent immunoassay, a chemilumineszent assay, an agglutination assay, nephelometric assay, turbidimetric assay, a Western blot, a competitive immunoassay, a noncompetitive immunoassay, a homogeneous immunoassay a heterogeneous immunoassay, a bioassay and a reporter-assay such as a Luciferase-Assay, particularly preferred the immunoassay is an ELISA.

The "sensitivity" of an assay relates to the proportion of actual positives which are correctly identified as such, i.e. the ability to identify positive results (true positives positive results / number of positives). Hence, the lower the concentrations of the analyte that can be detected with an assay, the more sensitive the immunoassay is. The "specificity" of an assay relates to the proportion of negatives which are correctly identified as such, i.e. the ability to identify negative results (true negatives / negative results). For an antibody the "specificity" is defined as the ability of an individual antigen binding site to react with only one antigenic epitope. The binding behaviour of an antibody can also be characterized in terms of its "affinity" and its "avidity". The "affinity" of an antibody is a measure for the strength of the reaction between a single antigenic epitope and a single antigen binding site. The "avidity" of an antibody is a measure for the overall strength of binding between an antigen with many epitopes and multivalent antibodies.

The term "patient" and "subject" as used herein refers to a subject, preferably the subject is a mammal, in particular human mammals are preferred. Preferably the subject to be diagnosed is a subject suspected to have an autoimmune disease, the autoimmune disease preferably being as defined herein.

Herein, the sample of the subject to be diagnosed in which the level of anti-CXCR antibodies are to be determined is preferably a bodily fluid such as whole blood or lymph or fractions of blood such as serum or plasma. Preferably in the context of the present invention the sample is plasma or serum. The sample according to the present invention is preferably a bodily fluid of the patient, however, also tissue samples, e.g. of the connective tissue or other tissues targeted by the autoimmune disease may be used. In a preferred embodiment the bodily fluid is blood. Further preferred samples are plasma and serum samples, most preferred serum.

Where appropriate, the sample may need to be homogenized, or extracted with a solvent prior to use in the present invention in order to obtain a liquid sample. A liquid sample hereby may be a solution or suspension. Liquid samples may be subjected to one or more pre-treatments prior to use in the present invention. Such pre-treatments include, but are not limited to dilution, filtration, centrifugation, concentration, sedimentation, precipitation, and dialysis. Pre-treatments may also include the addition of chemical or biochemical substances to the solution, such as acids, bases, buffers, salts, solvents, reactive dyes, detergents, emulsifiers, chelators.

"Plasma" in the context of the present invention is the virtually cell-free supernatant of blood containing anticoagulant obtained after centrifugation. Exemplary anticoagulants include calcium ion binding compounds such as EDTA or citrate and thrombin inhibitors such as heparinates or hirudin. Cell-free plasma can be obtained by centrifugation of the anticoagulated blood (e.g. citrated, EDTA or heparinized blood) for at least 15 minutes at 2000 to 3000 g.

"Serum" is the liquid fraction of whole blood that is collected after the blood is allowed to clot. When coagulated blood (clotted blood) is centrifuged serum can be obtained as supernatant. It does not contain fibrinogen, although some clotting factors remain.

"anti-CXCR antibody" and "CXCR antibody" are used interchangeably herein and refer to antibodies specifically binding CXC chemokine receptor or fragments thereof, preferably autoimmune antibodies. The anti-CXCR antibody binds specifically to the CXCR or in doing so shows specific immuno reactivity when the anti- CXCR antibody assumes its function in a binding reaction in the presence of a heterogeneous population of CXCRs or fragments thereof, thereby allowing a conclusion whether the CXCR or another biological structure is present. Under the present conditions of an immunoassay, the above-mentioned anti-CXCR antibodies will preferably bind to a specific portion of the CXCR, e.g. the extra cellular portion, while no significant binding to other proteins present in the sample will take place. In a preferred embodiment the anti-CXCR antibodies are selected from the group of antibodies consisting of IgG-antibodies and IgM-antibodies, preferably an IgG antibody, e.g. IgG1, IgG2, IgG3 and IgG4. In a certain preferred embodiment total IgG type anti-CXCR antibodies are to be detected in the methods of the present invention.

In connection with the present invention, the naturally occurring receptor as well as all modifications, mutants or derivatives of the CXC chemokine receptor can be used. Similarly, an CXC chemokine receptor produced by means of recombinant techniques, which receptor includes amino acid modifications, such as inversions, deletions, insertions, additions etc. can be used according to the invention provided that this part of the essential function of the CXC chemokine receptor is present, namely the capability of binding antibodies. The CXC chemokine receptor being used may also comprise exceptional amino acids and/or modifications of such as alkylation, oxidation, thiol-modification, denaturation, oligomerization and the like. The receptor can also be synthesized by chemical means. According to the invention the CXC chemokine receptor particularly can be a protein and/or peptide or a fusion protein, which in addition to other proteins, peptides or fragments thereof, includes the CXC chemokine receptor as a whole or in part. Using conventional methods, peptides or polypeptides of the CXC chemokine receptor which have functionally analogs, analogous properties can be determined by those skilled in the art. For example such polypeptides or peptides have 50 to 60%, 70% or 80%, preferably 90%, more preferably 95%, and most preferably 98% homology to peptides identified as CXC chemokine receptor, and said homology can be determined, e.g. by means of Smith-Waterman homology search algorithm, using the MPFRCH Programme (Oxford Molecular), for example.

An "immunogenic peptide" or "antigenic peptide" as used herein interchangeably is a portion of a CXCR protein that is recognized (i.e., specifically bound) by the anti- CXCR antibodies in the sample of the patient to be detected. Such immunogenic peptides generally comprise at least 5 amino acid residues, more preferably at least 10, and still more preferably at least 20 amino acid residues of CXCR. However, they may also comprise at least 30, 40, 50, 60, 70, 80, 90, 100, 110, 120, 130, 140 or 150 amino acid residues. In a preferred embodiment the immunogenic peptide is at least 112 amino residues in length. The term "immunogenic peptide" of an CXC chemokine receptor as used in the present invention, comprises also molecules differing from the original sequence by deletion(s), insertion(s), substitution(s) and/or other modifications well known in the prior art and/or comprising a fragment of the original amino acid molecule, the CXC chemokine receptor still exhibiting the properties mentioned above. Also included are allele variants and modifications. Methods of producing the above changes in the amino acid sequence are well known to those skilled in the art and have been described in the standard textbooks of molecular biology, e.g. Sambrook *et al.* Supra. Those skilled in the art will also be able to determine whether a CXC chemokine receptor (CXCR), thus, modified still has the properties mentioned above. Database entries exist in several well known Databases. When referreing to the amino acid sequence of CXCR any amino acid sequence known is meant or immunogenic fragments thereof, particularly those disclosed in common databases, preferably of human origin. The CXCR may be glycosylated *in vivo.* In the present specification all of the above illustrated modifications of the CXCR will be referred to as "functionally analogous peptides or proteins" in brief. Preferably the CXC chemokine receptor (CXCR) is selected from the group consisting of CXCR3 and CXCR4, preferably the CXC chemokine receptor (CXCR) has a sequence selected from the group consisting of SEQ ID NO:1 (CXCR3-A), SEQ ID NO:2 (CXCR3-B), SEQ ID NO:3 (CXCR4a), and SEQ ID NO:4 (CXCRb). In a preferred embodiment the CXCR at least comprises an immunogenic fragment thereof, preferred immunogenic fragments comprise a sequence at least 50 %, 60%, 70% or 80%, preferably at least 90%, more preferably at least 95%, and most preferably at least 98% identical to the sequence of SEQ ID NO:5 (immunogenic CXCR3 fragment common to both isoforms) or SEQ ID NO:6 (immunogenic CXCR4b fragment)

In the context of the immunoassays of the present invention the "CXCR" may be present in its natural cellular environment and can be used together with the material associated with CXCR in its natural state as well as in isolated form with respect to its primary, secondary and tertiary structures. CXCRs are well known to those skilled in the art. The receptor is preferably used in isolated form, i.e. essentially free of other proteins, lipids, carbohydrates or other substances naturally associated with CXCR. "Essentially free of' means that the receptor is at least 75%, preferably at least 85%, more preferably at least 95% and especially preferably at least 99% free of other proteins, lipids, carbohydrates or other substances naturally associated with the receptor. However, the CXCR may also be used as a membrane extract from cells expressing the vector naturally or my recombinant means.

In connection with the present invention, the naturally occurring receptor as well as all modifications, mutants, immunogenic peptides or derivatives of the CXCR can be used. Similarly, a CXCR produced by means of recombinant techniques, which includes amino acid modifications, such as inversions, deletions, insertions, additions etc. can be used according to the invention provided that this part of the essential function of the CXCR is present, namely the capability of binding antibodies. The CXCR being used may also comprise exceptional amino acids and/or modifications of such as alkylation, oxidation, thiol-modification, denaturation, oligomerization and the like. The CXCR can also be synthesized by chemical means. According to the invention the CXCR particularly can be a protein and/or peptide or a fusion protein, which in addition to other proteins, peptides or fragments thereof, includes a CXCR as a whole or in part. Using conventional methods, peptides or polypeptides of the CXCR which have functionally analogs, analogous properties can be determined by those skilled in the art. For example such polypeptides or peptides have at least 50 %, 60%, 70% or 80%, preferably at least 90%, more preferably at least 95%, and most preferably at least 98% sequence identity to CXCR, preferably to the sequence selected from the group consisting of SEQ ID NO:1, SEQ ID NO:2, and SEQ ID NO:3.

The determination of percent identity between two sequences is accomplished using the mathematical algorithm of Karlin and Altschul (1993) Proc. Natl. Acad. Sci. USA 90: 5873-5877. Such an algorithm is incorporated into the BLASTN and BLASTP programs of Altschul et al. (1990) J. Mol. Biol. 215: 403-410. BLAST nucleotide searches are performed with the BLASTN program, score = 100, word length = 12, to obtain nucleotide sequences homologous to the EPO variant polypeptide encoding nucleic acids. BLAST protein searches are performed with the BLASTP program, score = 50, wordlength = 3, to obtain amino acid sequences homologous to the EPO variant polypeptide, respectively. To obtain gapped alignments for comparative purposes, Gapped BLAST is utilized as described in Altschul et al. (1997) Nucleic Acids Res. 25: 3389-3402. When utilizing BLAST and Gapped BLAST programs, the default parameters of the respective programs are used.

The antibodies to be detected or determined according to the present invention are directed against CXCR. This means that the antibodies specifically bind CXCR. Specific binding of an antibody normally occurs via binding of a binding site of the antigen. The antibodies of the present invention are those specifically binding to CXCR or immunogenic fragments thereof. This binding may occur via recognition of sequence or structural epitopes. The skilled person is aware of methods of how to determine specific epitopes, e.g. fragments of the antigen CXCR, which are recognized and bound by the antibodies to be determined. Fragments of CXCR binding to the auto antibodies are called immunogenic or antigenic fragments. Methods for determining fragments of an antigen binding the antibody are described in several publications (e.g. Gershoni, JM; Roitburd-Berman, A; Siman-Tov, DD; Tarnovitski Freund, N; Weiss, Y (2007). "Epitope mapping: The first step in developing epitope-based vaccines". BioDrugs 21 (3): 145-56; Westwood, MR; Hay, FC (2001). Epitope Mapping: a practical approach. Oxford, Oxfordshire: Oxford University Press. ISBN 0-19-963652-4; Flanagan et al. (2011), "Mapping Epitopes with H/D-Ex Mass Spec". Genetic Engineering and Biotechnology news; 31(1); Gaseitsiwe, S.; Valentini, D.; Mahdavifar, S.; Reilly, M.; Ehrnst, A.; Maeurer, M. (2009) "Peptide Microarray-Based Identification of Mycobacterium tuberculosis Epitope Binding to HLA-DRB1*0101, DRB1*1501, and DRB1*0401". Clinical and Vaccine Immunology 17 (1): 168-75; Linnebacher, Michael; Lorenz, Peter; Koy, Cornelia; Jahnke, Annika; Born, Nadine; Steinbeck, Felix; Wollbold, Johannes; Latzkow, Tobias et al. (2012). "Clonality characterization of natural epitope-specific antibodies against the tumor-related antigen topoisomerase IIa by peptide chip and proteome analysis: A pilot study with colorectal carcinoma patient samples" Analytical and Bioanalytical Chemistry 403 (1): 227-38; Cragg, M. S. (2011). "CD20 antibodies: Doing the time warp". Blood 118 (2): 219-20; Banik, Soma S. R.; Doranz, Benjamin J. (2010). "Mapping Complex Antibody Epitopes". Genetic Engineering and Biotechnology News 3 (2): 25-8; and Paes, Cheryl; Ingalls, Jada; Kampani, Karan; Sulli, Chidananda; Kakkar, Esha; Murray, Meredith; Kotelnikov, Valery; Greene, Tiffani A. et al. (2009). "Atomic-Level Mapping of Antibody Epitopes on a GPCR". Journal of the American Chemical Society 131 (20): 6952-4). In context with the present invention anti-CXCR antibodies are understood as any immunoglobulin specifically recognizing/binding to a CXCR. The antibody in a preferred embodiment binds any CXCR variant, preferably to any CXCR3 or CXCR4 variant, more preferably to a sequence selected from the group consisting of SEQ ID NO:1, SEQ ID NO:2, SEQ ID NO:3, and SEQ ID NO:4. Preferably recognizing a peptide comprising a sequence at least 50 %, 60%, 70% or 80%, preferably at least 90%, more preferably at least 95%, and most preferably at least 98% to the sequence of SEQ ID NO:5 or SEQ ID NO:6.

The skilled person will understand that controls for comparing the determined levels may be of different nature e.g. depending on the assay used. The kit according to the present invention may for example comprise one or more controls comprising anti-CXCR antibody at the desired control level. Furthermore, the kit may comprise one or more standard solutions each solution comprising anti-CXCR antibody at different levels, such standard solutions are particularly preferred in cases were a standard curves are to be applied. Exemplary dilutions and levels for such standard solutions are outlined herein above.

The embodiments set out for the immunoassays apply also to the kit of the invention. The kits of the present invention are meant for the detection of autoimmune antibodies in samples of a subject. Hence, in one embodiment they comprise means for the preparation of blood, e.g. for gaining serum or plasma thereof. Furthermore, the kit may comprise control composition and/or standards. The control composition preferably comprises anti-CXCR antibodies as positive control. Furthermore, the kit may comprise one or a plurality of standard compositions. A standard composition comprises anti-CXCR antibodies at a defined concentration. As outlined herein, determination of concentration of autoimmune-antibodies may be performed using standard curves. These curves set out which concentration of antibodies in a sample or solution corresponds to what read-out value of the assay used, e.g. optical density or proportion of optical density at different wavelengths (e.g. 450nm/620nm). To this end the kits of the present invention may comprise one or more standard compositions having a defined concentration of anti-CXCR antibodies, preferably of the kind to be detected in the method. A standard composition of the kits according to the present invention comprise anti-CXCR antibodies at concentrations selected from the group consisting of 40 units/ml, 20 units/ml, 10 units/ml, 5 units/ml, 2.5 units/ml and 1.25 units/ml. In one embodiment the kit comprises five standard compositions with the recited concentration. In another embodiment the kit comprises one standard composition with the highest concentration of the standard curve, e.g. 40 units/ml or 80 units/ml. The other concentrations may be produced at the side of the end user by further dilutions, e.g. in PBS. A dilution buffer may therefore also be comprised in the kits according to the invention.

Such kits can comprise a carrier, package or container that is compartmentalized to receive one or more containers such as vials, tubes, and the like, each of the container(s) comprising one of the separate elements to be used in the method. The kit of the invention will typically comprise the container described above and one or more other containers comprising materials desirable from a commercial and user standpoint, including buffers, diluents, filters, needles, syringes, and package inserts with instructions for use. In addition, a label can be provided on the container to indicate that the composition is used for a specific therapeutic or non-therapeutic application, and can also indicate directions for either *in vivo* or *in vitro* use, such as those described herein. Directions and or other information can also be included on an insert which is included with the kit.

In a preferred embodiment the kit comprises a CXCR, an immunogenic peptide or a functional analog thereof.

The immunological test kit according to the invention comprises the CXCR or a functional analog thereof or immunogenic peptides or proteins of analogous function *per se.* The test kit of the invention comprises at least one complete CXCR or functionally analogous peptides or proteins of said receptor, optionally bound to a solid phase. Furthermore, the test kit may also comprise buffers, specific conjugate together with an enzyme, wash solution, substrate solution to detect the immune reaction and/or a quenching solution. Using these substances a person skilled in the art will be able to perform, e.g. an ELISA to detect the anti-CXCR antibodies. The buffers, specific conjugate plus enzyme, wash solution, substrate solution to detect immune reaction and quenching solution are well known to those skilled in the art. For example, it would be sufficient to have the test comprise a freeze-dried CXCR or immunogenic peptides or proteins of CXCR analogous function and to add the buffers and other solutions immediately prior to testing the biological material. However, it is also possible to provide the test kit with the CXCR or its functionally analogous peptides of proteins bound to a solid phase. To detect the anti-CXCR antibodies the specific conjugate, wash solution, substrate solution and quenching solution, which can be components of the test kit, have to be added according to a mode well known to those skilled in the art.

The kit according to the present invention preferably comprises means for detecting antibodies, preferably a secondary antibody binding the Fc portion of the anti-CXCR antibody to be detected.

In another advantageous embodiment of the invention, it is envisioned that the test kit is a test strip comprising the CXCR or immunogenic peptides or its functionally analogous peptides or proteins immobilized on a solid phase. For example, the test strip can be immersed in serum or other patient samples and incubated. Using a specific biochemical reaction on the test strip after formation of the anti-CXCR antibody complex, a specific color reaction can be triggered by means of which the anti-CXCR antibody can be detected.

The test system of the invention permits quantification of the anti-CXCR antibodies directly in a sample, e.g. in plasma of patients. The detection method according to the invention is time saving and cost effected. Large amounts of the samples can be tested and, owing to the low amount of the equipment required, routine laboratories can be used.

It has been surprisingly found by the inventors that antibodies directed against CXCR have a diagnostic and predictive value. As readily understood by those of ordinary skill in the art, a subject is only to be treated for an autoimmune disease if the subject indeed suffers from such disease. Hence, before treatment the patient has to be diagnosed with a method according to the present invention. If deemed appropriate, the disease activity should also be determined before treating the subject, as sometimes treatment is only desired in case the autoimmune disease is active. Hence, the present invention also relates to a method for treating an autoimmune disease in a subject, wherein the subject is treated upon diagnosing an autoimmune disease or an autoimmune disease with an active autoimmune disease with a method according to the present invention. In a preferred embodiment the invention relates to an immunosuppressant for use in the treatment of an autoimmune disease in a subject, wherein the immunosuppressant is administered to the subject upon diagnosing an autoimmune disease or an active autoimmune disease with a method according to the present invention. The disease is preferably systemic lupus erythematosus. The immunosuppressant preferably is selected from the group consisting of cyclophosphamides, glucocorticoids, and Rituximab.

As outlined above, the invention also relates to a method for removal of anti-CXCR antibodies from isolated blood. The skilled person will readily acknowledge that the sample in which the presence and/or level of anti-CXCR antibodies is determined may be the same as the isolated blood from which the anti-CXCR antibodies are removed. However, in a preferred embodiment the anti-CXCR antibodies are determined in a blood sample of a subject and anti-CXCR antibodies are than removed from isolated blood of said patient, the isolated blood not being said blood sample. The gist of the invention is the correlation of the presence and/or increased levels of anti-CXCR antibodies and the presence of an autoimmune disease, i.e. a disease in which the immune system of the subject directs its activity against the own body or tissues thereof. Hence, the methods as outlined herein above, in particular the method for diagnosing the autoimmune disease, gives the guidance that the immune system is active against own tissues. Hence, it is indicated to remove antibodies from the blood of the patient . Hence, the method for removal of anti-CXCR antibodies basically relates to two steps, i.e. the determining step and the removal step. It will be acknowledged that the removal step may comprise the removal of more than only anti-CXCR antibodies, e.g. the removal of a plurality of immunoglobulins. A specific removal of anti-CXCR antibodies is not necessary. Hence, in one embodiment of the method for removal of anti-CXCR antibodies the anti-CXCR antibodies are removed through removal of a plurality of antibodies comprising anti-CXCR antibodies, preferably the removal comprises the removal of all IgA-antibodies, and/or all IgG-antibodies and/or all IgM-antibody from said isolated blood, preferably all or a plurality of IgG antibodies, e.g. IgG1, IgG2, IgG3 and IgG4. In one embodiment of the present invention IgG antibodies are removed from isolated blood of a subject upon determination of an anti-CXCR antibody in a sample of said subject, preferably upon determination of anti-CXCR3 and/or anti-CXCR4 antibodies as disclosed herein. Removal of a plurality of antibodies is known in the art and also referred to as immunoadsorption.

Chemokine receptor CXCR3 (Gene ID: 2833) is a Gαᵢ, protein-coupled receptor in the CXC chemokine receptor family. Other names for CXCR3 are G protein-coupled receptor 9 (GPR9) and CD183, C-X-C chemokine receptor type 3, CXC-R3, CXCR-3, G protein-coupled receptor 9, IP-10 receptor, IP10 receptor, Mig receptor, chemokine (C-X-C) receptor 3, and interferon-inducible protein 10 receptor. There are two variants of CXCR3: CXCR3-A binds to the CXC chemokines CXCL9 (MIG), CXCL10 (IP-10), and CXCL11 (I-TAC) (Clark-Lewis I, Mattioli I, Gong JH, Loetscher P (2003). "Structure-function relationship between the human chemokine receptor CXCR3 and its ligands". J. Biol. Chem. 278 (1): 289-295), whereas CXCR3-B can also bind to CXCL4 in addition to CXCL9, CXCL10, and CXCL11 (Lasagni L, Francalanci M, Annunziato F, Lazzeri E, Giannini S, Cosmi L et al. (2003). "An Alternatively Spliced Variant of CXCR3 Mediates the Inhibition of Endothelial Cell Growth Induced by IP-10, Mig, and I-TAC, and Acts as Functional Receptor for Platelet Factor 4". J. Exp. Med. 197 (11): 1537-1549). CXCR3 is expressed primarily on activated T lymphocytes and NK cells (Qin S, Rottman JB, Myers P, Kassam N, Weinblatt M, Loetscher M et al. (1998). "The chemokine receptors CXCR3 and CCR5 mark subsets of T cells associated with certain inflammatory reactions". J. Clin. Invest. 101 (4): 746-754), and some epithelial cells. CXCR3 and CCR5 are preferentially expressed on Th1 cells, whereas Th2 cells favor the expression of CCR3 and CCR4. CXCR3 ligands that attract Th1 cells can concomitantly block the migration of Th2 cells in response to CCR3 ligands, thus enhancing the polarization of effector T cell recruitment. Binding of CXCL9, CXCL10, and CXCL11 to CXCR3 is able to elicit increases in intracellular Ca2++ levels and activate phosphoinositide 3-kinase and mitogen-activated protein kinase (MAPK).[4] Detailed signaling pathway has not yet been established, but may include the same enzymes that were identified in the signaling cascade induced by other chemokine receptors. CXCR3 is able to regulate leukocyte trafficking. Binding of chemokines to CXCR3 induces various cellular responses, most notably integrin activation, cytoskeletal changes and chemotactic migration. CXCR3-ligand interaction attracts Th1 cells and promotes Th1 cell maturation. As a consequence of chemokine-induced cellular desensitization (phosphorylation-dependent receptor internalization), cellular responses are typically rapid and short in duration. Cellular responsiveness is restored after dephosphorylation of intracellular receptors and subsequent recycling to the cell surface. A hallmark of CXCR3 is its prominent expression in *in vitro* cultured effector/memory T cells, and in T cells present in many types of inflamed tissues. In addition, CXCL9, CXCL10 and CXCL11 are commonly produced by local cells in inflammatory lesion, suggesting that CXCR3 and its chemokines participate in the recruitment of inflammatory cells. Additionally, CXCR3 has been implicated in wound healing (Yates CC, Whaley D, Kulasekeran P, Hancock WW, Lu B, Bodnar R et al. (2007). "Delayed and Deficient Dermal Maturation in Mice Lacking the CXCR3 ELR-Negative CXC Chemokine Receptor". Am. J. Pathol. 171 (2): 484-495). CXCR3 has been implicated in atherosclerosis, multiple sclerosis, pulmonary fibrosis, type 1 diabetes, autoimmune myasthenia gravis, nephrotoxic nephritis, acute cardiac allograft rejection and possibly Celiac Disease.

"Anti-CXCR antibody" and "CXCR antibody" are used interchangeably herein and refer to antibodies specifically binding a CXC chemokine receptor or fragments thereof, preferably autoimmune antibodies. In a particularly preferred embodiment the anti-CXCR antibody is an antibody directed against an isoform of CXCR3, i.e. being an anti-CXCR3 antibody. The anti-CXCR3 antibody binds specifically to CXCR3 or in doing so shows specific immuno reactivity when the anti-CXCR3 antibody assumes its function in a binding reaction in the presence of a heterogeneous population of CXCR3s or fragments thereof, thereby allowing a conclusion whether the CXCR3 or another biological structure is present. Under the present conditions of an immunoassay, the above-mentioned anti-CXCR3 antibodies will preferably bind to a specific portion of the CXCR3, e.g. the extra cellular portion, while no significant binding to other proteins present in the sample will take place. It preferably binds to a portion common to the isoforms of CXCR3 (e.g. SEQ ID NO:1 or 2). A preferred portion is exemplified in SEQ ID NO:5. In a preferred embodiment the anti-CXCR3 antibodies are selected from the group of antibodies consisting of IgG-antibodies and IgM-antibodies, preferably an IgG antibody, e.g. IgG1, IgG2, IgG3 and IgG4. In a certain preferred embodiment total IgG type anti-CXCR3 antibodies are to be detected in the methods of the present invention.

In connection with the present invention, the naturally occurring receptor as well as all modifications, mutants or derivatives of the CXC chemokine receptor 3 (CXCR3) can be used. Similarly, an CXCR3 produced by means of recombinant techniques, which receptor includes amino acid modifications, such as inversions, deletions, insertions, additions etc. can be used according to the invention provided that this part of the essential function of the CXCR3 is present, namely the capability of binding antibodies. The CXCR3 being used may also comprise exceptional amino acids and/or modifications of such as alkylation, oxidation, thiol-modification, denaturation, oligomerization and the like. The receptor can also be synthesized by chemical means. According to the invention the CXCR3 particularly can be a protein and/or peptide or a fusion protein, which in addition to other proteins, peptides or fragments thereof, includes the CXCR3 as a whole or in part. Using conventional methods, peptides or polypeptides of the CXCR3 which have functionally analogs, analogous properties can be determined by those skilled in the art. For example such polypeptides or peptides have 50 to 60%, 70% or 80%, preferably 90%, more preferably 95%, and most preferably 98% sequence identity to peptides identified as CXCR3, and said homology can be determined, e.g. by means of Smith-Waterman homology search algorithm, using the MPFRCH Programme (Oxford Molecular), for example.

An "immunogenic peptide" or "antigenic peptide" as used herein interchangeably is a portion of a CXCR3 protein that is recognized (i.e., specifically bound) by the anti-CXCR3 antibodies in the sample of the patient to be detected. Such immunogenic peptides generally comprise at least 5 amino acid residues, more preferably at least 10, and still more preferably at least 20 amino acid residues of CXCR3. However, they may also comprise at least 30, 40, 50, 60, 70, 80, 90, 100, 110, 120, 130, 140 or 150 amino acid residues. In a preferred embodiment the immunogenic peptide is at least 112 amino residues in length. The term "immunogenic peptide" of an CXCR3 as used in the present invention, comprises also molecules differing from the original sequence by deletion(s), insertion(s), substitution(s) and/or other modifications well known in the prior art and/or comprising a fragment of the original amino acid molecule, the CXCR3 still exhibiting the properties mentioned above. Also included are allele variants and modifications. Methods of producing the above changes in the amino acid sequence are well known to those skilled in the art and have been described in the standard textbooks of molecular biology, *see,* e.g., Sambrook *et al.; supra.* Those skilled in the art will also be able to determine whether a CXCR3, thus, modified still has the properties mentioned above. Database entries exist in several well known Databases. When refereeing to the amino acid sequence of CXCR3 any amino acid sequence known is meant, particularly those disclosed in common databases, preferably of human origin. The CXCR3 may be glycosylated *in vivo.* In the present specification all of the above illustrated modifications of the CXCR3 will be referred to as "functionally analogous peptides or proteins" in brief. Preferably the CXCR3 is selected from the group consisting of CXCR3-A and CXCR3-B. CXCR3 preferably has a sequence selected from the group consisting of SEQ ID NO: 1 (CXCR3-A), and SEQ ID NO:2 (CXCR3-B). In a particular preferred embodiment the CXCR3 as used herein refers to an immunogenic peptide fragment of CXCR3. It is preferred that the immunogenic peptide comprises a sequence common to the outlined isoforms. In a particular preferred embodiment CXCR3 refers to a polypeptide comprising a sequence having at least 50 %, 60%, 70% or 80%, preferably at least 90%, more preferably at least 95%, and most preferably at least 98% sequence identity to the sequence of SEQ ID NO:5, more preferably it is identical to the sequence.

In the context of the immunoassays of the present invention the "CXCR3" may be present in its natural cellular environment and can be used together with the material associated with CXCR3 in its natural state as well as in isolated form with respect to its primary, secondary and tertiary structures. CXCR3s are well known to those skilled in the art. The receptor is preferably used in isolated form, i.e. essentially free of other proteins, lipids, carbohydrates or other substances naturally associated with CXCR3. "Essentially free of' means that the receptor is at least 75%, preferably at least 85%, more preferably at least 95% and especially preferably at least 99% free of other proteins, lipids, carbohydrates or other substances naturally associated with the receptor. However, the CXCR3 may also be used as a membrane extract from cells expressing the vector naturally or my recombinant means.

In connection with the present invention, the naturally occurring receptor as well as all modifications, mutants, immunogenic peptides or derivatives of the CXCR3 can be used. Similarly, a CXCR3 produced by means of recombinant techniques, which includes amino acid modifications, such as inversions, deletions, insertions, additions etc. can be used according to the invention provided that this part of the essential function of the CXCR3 is present, namely the capability of binding antibodies. The CXCR3 being used may also comprise exceptional amino acids and/or modifications of such as alkylation, oxidation, thiol-modification, denaturation, oligomerization and the like. The CXCR3 can also be synthesized by chemical means. According to the invention the CXCR3 particularly can be a protein and/or peptide or a fusion protein, which in addition to other proteins, peptides or fragments thereof, includes a CXCR3 as a whole or in part. Using conventional methods, peptides or polypeptides of the CXCR3 which have functionally analogs, analogous properties can be determined by those skilled in the art. For example such polypeptides or peptides have at least 50 %, 60%, 70% or 80%, preferably at least 90%, more preferably at least 95%, and most preferably at least 98% sequence identity to CXCR3, preferably to CXCR3-A or CXCR3-B, more preferred to the sequence selected from the group consisting of SEQ ID NO:1, and SEQ ID NO:2, particularly preferred the polypeptides or peptides of CXCR3 comprise a sequence having at least 50 %, 60%, 70% or 80%, preferably at least 90%, more preferably at least 95%, and most preferably at least 98% sequence identity to polypeptides or peptides have at least 50 %, 60%, 70% or 80%, preferably at least 90%, more preferably at least 95%, and most preferably at least 98% sequence identity toSEQ ID NO:5.

The antibodies to be detected or determined according to the present invention are directed against CXCR. In a particularly preferred embodiment the antibodies to be detected or the level of which is to be determined are directed against a CXCR3, preferably selected from the group consisting of CXCR3-A and CXCR3-B, particularly preferred both, as the examples use a sequence common to both isoforms. This means that the antibodies specifically bind a CXCR3. Specific binding of an antibody normally occurs via binding of a binding site of the antigen. The antibodies of the present invention are those specifically binding to CXCR3 or immunogenic fragments thereof. This binding may occur via recognition of sequence or structural epitopes. The skilled person is aware of methods of how to determine specific epitopes, e.g. fragments of the antigen CXCR3, which are recognized and bound by the antibodies to be determined. Fragments of CXCR3 binding to the auto antibodies are called immunogenic or antigenic fragments. Methods for determining fragments of an antigen binding the antibody are described in several publications (e.g. Gershoni, JM; Roitburd-Berman, A; Siman-Tov, DD; Tarnovitski Freund, N; Weiss, Y (2007). "Epitope mapping: The first step in developing epitope-based vaccines". BioDrugs 21 (3): 145-56; Westwood, MR; Hay, FC (2001). Epitope Mapping: a practical approach. Oxford, Oxfordshire: Oxford University Press. ISBN 0-19-963652-4; Flanagan et al. (2011), "Mapping Epitopes with H/D-Ex Mass Spec". Genetic Engineering and Biotechnology news; 31(1); Gaseitsiwe, S.; Valentini, D.; Mahdavifar, S.; Reilly, M.; Ehrnst, A.; Maeurer, M. (2009) "Peptide Microarray-Based Identification of Mycobacterium tuberculosis Epitope Binding to HLA-DRB1*0101, DRB1*1501, and DRB1*0401". Clinical and Vaccine Immunology 17 (1): 168-75; Linnebacher, Michael; Lorenz, Peter; Koy, Cornelia; Jahnke, Annika; Born, Nadine; Steinbeck, Felix; Wollbold, Johannes; Latzkow, Tobias et al. (2012). "Clonality characterization of natural epitope-specific antibodies against the tumor-related antigen topoisomerase IIa by peptide chip and proteome analysis: A pilot study with colorectal carcinoma patient samples" Analytical and Bioanalytical Chemistry 403 (1): 227-38; Cragg, M. S. (2011). "CD20 antibodies: Doing the time warp". Blood 118 (2): 219-20; Banik, Soma S. R.; Doranz, Benjamin J. (2010). "Mapping Complex Antibody Epitopes". Genetic Engineering and Biotechnology News 3 (2): 25-8; and Paes, Cheryl; Ingalls, Jada; Kampani, Karan; Sulli, Chidananda; Kakkar, Esha; Murray, Meredith; Kotelnikov, Valery; Greene, Tiffani A. et al. (2009). "Atomic-Level Mapping of Antibody Epitopes on a GPCR". Journal of the American Chemical Society 131 (20): 6952-4).

In context with the present invention anti-CXCR antibodies are understood as any immunoglobulin specifically recognizing/binding to a CXCR. The antibody in a preferred embodiment binds any CXCR3 variant, preferably to any CXCR3-A or CXCR3-B variant, more preferably to a sequence selected from the group consisting of SEQ ID NO:1, and SEQ ID NO:2, particularly preferred the CXCR variant comprises the sequence according to SEQ ID NO:5.

The present invention hence also relates to a method for diagnosis an autoimmune disease in a subject wherein, presence or absence of an anti-CXC chemokine receptor 3 (CXCR3) antibody is detected in a sample from the subject diagnosed, and wherein the presence of an anti-CXCR3 antibody is indicative of the disease. In one embodiment the autoimmune disease is preferably a connective tissue autoimmune disease connective tissue, preferably selected from the group consisting of systemic lupus erythematosus, rheumatoid arthritis, scleroderma, Sjögren's syndrome, mixed connective tissue disease, Graft versus Host Disease (cGvHD), chronic Graft versus Host Disease (cGvHD) systemic sclerosis (SSc); dermatomyositis (DM), polymyositis (PM), anti-synthetase syndrome, and psoriatic arthritis, more preferably systemic lupus erythematosus. As outlined herein, presence of antibodies may also be determined by comparison to a control level. Hence, in one embodiment the presence or absence of the anti-CXCR3 antibody is detected by the following steps: (i) determining the level of anti-CXCR3 antibodies in a sample of the subject to be diagnosed; and (ii) comparing the determined level to an anti-CXCR3 antibody control level derived from a subject without an autoimmune disease; wherein an increased level of anti-CXCR3 antibody in the sample of the subject to be diagnosed as compared to the anti-CXCR3 antibody control level denotes the presence of an anti-CXCR3 antibody. As outlined in great detail herein, ratios of the determined level compared to the control level may be applied. Hence, in one embodiment of the method for diagnosis of an autoimmune disease a level in the sample of the subject to be diagnosed of more than 1.1 ok fold as compared to the control level from subjects without an autoimmune disease is indicative of the presence of an autoimmune disease in the subject to be diagnosed, preferably a level in the sample of the subject to be diagnosed of more than 1.8 ok fold as compared to the control level from subjects without an autoimmune disease is indicative of the presence of an autoimmune disease in the subject to be diagnosed, more preferably a level in the sample of the subject to be diagnosed of more than 2 ok fold as compared to the control level from subjects without an autoimmune disease is indicative of the presence of an autoimmune disease in the subject to be diagnosed. The autoimmune disease is preferably selected from the embodiments disclosed herein, particularly preferred the autoimmune disease is systemic lupus erythematosus.

As also outlined herein, levels of anti-CXCR3 antibodies in samples of patient/subjects may be measured in terms of units/ml. In a preferred embodiment of the method for diagnosing according to the present invention a level of anti-CXCR3 antibodies in the sample of the patient to be diagnosed of more than 3 units/ml is indicative of graft intolerance, preferably a level of more than 3.5 units/ml, even more preferably of more than 5 units/ml. As outlined herein, the skilled person is aware of methods for determining units/ml of an antibody. However, in a preferred embodiment the units according to the present invention are determined using the unit-standards or unit solutions as outlined in greater detail herein. Furthermore, the invention relates to a method for determining disease activity of an autoimmune disease comprising the steps of: (i) determining the level of anti-CXCR3 antibodies in a sample from a subject to be diagnosed, (ii) comparing the determined level in the sample to either one or both of a first and second anti-CXCR3 antibody control level, a) wherein the first anti-CXCR3 antibody control level is derived from subjects suffering an inactive autoimmune disease; and b) wherein the second anti-CXCR3 antibody control level derived from subjects suffering an active autoimmune disease; wherein a increased level in the sample from the subject to be diagnosed as compared to the first anti-CXCR3 antibody control level and/or an equal level as compared to the second anti-CXCR3 antibody control level is indicative of the presence of an active autoimmune disease in the subject to be diagnosed, and wherein an decreased level in the sample from the subject to be diagnosed as compared to the second anti-CXCR3 antibody control level and/or an equal level as compared to the first anti-CXCR3 antibody control level is indicative of the presence of an inactive autoimmune disease. The autoimmune disease the activity status of which is to be diagnosed is preferably systemic lupus erythematosus.

Further, the skilled person with the disclosure of the present application and in particular the data presented in the examples will acknowledge that the present invention also provides for a method of diagnosis the grade of an autoimmune disease, e.g. SLE. A scoring nowadays is performed using the BILAG-2004 activity index as published (see Isenberg DA et al. BILAG 2004. Development and initial validation of an updated version of the British Isles Lupus Assessment Group's disease activity index for patients with systemic lupus erythematous. Rheumatology. 2005; 44:902-906; Yee CS, et al. Revised British Isles Lupus Assessment Group 2004 index: a reliable tool for assessment of systemic lupus erythematosus activity. Arthritis Rheum. 2006; 54:3300-3305; and Yee CS, et al. British Isles Lupus Assessment Group 2004 index is valid for assessment of disease activity in systemic lupus erythematosus. Arthritis Rheum. 2007; 56:4113-4119; all of which are incorporated herein by reference). The scoring implies a rating into mild, moderate and severe activity. The inventors that the higher the levels of the anti-CXCR3 antibodies the more severe the activity of the autoimmune disease. Hence, the method for determining disease activity of an autoimmune disease is preferably a method for determining the activity status of an autoimmune disease comprising the steps of: (i) determining the level of anti-CXCR3 antibodies in a sample from a subject to be diagnosed, (ii) comparing the determined level in the sample to either one or two or all three of a first, second and third anti-CXCR3 antibody control level, a) wherein the first anti-CXCR3 antibody control level is derived from subjects suffering an autoimmune disease with mild activity; and b) wherein the second anti-CXCR3 antibody control level derived from subjects suffering an autoimmune disease with moderate activity; and c) wherein the third anti-CXCR3 antibody control level derived from subjects suffering an autoimmune disease with severe activity; wherein an equal level in the sample from the subject to be diagnosed as compared to the first anti-CXCR3 antibody control level and/or an decreased level as compared to the second and/or third anti-CXCR3 antibody control level is indicative of the presence of an active autoimmune disease with mild activity in the subject to be diagnosed, and wherein an increased level in the sample from the subject to be diagnosed as compared to the first anti-CXCR3 antibody control level and a decreased level as compared to the third anti-CXCR3 antibody control level; and/or an equal level as compared to the second anti-CXCR3 antibody control level is indicative of the presence of an active autoimmune disease with moderate activity; and wherein an equal or increased level in the sample from the subject to be diagnosed as compared to the third anti-CXCR3 antibody control level and/or an increased level as compared to the first and/or second anti-CXCR3 antibody control level is indicative of the presence of an active autoimmune disease with severe activity in the subject to be diagnosed. Preferred anti-CXCR antibodies are those binding to a polypeptide comprising a sequence having at least 95% identity to the sequence of SEQ ID NO:5. The autoimmune disease is preferably SLE. The definition of the activity status of SLE is preferably as disclosed in above references for BILAG2004.

Furthermore, the invention relates to a method for treating an autoimmune disease in a subject, wherein the subject is treated upon diagnosing an autoimmune disease or an autoimmune disease with active autoimmune disease with a method according to the present invention.

Moreover, the invention relates to an immunosuppressant for use in the treatment of an autoimmune disease in a subject, wherein the immunosuppressant is administered to the subject upon determination of the presence or increased levels of anti-CXCR3 antibodies in a sample of said subject. Preferably presence of the antibodies was performed as outlined for the methods of diagnosis of an autoimmune disease or an active autoimmune disease according to the present invention. The disease is preferably systemic lupus erythematosus. The immunosuppressant being preferably selected from the group consisting of cyclophosphamides, glucocorticoids, and Rituximab.

As outlined herein, the detection of the anti-CXCR antibodies may preferably be performed by an immunoassay. In a preferred embodiment of the immunoassay according to the present invention anti-CXCR3 antibodies are to bed detected. Hence, the invention also pertains to an immunoassay method for detecting an anti-CXCR3 antibody in a sample from a subject, comprising the steps of: (a) contacting the sample suspected of comprising an anti-CXCR3 antibody with a CXCR or an immunogenic peptide fragment thereof under conditions allowing for the formation of a complex between the anti-CXCR3 antibody with CXCR3 or the peptide fragment thereof; (b) detecting the complex. Embodiments of the immunoassay for detecting an anti-CXCR3 antibody are as outlined herein for the immunoassay detecting anti-CXCR antibody in general, *supra.* Embodiments of CXCR3 and immunogenic fragments thereof are also outlined herein above.

Furthermore, the invention relates to the use of a CXCR3 protein or an immunogenic peptide thereof for the diagnosis of an autoimmune disease.

The invention also pertains the use of a kit in for the diagnosis of an autoimmune disease, wherein the kit comprises a CXCR3 protein or an immunogenic peptide thereof.
The invention likewise relates to a method for the removal of anti-CXCR3 antibodies from isolated blood, (i) wherein in a first step the presence or absence of an anti-CXCR3 antibody is determined in a blood sample from a subject to be diagnosed for an autoimmune disease, (ii) wherein upon determining the presence of an anti-CXCR3 antibody the antibody is removed from isolated blood of the subject. Embodiments of the method for the removal of anti-CXCR antibodies from isolated blood as outlined herein in great detail, apply, *mutatis mutandis,* also the method for removal of anti-CXCR3 antibodies from isolated blood.

The invention also relates to a kit for detecting an anti-CXCR3 antibody comprising a CXCR3 protein or an immunogenic peptide thereof. Further embodiments for kits are outlined herein in great detail and apply also to the kit for detecting an anti-CXCR3 antibody.

CXC chemokine receptor type 4 (CXCR-4; NP_001008540) also known as fusin or CD184 (cluster of differentiation 184) is a protein that in humans is encoded by the CXCR4 gene (Moriuchi M, Moriuchi H, Turner W, Fauci AS (November 1997). "Cloning and analysis of the promoter region of CXCR4, a coreceptor for HIV-1 entry". J. Immunol. 159 (9): 4322-4329; and Caruz A, Samsom M, Alonso JM, Alcami J, Baleux F, Virelizier JL et al. (April 1998). "Genomic organization and promoter characterization of human CXCR4 gene". FEBS Lett. 426 (2): 271-278). CXCR-4 is an alpha-chemokine receptor specific for stromal-derived-factor-1 (SDF-1 also called CXCL12), a molecule endowed with potent chemotactic activity for lymphocytes. CXCR4 is upregulated during the implantation window in natural and hormone replacement therapy cycles in the endometrium, producing, in presence of a human blastocyst, a surface polarization of the CXCR4 receptors suggesting that this receptor is implicated in the adhesion phase of human implantation. CXCR4's ligand SDF-1 is known to be important in hematopoietic stem cell homing to the bone marrow and in hematopoietic stem cell quiescence. Until recently, SDF-1 and CXCR4 were believed to be a relatively monogamous ligand-receptor pair (other chemokines are promiscuous, tending to use several different chemokine receptors). Recent evidence demonstrates ubiquitin is also a natural ligand of CXCR4 (Saini V, Marchese A, Majetschak M (May 2010). "CXC chemokine receptor 4 is a cell surface receptor for extracellular ubiquitin". J. Biol. Chem. 285 (20): 15566-15576).

Ubiquitin is a small (76-amino acid) protein highly conserved among eukaryotic cells. It is best known for its intracellular role in targeting ubiquitylated proteins for degradation via the ubiquitin proteasome system. Evidence in numerous animal models suggests ubiquitin is antiinflammatory immune modulator and endogenous opponent of proinflammatory damage associated molecular pattern molecules (Majetschak M (August 2010). "Extracellular ubiquitin: immune modulator and endogenous opponent of damage-associated molecular pattern molecules". J. Leukoc. Biol. 89 (2): 205-219). It is speculated this interaction may be through CXCR4 mediated signalling pathways. CXCR4 is present in newly generated neurons during embryogenesis and adult life where it plays a role in neuronal guidance. The levels of the receptor decrease as neurons mature. Drugs that block the CXCR4 receptor appear to be capable of "mobilizing" hematopoietic stem cells into the bloodstream as peripheral blood stem cells. Peripheral blood stem cell mobilization is very important in hematopoietic stem cell transplantation (as a recent alternative to transplantation of surgically harvested bone marrow) and is currently performed using drugs such as G-CSF. G-CSF is a growth factor for neutrophils (a common type of white blood cells), and may act by increasing the activity of neutrophil-derived proteases such as neutrophil elastase in the bone marrow leading to proteolytic degradation of SDF-1. Plerixafor (AMD3100) is a drug, recently approved for routine clinical use,[9] which directly blocks the CXCR4 receptor. It is a very efficient inducer of hematopoietic stem cell mobilization in animal and human studies. It has been associated with WHIM syndrome.[10] WHIM like mutations in CXCR4 were recently identified in patients with Waldenstrom's macroglobulinemia, a B-cell malignancy (Hunter ZR, Xu L, Yang G, Zhou Y, Liu X, Cao Y et al. (2014). "The genomic landscape of Waldenstrom macroglobulinemia is characterized by highly recurring MYD88 and WHIM-like CXCR4 mutations, and small somatic deletions associated with B-cell lymphomagenesis". Blood 123 (11): 1637-46).

While CXCR4's expression is low or absent in many healthy tissues, it was demonstrated to be expressed in over 23 types of cancer, including breast cancer, ovarian cancer, melanoma, and prostate cancer. Expression of this receptor in cancer cells has been linked to metastasis to tissues containing a high concentration of CXCL12, such as lungs, liver and bone marrow (Sun X, Cheng G, Hao M, Zheng J, Zhou X, Zhang J et al. (December 2010). "CXCL12 / CXCR4 / CXCR7 chemokine axis and cancer progression". Cancer Metastasis Rev. 29 (4): 709-722). "Anti-CXCR antibody" and "CXCR antibody" are used interchangeably herein and refer to antibodies specifically binding CXC chemokine receptor or fragments thereof, preferably autoimmune antibodies. In a particularly preferred embodiment the anti-CXCR antibody is an antibody directed against CXCR4, i.e. being an anti-CXCR4 antibody. The anti-CXCR4 antibody binds specifically to the CXCR4 or in doing so shows specific immuno reactivity when the anti- CXCR4 antibody assumes its function in a binding reaction in the presence of a heterogeneous population of CXCR4 or fragments thereof, thereby allowing a conclusion whether the CXCR4 or another biological structure is present. Under the present conditions of an immunoassay, the above-mentioned anti-CXCR4 antibodies will preferably bind to a specific portion of the CXCR4, e.g. the extra cellular portion, while no significant binding to other proteins present in the sample will take place. In a preferred embodiment the anti-CXCR4 antibodies are selected from the group of antibodies consisting of IgG-antibodies and IgM-antibodies, preferably an IgG antibody, e.g. IgG1, IgG2, IgG3 and IgG4. In a certain preferred embodiment total IgG type anti-CXCR3 antibodies are to be detected in the methods of the present invention.

In connection with the present invention, the naturally occurring receptor as well as all modifications, mutants or derivatives of the CXC chemokine receptor 4 (CXCR4) can be used. Similarly, a CXCR4 produced by means of recombinant techniques, which receptor includes amino acid modifications, such as inversions, deletions, insertions, additions etc. can be used according to the invention provided that this part of the essential function of the CXCR4is present, namely the capability of binding antibodies. The CXCR4 being used may also comprise exceptional amino acids and/or modifications of such as alkylation, oxidation, thiol-modification, denaturation, oligomerization and the like. The receptor can also be synthesized by chemical means. According to the invention the CXCR4 particularly can be a protein and/or peptide or a fusion protein, which in addition to other proteins, peptides or fragments thereof, includes the CXCR4 as a whole or in part. Using conventional methods, peptides or polypeptides of the CXCR4 which have functionally analogs, analogous properties can be determined by those skilled in the art. For example such polypeptides or peptides have 50 to 60%, 70% or 80%, preferably 90%, more preferably 95%, and most preferably 98% homology to peptides identified as CXCR4, and said homology can be determined, e.g. by means of Smith-Waterman homology search algorithm, using the MPFRCH Programme (Oxford Molecular), for example.

An "immunogenic peptide" or "antigenic peptide" as used herein interchangeably is a portion of a CXCR4 protein that is recognized (i.e., specifically bound) by the anti-CXCR4 antibodies in the sample of the patient to be detected. Such immunogenic peptides generally comprise at least 5 amino acid residues, more preferably at least 10, and still more preferably at least 20 amino acid residues of CXCR4. However, they may also comprise at least 30, 40, 50, 60, 70, 80, 90, 100, 110, 120, 130, 140 or 150 amino acid residues. In a preferred embodiment the immunogenic peptide is at least 112 amino residues in length. The term "immunogenic peptide" of an CXCR4 as used in the present invention, comprises also molecules differing from the original sequence by deletion(s), insertion(s), substitution(s) and/or other modifications well known in the prior art and/or comprising a fragment of the original amino acid molecule, the CXCR4 still exhibiting the properties mentioned above. Also included are allele variants and modifications. Methods of producing the above changes in the amino acid sequence are well known to those skilled in the art and have been described in the standard textbooks of molecular biology, *see,* e.g., Sambrook *et al.; supra.* Those skilled in the art will also be able to determine whether a CXCR4, thus, modified still has the properties mentioned above. Database entries exist in several well known Databases. When refereeing to the amino acid sequence of CXCR4 any amino acid sequence known is meant, particularly those disclosed in common databases, preferably of human origin. The CXCR4 may be glycosylated *in vivo.* In the present specification all of the above illustrated modifications of the CXCR4 will be referred to as "functionally analogous peptides or proteins" in brief. Preferably the CXCR4 has the sequence of SEQ ID NO:3.

In the context of the immunoassays of the present invention the "CXCR4" may be present in its natural cellular environment and can be used together with the material associated with CXCR4 in its natural state as well as in isolated form with respect to its primary, secondary and tertiary structures. CXCR4s are well known to those skilled in the art. The receptor is preferably used in isolated form, i.e. essentially free of other proteins, lipids, carbohydrates or other substances naturally associated with CXCR4. "Essentially free of" means that the receptor is at least 75%, preferably at least 85%, more preferably at least 95% and especially preferably at least 99% free of other proteins, lipids, carbohydrates or other substances naturally associated with the receptor. However, the CXCR4 may also be used as a membrane extract from cells expressing the vector naturally or my recombinant means.

In connection with the present invention, the naturally occurring receptor as well as all modifications, mutants, immunogenic peptides or derivatives of the CXCR4 can be used. Similarly, a CXCR4 produced by means of recombinant techniques, which includes amino acid modifications, such as inversions, deletions, insertions, additions etc. can be used according to the invention provided that this part of the essential function of the CXCR4 is present, namely the capability of binding antibodies. The CXCR4 being used may also comprise exceptional amino acids and/or modifications of such as alkylation, oxidation, thiol-modification, denaturation, oligomerization and the like. The CXCR4 can also be synthesized by chemical means. According to the invention the CXCR4 particularly can be a protein and/or peptide or a fusion protein, which in addition to other proteins, peptides or fragments thereof, includes a CXCR4 protein of e.g. isoform a or b as a whole or in part. Using conventional methods, peptides or polypeptides of the CXCR4 which have functionally analogs, analogous properties can be determined by those skilled in the art. For example such polypeptides or peptides have at least 50 %, 60%, 70% or 80%, preferably at least 90%, more preferably at least 95%, and most preferably at least 98% sequence identity to CXCR4, preferably to the sequence of SEQ ID NO:3 or SEQ ID NO:4, preferably SEQ ID NO:4. It is further preferred that the polypeptide or peptides are a fragment of CXCR4, e.g. of CXCR4 isoform a or isoform b. The fragments preferably comprise a sequence having at least 50 %, 60%, 70% or 80%, preferably at least 90%, more preferably at least 95%, and most preferably at least 98% sequence identity to SEQ ID NO:6, e.g. the fragment has the sequence of SEQ ID NO:6.

The antibodies to be detected or determined according to the present invention are directed against CXCR. In a particularly preferred embodiment the antibodies to be detected or the level of which is to be determined are directed against CXCR4, preferably isoform b. This means that the antibodies specifically bind a CXCR4. Specific binding of an antibody normally occurs via binding of a binding site of the antigen. The antibodies of the present invention are in one embodiment preferably those specifically binding to CXCR4 or immunogenic fragments thereof. This binding may occur via recognition of sequence or structural epitopes. The skilled person is aware of methods of how to determine specific epitopes, e.g. fragments of the antigen CXCR4, which are recognized and bound by the antibodies to be determined. Fragments of CXCR4 binding to the auto antibodies are called immunogenic or antigenic fragments. Methods for determining fragments of an antigen binding the antibody are described in several publications (e.g. Gershoni, JM; Roitburd-Berman, A; Siman-Tov, DD; Tarnovitski Freund, N; Weiss, Y (2007). "Epitope mapping: The first step in developing epitope-based vaccines". BioDrugs 21 (3): 145-56; Westwood, MR; Hay, FC (2001). Epitope Mapping: a practical approach. Oxford, Oxfordshire: Oxford University Press. ISBN 0-19-963652-4; Flanagan et al. (2011), "Mapping Epitopes with H/D-Ex Mass Spec". Genetic Engineering and Biotechnology news; 31(1); Gaseitsiwe, S.; Valentini, D.; Mahdavifar, S.; Reilly, M.; Ehrnst, A.; Maeurer, M. (2009) "Peptide Microarray-Based Identification of Mycobacterium tuberculosis Epitope Binding to HLA-DRB1*0101, DRB1*1501, and DRB1*0401". Clinical and Vaccine Immunology 17 (1): 168-75; Linnebacher, Michael; Lorenz, Peter; Koy, Cornelia; Jahnke, Annika; Born, Nadine; Steinbeck, Felix; Wollbold, Johannes; Latzkow, Tobias et al. (2012). "Clonality characterization of natural epitope-specific antibodies against the tumor-related antigen topoisomerase IIa by peptide chip and proteome analysis: A pilot study with colorectal carcinoma patient samples" Analytical and Bioanalytical Chemistry 403 (1): 227-38; Cragg, M. S. (2011). "CD20 antibodies: Doing the time warp". Blood 118 (2): 219-20; Banik, Soma S. R.; Doranz, Benjamin J. (2010). "Mapping Complex Antibody Epitopes". Genetic Engineering and Biotechnology News 3 (2): 25-8; and Paes, Cheryl; Ingalls, Jada; Kampani, Karan; Sulli, Chidananda; Kakkar, Esha; Murray, Meredith; Kotelnikov, Valery; Greene, Tiffani A. et al. (2009). "Atomic-Level Mapping of Antibody Epitopes on a GPCR". Journal of the American Chemical Society 131 (20): 6952-4). In context with the present invention anti-CXCR antibodies are understood as any immunoglobulin specifically recognizing/binding to a CXCR. The antibody in a preferred embodiment binds any CXCR4 variant, e.g. isoform a or b, preferably to the sequence of SEQ ID NO:3 or of SEQ ID NO:4, preferably to a sequence at least 50 %, 60%, 70% or 80%, preferably at least 90%, more preferably at least 95%, and most preferably at least 98% identical to the sequence of SEQ ID NO:4. Furht.

The present invention hence also relates to a method for diagnosis an autoimmune disease in a subject wherein, presence or absence of an anti-CXC chemokine receptor 4 (CXCR4) antibody is detected in a sample from the subject diagnosed, and wherein the presence of an anti-CXCR3 antibody is indicative of the disease. In one embodiment the autoimmune disease is preferably a connective tissue autoimmune disease connective tissue, preferably selected from the group consisting of systemic lupus erythematosus, GvHD, cGvHD, rheumatoid arthritis, scleroderma, Sjögren's syndrome, mixed connective tissue disease, systemic sclerosis (SSc); dermatomyositis (DM), polymyositis (PM), anti-synthetase syndrome, and psoriatic arthritis, more preferably systemic lupus erythematosus. As outlined herein, presence of antibodies may also be determined by comparison to a control level. Hence, in one embodiment the presence or absence of the anti-CXCR4 antibody is detected by the following steps: (i) determining the level of anti-CXCR4 antibodies in a sample of the subject to be diagnosed; and (ii) comparing the determined level to an anti-CXCR4 antibody control level derived from a subject without an autoimmune disease; wherein an increased level of anti-CXCR4 antibody in the sample of the subject to be diagnosed as compared to the anti-CXCR4 antibody control level denotes the presence of an anti-CXCR4 antibody. As outlined in great detail herein, ratios of the determined level compared to the control level may be applied. Hence, in one embodiment of the method for diagnosis of an autoimmune disease a level in the sample of the subject to be diagnosed of more than 1.1 fold as compared to the control level from subjects without an autoimmune disease is indicative of the presence of an autoimmune disease in the subject to be diagnosed, preferably a level in the sample of the subject to be diagnosed of more than 1.8 fold as compared to the control level from subjects without an autoimmune disease is indicative of the presence of an autoimmune disease in the subject to be diagnosed, more preferably a level in the sample of the subject to be diagnosed of more than 2 fold as compared to the control level from subjects without an autoimmune disease is indicative of the presence of an autoimmune disease in the subject to be diagnosed. The autoimmune disease is preferably selected from the embodiments disclosed herein, particularly preferred the autoimmune disease is systemic lupus erythomatosus.

As also outlined herein, levels of anti-CXCR4 antibodies in samples of patient/subjects may be measured in terms of units/ml. In a preferred embodiment of the method for diagnosing according to the present invention a level of anti-CXCR4 antibodies in the sample of the patient to be diagnosed of more than 3 units/ml is indicative of graft intolerance, preferably a level of more than 3.5 units/ml, even more preferably of more than 5 units/ml. As outlined herein, the skilled person is aware of methods for determining units/ml of an antibody. However, in a preferred embodiment the units according to the present invention are determined using the unit-standards or unit solutions as outlined in greater detail herein.

Furthermore, the invention relates to a method for determining disease activity of an autoimmune disease comprising the steps of: (i) determining the level of anti-CXCR4 antibodies in a sample from a subject to be diagnosed, (ii) comparing the determined level in the sample to either one or both of a first and second anti-CXCR4 antibody control level, a) wherein the first anti-CXCR4 antibody control level is derived from subjects suffering an inactive autoimmune disease; and b) wherein the second anti-CXCR4 antibody control level derived from subjects suffering an active autoimmune disease; wherein a increased level in the sample from the subject to be diagnosed as compared to the first anti-CXCR4 antibody control level and/or an equal level as compared to the second anti-CXCR4 antibody control level is indicative of the presence of an active autoimmune disease in the subject to be diagnosed, and wherein an decreased level in the sample from the subject to be diagnosed as compared to the second anti-CXCR4 antibody control level and/or an equal level as compared to the first anti-CXCR4 antibody control level is indicative of the presence of an inactive autoimmune disease. The autoimmune disease the activity status of which is to be diagnosed is preferably systemic lupus erythematosus.

Further, the skilled person with the disclosure of the present application and in particular the data presented in the examples will acknowledge that the present invention also provides for a method of diagnosis the grade of an autoimmune disease, e.g. SLE. A scoring nowadays is performed using the BILAG-2004 activity index as published (see Isenberg DA et al. BILAG 2004. Development and initial validation of an updated version of the British Isles Lupus Assessment Group's disease activity index for patients with systemic lupus erythematous. Rheumatology. 2005; 44:902-906; Yee CS, et al. Revised British Isles Lupus Assessment Group 2004 index: a reliable tool for assessment of systemic lupus erythematosus activity. Arthritis Rheum. 2006; 54:3300-3305; and Yee CS, et al. British Isles Lupus Assessment Group 2004 index is valid for assessment of disease activity in systemic lupus erythematosus. Arthritis Rheum. 2007; 56:4113-4119; all of which are incorporated herein by reference). The scoring implies a rating into mild, moderate and severe activity. The inventors that the higher the levels of the anti-CXCR4 antibodies the more severe the activity of the autoimmune disease. Hence, the method for determining disease activity of an autoimmune disease is preferably a method for determining the activity status of an autoimmune disease comprising the steps of: (i) determining the level of anti-CXCR4 antibodies in a sample from a subject to be diagnosed, (ii) comparing the determined level in the sample to either one or two or all three of a first, second and third anti-CXCR4 antibody control level, a) wherein the first anti-CXCR4 antibody control level is derived from subjects suffering an autoimmune disease with mild activity; and b) wherein the second anti-CXCR4 antibody control level derived from subjects suffering an autoimmune disease with moderate activity; and c) wherein the third anti-CXCR4 antibody control level derived from subjects suffering an autoimmune disease with severe activity; wherein an equal level in the sample from the subject to be diagnosed as compared to the first anti-CXCR4 antibody control level and/or an decreased level as compared to the second and/or third anti-CXCR4 antibody control level is indicative of the presence of an active autoimmune disease with mild activity in the subject to be diagnosed, and wherein an increased level in the sample from the subject to be diagnosed as compared to the first anti-CXCR4 antibody control level and a decreased level as compared to the third anti-CXCR4 antibody control level; and/or an equal level as compared to the second anti-CXCR4 antibody control level is indicative of the presence of an active autoimmune disease with moderate activity; and wherein an equal or increased level in the sample from the subject to be diagnosed as compared to the third anti-CXCR4 antibody control level and/or an increased level as compared to the first and/or second anti-CXCR4 antibody control level is indicative of the presence of an active autoimmune disease with severe activity in the subject to be diagnosed. Preferred anti-CXCR4 antibodies are those binding to a polypeptide comprising a sequence having at least 95% identity to the sequence of SEQ ED NO:6. The autoimmune disease is preferably SLE. The definition of the activity status of SLE is preferably as disclosed in above references for BILAG2004.

Furthermore, the invention relates to a method for treating an autoimmune disease in a subject, wherein the subject is treated upon diagnosing an autoimmune disease or an autoimmune disease with active autoimmune disease with a method according to the present invention.

Moreover, the invention relates to an immunosuppressant for use in the treatment of an autoimmune disease in a subject, wherein the immunosuppressant is administered to the subject upon determination of the presence of anti-CXCR4 antibodies in a sample of said subject. Preferably presence of the antibodies was performed as outlined for the methods of diagnosis of an autoimmune disease or an active autoimmune disease according to the present invention. The disease is preferably systemic lupus erythematosus. The immunosuppressant being preferably selected from the group consisting of cyclophosphamides, glucocorticoids, and Rituximab.

As outlined herein, the detection of the anti-CXCR antibodies may preferably be performed by an immunoassay. In a preferred embodiment of the immunoassay according to the present invention anti-CXCR4 antibodies are to be detected. Hence, the invention also pertains to an immunoassay method for detecting an anti-CXCR4 antibody in a sample from a subject, comprising the steps of: (a) contacting the sample suspected of comprising an anti-CXCR4 antibody with a CXCR or an immunogenic peptide fragment thereof under conditions allowing for the formation of a complex between the anti-CXCR4 antibody with CXCR4 or the peptide fragment thereof; (b) detecting the complex. Embodiments of the immunoassay for detecting an anti-CXCR4 antibody are as outlined herein for the immunoassay detecting anti-CXCR antibody in general, *supra.* Embodiments of CXCR4 and immunogenic fragments thereof are also outlined herein above.

Furthermore, the invention relates to the use of a CXCR4 protein or an immunogenic peptide thereof for the diagnosis of an autoimmune disease.

The invention also pertains the use of a kit in for the diagnosis of an autoimmune disease, wherein the kit comprises a CXCR4 protein or an immunogenic peptide thereof.

The invention likewise relates to a method for the removal of anti-CXCR4 antibodies from isolated blood, (i) wherein in a first step the presence or absence of an anti-CXCR4 antibody is determined in a blood sample from a subject to be diagnosed for an autoimmune disease, (ii) wherein upon determining the presence of an anti-CXCR4 antibody the antibody is removed from isolated blood of the subject. Embodiments of the method for the removal of anti-CXCR antibodies from isolated blood as outlined herein in great detail, apply, *mutatis mutandis,* also the method for removal of anti-CXCR4 antibodies from isolated blood.

The invention also relates to a kit for detecting an anti-CXCR4 antibody comprising a CXCR4 protein or an immunogenic peptide thereof. Further embodiments for kits are outlined herein in great detail and apply also to the kit for detecting an anti-CXCR4 antibody.
It will be readily understood that the embodiments outlined above shall apply to the invention as a whole and not be limited to a specific method, unless stated otherwise. It will for example be understood the embodiments outlined for subject-matter relating to CXCR in general or the immunogenic peptide shall be applied to every method, kit or the like disclosed herein, and in particular to specific preferred CXCR, e.g. CXCR3, CXCR3-A, CXCR3-B or CXCR4. The invention is further illustrated by the following non-limiting examples and figures.

### Specific aspect: Prognosis of graft-versus-host-disease

The present invention is further based on the inventors' unexpected finding that low levels of autoanti-CXCR3 antibodies and high levels of C-X-C chemokines in a sample of a subject having acute GVHD are predictive for an adverse outcome for the subject. In the following, this aspect of the invention is further described. Unless stated otherwise, the same definitions and explanations apply as for the general concept of the invention described herein above.

Acute graft-versus-host disease (GVHD) occurs after allogeneic hematopoietic stem cell transplant and is a reaction of donor immune cells against host tissues. In contrast to chronic GVHD, acute GVHD is normally observed within the first 100 days post-transplant. Patients can have involvement of three organs: skin (rash/dermatitis), liver (hepatitis/jaundice), and gastrointestinal tract (abdominal pain/diarrhea). One or more organs may be involved. Acute GVHD is staged and graded (grade 0-IV) by the number and extent of organ involvement.

The following Table 1 summarizes the stages and grades of acute GVHD (Jacobsohn and Vogelsang, Orphanet J Rare Dis. 2007; 2: 35):

**Table 1: Stages and grades of acute GVHD**

| **Stage** | **Skin** | **Liver (b**i**lirubin)** | **Gastrointestinal tract (stool output/day)** |
|---|---|---|---|
| **0** | No GVHD rash | < 2 mg/dl | < 500 ml/day or persistent nausea. |
| **1** | Maculopapular rash< 25% BSA | 2-3 mq/dl | 500-999 ml/day |
| **2** | Maculopapular rash 25 - 50% BSA | 3.1-6 mg/dl | 1000-1500 ml/day |
| **3** | Maculopapular rash > 50% BSA | 6.1-15 m/dl | Adult: >1500 ml/day |
| **4** | Generalized erythroderma plus bullous formation | >15 mq/dl | Severe abdominal pain with or without ileus |
| **Grade** | | | |
| **I** | Stage 1-2 | None | None |
| **II** | Stage 3 or | Stage 1 or | Stage 1 |
| **III** | - | Stage 2-3 or | Stage 2-4 |
| **IV** | Stage 4 or | Stage 4 | - |

Grades III and IV are considered "high grade" acute GVHD. Patients with grade III/IV acute GVHD tend to have a poor outcome.

In particular, the present invention relates in one aspect to a method for the prognosis of acute graft-versus host-disease (GVHD) in a subject comprising the determination of the level of an antibody against the C-X-C chemokine receptor type 3 (CXCR3) and/or the level of a C-X-C chemokine selected from the group consisting of CXCL4, CXCL9, CXCL10 and CXCL11, preferably CXCL9, in a sample of a bodily fluid of said subject.

It is preferred in the context of the methods of this aspect of the present invention that the level of anti-CXCR3 and the level of a C-X-C chemokine selected from the group consisting of CXCL4, CXCL9, CXCL10 and CXCL11, preferably CXCL9, in the sample is detected.

The present invention also relates in this aspect to a method for the prognosis of acute graft-versus host-disease (GVHD) in a subject, wherein said method comprises
(a)
   (i) determining the level of an antibody against the C-X-C chemokine receptor type 3 (CXCR3) in a sample of a bodily fluid of said subject and
   (ii) comparing the determined level of the anti-CXCR3 antibody to a control level of anti-CXCR3 antibody, and/or
(b)
   (i) determining the level of a C-X-C chemokine selected from the group consisting of CXCL4, CXCL9, CXCL10 and CXCL11, preferably CXCL9, in a sample of a bodily fluid of said subject and
   (ii) comparing the determined level of said C-X-C chemokine to a control level of said C-X-C chemokine,
wherein a decreased level of said anti-CXCR3 antibody with respect to the anti-CXCR3 antibody control level and/or an increased level of said C-X-C chemokine with respect to the C-X-C chemokine control level in said sample is indicative for an adverse outcome for said subject.

The subject in this aspect has typically received an allogeneic stem cell transplantation (alloSCT) and suffers from acute GVHD, preferably high grade acute GVHD.

Outcome can generally be expressed in terms of different endpoints such as overall survival (OS), mortality, disease-free survival, progression-free survival, relapse-incidence and time to progression. Overall survival is the time to death, irrespective of the cause.
An "adverse outcome" in the context of the present invention is for example a low overall survival or a high mortality rate, i.e. the worst outcome would be death for the subject. Non-relapse mortality (NRM) is also a typical endpoint in the prediction of the outcome in GVHD after allogeneic stem cell transplantation (alloSCT). NRM is the time to deaths (of any cause) without relapse/recurrence.
Hence, the methods of the present invention can be used for the prediction of mortality, e.g. non-relapse mortality, in patients having acute GVHD, particularly high grade GVHD. The outcome can be predicted for different time points, e.g. a set time after onset of GVHD. It is preferred that the outcome 100 days, 1 year, most preferably 2 years after onset of GVHD is predicted.

The determined levels of the anti-CXR3 autoantibodies and the C-X-C chemokines in this aspect of the invention are compared to "control levels". The control levels as used refer to control levels derived from the defined control group or subject. It will be readily understood by the skilled person that the control level(s) are not necessarily determined in parallel to the determined level(s) of the subject but may be represented by previously determined levels. Nevertheless, control levels may be determined in parallel. Hence, the control levels of the present invention may be previously defined thresholds. Preferably, the sample(s) from which the control level(s) is/are derived is the same kind of sample as the sample of the subject to be prognosed. However, as outlined the control levels according to the present invention may also be predetermined control levels which are also integrated into the assay used. The control level(s) can for example be derived from a subject or a group of subjects not having acute GVHD, preferably a subject or a group of subjects not having high grade acute GVHD. A preferred control level is derived from a subject or a group of subjects prior to allogeneic stem cell transplantation, i.e. the control level is the level or average level of such subjects' prior transplantation. A further preferred control level is a level of a subject or a group of subjects on day 50 after allogeneic stem cell transplantation which corresponds to the median onset of acute GVHD.

Hence, in the case of the anti-CXCR3 antibody, e.g. a relatively low (i.e. decreased) level with respect to
- subjects without acute GVHD on day 50 after alloSCT,
- subjects having survived acute GVHD, or
- subjects prior to allogeneic stem cell transplantation, is indicative for an adverse outcome.

In the case of the C-X-C chemokines, preferably CXCL9, e.g. a relatively high (i.e. increased) level with respect to
- subjects without acute GVHD on day 50 after alloSCT,
- subjects having survived acute GVHD, or
- subjects prior to allogeneic stem cell transplantation, is indicative for an adverse outcome.

In particular, a level of said anti-CXCR3 antibody in the sample of said subject of less than 0.9-fold, preferably less than 0.8-fold, more preferably less than 0.5-fold, as compared to the control level is indicative of a negative outcome.

Also in particular, a level of said C-X-C chemokine, preferably CXCL9, in the sample of said subject of more than 1.1-fold, preferably more than 1.8-fold, more preferably more than 2.0-fold, as compared to the control level is indicative of a negative outcome.

Exemplary control levels are 600 pg/ml, 650 pg/ml, 670 pg/ml, 679 pg/ml, 680 pg/ml, 700 pg/ml, preferably 679 pg/ml for CXCL9 and 2.60 U/ml, 2.70 U/ml, 2.74 U/ml, 2.80 U/ml, preferably 2.74 U/ml for anti-CXCR3.

The methods of this aspect of the invention may for example be used for the prognosis, risk assessment, risk stratification, monitoring, therapy guidance and/or therapy control of acute GVHD, preferably high grade acute GVHD.

The anti-CXCR3 antibody may preferably be detected using an immunoassay comprising the steps of
(a) contacting the sample with CXC3R or an immunogenic peptide fragment thereof under conditions allowing for the formation of a complex between the anti-CXCR3 with CXCR3 or the antigenic peptide fragment thereof;
(b) detecting the complex.
The CXCR3 or the peptide fragment thereof may advantageously be immobilized on a surface. CXCR3 may have an amino acid sequence selected from the group consisting of SEQ ID NO:1 (CXCR3-A) and SEQ ID NO:2 (CXCR3-B). A particular immunogenic peptide may comprise a sequence according to SEQ ID NO:5. Said complex may be detected using a secondary antibody against the Fc portion of the anti-CXCR3 antibody. "anti-CXCR3 antibody" and "CXCR3 antibody" are used interchangeably herein and refer to antibodies specifically binding CXC3 chemokine receptor or fragments thereof, preferably autoimmune antibodies. Under the present conditions of an immunoassay, the above-mentioned anti-CXCR antibodies will preferably bind to a specific portion of the CXCR3, e.g. the extra cellular portion, while no significant binding to other proteins present in the sample will take place. In a preferred embodiment the anti-CXCR3 antibodies are selected from the group of antibodies consisting of IgG-antibodies and IgM-antibodies, preferably an IgG antibody, e.g. IgG1, IgG2, IgG3 and IgG4. In a certain preferred embodiment total IgG type anti-CXCR3 antibodies are to be detected in the methods of the present invention.

In connection with the immunoassay methods for detection of the CXCR3 autoantibodies of the present invention, the naturally occurring CXCR3 receptor as well as all modifications, mutants or derivatives of the CXC chemokine receptor type 3 can be used. Similarly, an CXCR3 produced by means of recombinant techniques, which receptor includes amino acid modifications, such as inversions, deletions, insertions, additions etc. can be used according to the invention provided that this part of the essential function of the CXCR3 is present, namely the capability of binding antibodies. The CXCR3 being used may also comprise exceptional amino acids and/or modifications of such as alkylation, oxidation, thiol-modification, denaturation, oligomerization and the like. The receptor can also be synthesized by chemical means. According to the invention the CXCR3 particularly can be a protein and/or peptide or a fusion protein, which in addition to other proteins, peptides or fragments thereof, includes the CXCR3 as a whole or in part. Using conventional methods, peptides or polypeptides of the CXCR3 which have functionally analogs, analogous properties can be determined by those skilled in the art. For example such polypeptides or peptides have 50 to 60%, 70% or 80%, preferably 90%, more preferably 95%, and most preferably 98% homology to peptides identified as CXC chemokine receptor, and said homology can be determined, e.g. by means of Smith-Waterman homology search algorithm, using the MPFRCH Programme (Oxford Molecular), for example.

An "immunogenic peptide" or "antigenic peptide" as used interchangeably in this aspect of the invention is a portion of a CXCR3 protein that is recognized (i.e., specifically bound) by the anti-CXCR3 antibodies in the sample of the patient to be detected. Such immunogenic peptides generally comprise at least 5 amino acid residues, more preferably at least 10, and still more preferably at least 20 amino acid residues of CXCR3. However, they may also comprise at least 30, 40, 50, 60, 70, 80, 90, 100, 110, 120, 130, 140 or 150 amino acid residues. In a preferred embodiment the immunogenic peptide is at least 112 amino residues in length. The term "immunogenic peptide" of an CXCR3 polypeptide/protein as used in the present invention, comprises also molecules differing from the original sequence by deletion(s), insertion(s), substitution(s) and/or other modifications well known in the prior art and/or comprising a fragment of the original amino acid molecule, the CXCR3 still exhibiting the properties mentioned above. Also included are allele variants and modifications. Methods of producing the above changes in the amino acid sequence are well known to those skilled in the art and have been described in the standard textbooks of molecular biology, e.g. Sambrook *et al.* Supra. Those skilled in the art will also be able to determine whether a CXCR3, thus, modified still has the properties mentioned above. Database entries exist in several well known Databases. When referreing to the amino acid sequence of CXCR3 any amino acid sequence known is meant or immunogenic fragments thereof, particularly those disclosed in common databases, preferably of human origin. The CXCR3 may be glycosylated *in vivo.* In the present specification all of the above illustrated modifications of the CXC3R will be referred to as "functionally analogous peptides or proteins" of CXCR3 in brief. Preferably, the CXCR3 has a sequence selected from the group consisting of SEQ ID NO:1 (CXCR3-A) and SEQ ID NO:2 (CXCR3-B). In a preferred embodiment the CXCR3 at least comprises an immunogenic fragment thereof, preferred immunogenic fragments comprise a sequence at least 50 %, 60%, 70% or 80%, preferably at least 90%, more preferably at least 95%, and most preferably at least 98% identical to the sequence of SEQ ID NO:5 (immunogenic CXCR3 fragment common to both isoforms).

In the context of the immunoassays of this aspect of the present invention the CXCR3 may be present in its natural cellular environment and can be used together with the material associated with CXCR3 in its natural state as well as in isolated form with respect to its primary, secondary and tertiary structures. The receptor is preferably used in isolated form, i.e. essentially free of other proteins, lipids, carbohydrates or other substances naturally associated with CXCR3. "Essentially free of' means that the receptor is at least 75%, preferably at least 85%, more preferably at least 95% and especially preferably at least 99% free of other proteins, lipids, carbohydrates or other substances naturally associated with the receptor. However, the CXCR3 may also be used as a membrane extract from cells expressing the vector naturally or my recombinant means.

In connection with this aspect of the present invention, the naturally occurring receptor as well as all modifications, mutants, immunogenic peptides or derivatives of the CXCR3 can be used. Similarly, a CXCR3 produced by means of recombinant techniques, which includes amino acid modifications, such as inversions, deletions, insertions, additions etc. can be used according to the invention provided that this part of the essential function of the CXCR3 is present, namely the capability of binding antibodies. The CXCR3 being used may also comprise exceptional amino acids and/or modifications of such as alkylation, oxidation, thiol-modification, denaturation, oligomerization and the like. The CXCR3 can also be synthesized by chemical means. According to the invention the CXCR3 particularly can be a protein and/or peptide or a fusion protein, which in addition to other proteins, peptides or fragments thereof, includes a CXCR3 as a whole or in part. Using conventional methods, peptides or polypeptides of the CXCR3 which have functionally analogs, analogous properties can be determined by those skilled in the art. For example such polypeptides or peptides have at least 50 %, 60%, 70% or 80%, preferably at least 90%, more preferably at least 95%, and most preferably at least 98% sequence identity to CXCR3, preferably to the sequence selected from the group consisting of SEQ ID NO:1 and SEQ ID NO:2.

The determination of percent identity between two sequences is accomplished using the mathematical algorithm of Karlin and Altschul (1993) Proc. Natl. Acad. Sci. USA 90: 5873-5877. Such an algorithm is incorporated into the BLASTN and BLASTP programs of Altschul et al. (1990) J. Mol. Biol. 215: 403-410. BLAST nucleotide searches are performed with the BLASTN program, score = 100, word length = 12, to obtain nucleotide sequences homologous to the EPO variant polypeptide encoding nucleic acids. BLAST protein searches are performed with the BLASTP program, score = 50, wordlength = 3, to obtain amino acid sequences homologous to the EPO variant polypeptide, respectively. To obtain gapped alignments for comparative purposes, Gapped BLAST is utilized as described in Altschul et al. (1997) Nucleic Acids Res. 25: 3389-3402. When utilizing BLAST and Gapped BLAST programs, the default parameters of the respective programs are used.

The antibodies to be detected or determined according to this aspect of the present invention are directed against CXCR3. This means that the antibodies specifically bind CXCR3. Specific binding of an antibody normally occurs via binding of a binding site of the antigen. The antibodies of this aspect of the present invention are those specifically binding to CXCR3 or immunogenic fragments thereof. This binding may occur via recognition of sequence or structural epitopes. The skilled person is aware of methods of how to determine specific epitopes, e.g. fragments of the antigen CXCR3, which are recognized and bound by the antibodies to be determined. Fragments of CXCR3 binding to the auto antibodies are called immunogenic or antigenic fragments. Methods for determining fragments of an antigen binding the antibody are described in several publications (e.g. Gershoni, JM; Roitburd-Berman, A; Siman-Tov, DD; Tarnovitski Freund, N; Weiss, Y (2007). "Epitope mapping: The first step in developing epitope-based vaccines". BioDrugs 21 (3): 145-56; Westwood, MR; Hay, FC (2001). Epitope Mapping: a practical approach. Oxford, Oxfordshire: Oxford University Press. ISBN 0-19-963652-4; Flanagan et al. (2011), "Mapping Epitopes with H/D-Ex Mass Spec". Genetic Engineering and Biotechnology news; 31(1); Gaseitsiwe, S.; Valentini, D.; Mahdavifar, S.; Reilly, M.; Ehrnst, A.; Maeurer, M. (2009) "Peptide Microarray-Based Identification of Mycobacterium tuberculosis Epitope Binding to HLA-DRB1∗0101, DRB1∗1501, and DRB1∗0401". Clinical and Vaccine Immunology 17 (1): 168-75; Linnebacher, Michael; Lorenz, Peter; Koy, Cornelia; Jahnke, Annika; Born, Nadine; Steinbeck, Felix; Wollbold, Johannes; Latzkow, Tobias et al. (2012). "Clonality characterization of natural epitope-specific antibodies against the tumor-related antigen topoisomerase IIa by peptide chip and proteome analysis: A pilot study with colorectal carcinoma patient samples" Analytical and Bioanalytical Chemistry 403 (1): 227-38; Cragg, M. S. (2011). "CD20 antibodies: Doing the time warp". Blood 118 (2): 219-20; Banik, Soma S. R.; Doranz, Benjamin J. (2010). "Mapping Complex Antibody Epitopes". Genetic Engineering and Biotechnology News 3 (2): 25-8; and Paes, Cheryl; Ingalls, Jada; Kampani, Karan; Sulli, Chidananda; Kakkar, Esha; Murray, Meredith; Kotelnikov, Valery; Greene, Tiffani A. et al. (2009). "Atomic-Level Mapping of Antibody Epitopes on a GPCR". Journal of the American Chemical Society 131 (20): 6952-4). In context with the present invention anti-CXCR3 antibodies are understood as any immunoglobulin specifically recognizing/binding to a CXCR3. The antibody in a preferred embodiment binds any CXCR3 variant more preferably to a sequence selected from the group consisting of SEQ ID NO:1 and SEQ ID NO:2. Preferably recognizing a peptide comprising a sequence at least 50 %, 60%, 70% or 80%, preferably at least 90%, more preferably at least 95%, and most preferably at least 98% to the sequence of SEQ ID NO:5.

The C-X-C chemokine according to the present invention is selected from the group consisting of CXCL4, CXCL9, CXCL10 and CXCL11, preferably CXCL9. It may preferably be detected using an immunoassay comprising the steps of
(a) contacting the sample with an anti-C-X-C chemokine antibody, preferably an anti-CXCL9 antibody, under conditions allowing for the formation of a complex between said antibody with the C-X-C chemokine, preferably CXCL9;
(b) detecting the complex.

The anti-C-X-C chemokine antibody, preferably the anti-CXCL9 antibody, is immobilized on a surface. The complex may be detected using a secondary antibody against the C-X-C chemokine, preferably CXCL9.

In the immunoassay methods of this aspect of the invention, the secondary antibody may advantageously be labeled with a detectable marker. Immunoassay formats include but are not limited to immunoprecipitation, enzyme immunoassay (EIA), radioimmunoassay (RIA) or fluorescencent immunoassay, a chemiluminescent assay, an agglutination assay, nephelometric assay, turbidimetric assay, a Western blot, a competitive immunoassay, a noncompetitive immunoassay, a homogeneous immunoassay a heterogeneous immunoassay, a bioassay and a reporter-assay such as a Luciferase-Assay. Preferably, the immunoassay in the context of the present invention is an ELISA, e.g. a sandwich ELISA format.

The immunoassays can be homogenous or heterogeneous assays, competitive and non-competitive assays. In a particularly preferred embodiment, the assay is in the form of a sandwich assay, which is a non-competitive immunoassay, wherein the "analyte" (e.g. the anti-CXCR3 antibody) to be detected and/or quantified is allowed to bind to a "ligand" thereof (e.g. a CXCR3 polypeptide or immunogenic fragment thereof). The CXCR3 protein or immunogenic fragment thereof (i.e. a peptide), may e.g., be bound to a solid phase, e.g. a bead, a surface of a well or other container, a chip or a strip, and the secondary antibody detection mean is an antibody or detection mean which is labeled, e.g. with a dye, with a radioisotope, or a reactive or catalytically active moiety such as a peroxidase, e.g. horseradish peroxidase. The amount of labeled antibody bound to the analyte is then measured by an appropriate method. The general composition and procedures involved with "sandwich assays" are well-established and known to the skilled person (The Immunoassay Handbook, Ed. David Wild, Elsevier LTD, Oxford; 3rd ed. (May 2005), ISBN-13: 978-0080445267*;* Hultschig C et al., Curr Opin Chem Biol. 2006 Feb;10(1):4-10*. PMID: 16376134,* incorporated herein by reference). Sandwich immunoassays can for example be designed as one-step assays or as two-step assays.

The present invention in this aspect also relates to the use of CXCR3 or an immunogenic peptide thereof for the prognosis of acute GVHD, preferably high grade acute GVHD.

The invention further pertains in this aspect to the use of an anti-C-X-C chemokine antibody, preferably an anti-CXCL9 antibody, for the prognosis of acute GVHD, preferably high grade acute GVHD.
Further, the invention relates in this aspect to the use of a kit for the prognosis of acute GVHD, preferably high grade acute GVHD, wherein the kit comprises CXCR3 or an immunogenic peptide thereof. The invention also relates in this aspect to the use of a kit for the prognosis of acute GVHD, preferably high grade acute GVHD, wherein the kit comprises an anti-C-X-C chemokine antibody, preferably an anti-CXCL9 antibody.

The present invention in this aspect relates to medical uses for the treatment of acute GVHD based on the prognostic markers of the invention. In particular, the invention relates to a method for treating acute GVHD, preferably high grade GVHD, in a subject, wherein the subject is treated upon detecting a decreased level of anti-CXCR3 antibody with respect to an anti-CXCR3 antibody control level and/or an increased level of a C-X-C chemokine, preferably CXCL9, with respect to a C-X-C chemokine control level, preferably a CXCL9 control level, in a sample of a bodily fluid of said subject. Preferably, the subject is treated with an immunosuppressant and/or a statin, preferably wherein the immunosuppressant is preferably prednisolone, mycofenolate mofetil or a calcineurin inhibitor such as ciclosporin or tacrolimus. The immunosuppressant is advantageously combined with a statin such as pravastatin.
In this context, the subject can be treated with a CXCR3 antagonist, preferably an anti-CXCR3 antibody.

The invention also pertains in this aspect to an immunosuppressant for use in the treatment of acute GVHD, preferably high grade acute GVHD, wherein the subject is treated upon detecting a decreased level of anti-CXCR3 antibody with respect to an anti-CXCR3 antibody control level and/or an increased level of a C-X-C chemokine, preferably CXCL9, with respect to a C-X-C chemokine control level, preferably a CXCL9 control level, in a sample of a bodily fluid of said subject.
The immunosuppressant is preferably prednisolone, mycofenolate mofetil or a calcineurin inhibitor such as ciclosporin or tacrolimus. The immunosuppressant is advantageously combined with a statin such as pravastatin.

The invention further pertains in this aspect to an CXCR3 antagonist, preferably an anti-CXCR3 antibody, for use in the treatment of acute GVHD, preferably high grade acute GVHD, wherein the subject is treated upon detecting a decreased level of anti-CXCR3 antibody with respect to an anti-CXCR3 antibody control level and/or an increased level of a C-X-C chemokine, preferably CXCL9, with respect to a C-X-C chemokine control level, preferably a CXCL9 control level, in a sample of a bodily fluid of said subject.

The invention further pertains in this aspect to a C-X-C chemokine antagonist, preferably an CXCL9 antagonist, or an anti-C-X-C chemokine antibody, preferably an anti-CXCL9 antibody, for use in the treatment of acute GVHD, preferably high grade acute GVHD, wherein the subject is treated upon detecting a decreased level of anti-CXCR3 antibody with respect to an anti-CXCR3 antibody control level and/or an increased level of a C-X-C chemokine, preferably CXCL9, with respect to a C-X-C chemokine control level, preferably a CXCL9 control level, in a sample of a bodily fluid of said subject.

The sensitivity and specificity of a diagnostic and/or prognostic test depends on more than just the analytical "quality" of the test, they also depend on the definition of what constitutes an abnormal result. In practice, Receiver Operating Characteristic curves (ROC curves), are typically calculated by plotting the value of a variable versus its relative frequency in "normal" (i.e. the control group) and "disease" populations (or specific outcome as the case may be). For any particular marker, a distribution of marker levels for subjects with and without a disease or outcome will likely overlap. Under such conditions, a test does not absolutely distinguish normal from disease (or specific outcome) with 100% accuracy, and the area of overlap indicates where the test cannot distinguish normal from disease (or specific outcome). A threshold is selected, below which the test is considered to be abnormal and above which the test is considered to be normal. The area under the ROC curve is a measure of the probability that the perceived measurement will allow correct identification of a condition. ROC curves can be used even when test results do not necessarily give an accurate number. As long as one can rank results, one can create a ROC curve. For example, results of a test on "disease" samples might be ranked according to degree (e.g. 1=low, 2=normal, and 3=high). This ranking can be correlated to results in the "normal" population, and a ROC curve created. These methods are well known in the art; *see,* e.g., Hanley et al. 1982. Radiology 143: 29-36*.* Preferably, a threshold is selected to provide a ROC curve area of greater than about 0.5, more preferably greater than about 0.7, still more preferably greater than about 0.8, even more preferably greater than about 0.85, and most preferably greater than about 0.9. The term "about" in this context refers to +/- 5% of a given measurement.

The skilled artisan will understand that associating a diagnostic or prognostic indicator, with a diagnosis or with a prognostic risk of a future clinical outcome is a statistical analysis. For example, a marker level of lower than X may signal that a patient is more likely to suffer from an adverse outcome than patients with a level more than or equal to X (or vice versa as the case may be), as determined by a level of statistical significance. Additionally, a change in marker concentration from baseline levels may be reflective of patient prognosis, and the degree of change in marker level may be related to the severity or activity of the disease. Statistical significance is often determined by comparing two or more populations, and determining a confidence interval and/or a p value; *see,* e.g., Dowdy and Wearden, Statistics for Research, John Wiley & Sons, New York, 1983. Preferred confidence intervals of the invention are 90%, 95%, 97.5%, 98%, 99%, 99.5%, 99.9% and 99.99%, while preferred p values are 0.1, 0.05, 0.025, 0.02, 0.01, 0.005, 0.001, and 0.0001.

Suitable threshold levels for the stratification of subjects into different groups (categories) have to be determined for each particular combination of marker (i.e. anti-CXCR3 antibody and/or C-X-C chemokines, preferably CXCL9), outcome and/or medication. This can e.g. be done by grouping a reference population of patients according to their level of marker into certain quantiles as described herein above.

Kaplan-Meier estimators may be used for the assessment or prediction of the outcome or risk (e.g. prognosis) of a subject.

Furthermore, in the methods of the present invention further parameters of the subject may be considered as well for diagnosis, differential diagnosis, assessment of the need for an immunosuppressant, therapy monitoring and prognosis etc. Such parameters in a multivariate model may include gender, age, histological evaluation, and other markers. A Cox-Proportional-Hazard regression predicts the dependent variable based on one or more independent variables. These predictors can either be measures (as e.g. level of a biomarker) or categorical data. The skilled person is aware of the fact that diagnostic/prognostic markers only give a certain degree of sensitivity and specificity. He knows that different further parameters might be considered in order to increase both. Nevertheless, the present invention provides a new and superior marker for prognosis as outlined herein in great detail.

The term "patient" and "subject" as used in this aspect of the invention refers to a subject, preferably the subject is a mammal, in particular humans are preferred. Preferably the subject to be prognosed is a subject having acute GVHD or high grade acute GVHD as defined herein.

The skilled person will understand that controls for comparing the determined levels may be of different nature e.g. depending on the assay used. The kit according to this aspect of the present invention may for example comprise one or more controls comprising anti-CXCR3 antibody and/or C-X-C chemokines, preferably CXCL9, at the desired control level. Furthermore, the kit may comprise one or more standard solutions each solution comprising anti-CXCR3 antibody and/or C-X-C chemokines, preferably CXCL9, at different levels, such standard solutions are particularly preferred in cases were a standard curves are to be applied. Exemplary dilutions and levels for such standard solutions are outlined herein above.

The embodiments set out for the immunoassays apply also to the kit of the invention. The kits of this aspect of the present invention are meant for the detection of autoimmune antibodies (anti-CXCR3 autoantibodies) and/or C-X-C chemokines, preferably CXCL9, in samples of a subject. Hence, in one embodiment they comprise means for the preparation of blood, e.g. for gaining serum or plasma thereof. Furthermore, the kit may comprise control composition and/or standards. The control composition preferably comprises anti-CXCR3 antibodies and/or C-X-C chemokines, preferably CXCL9, as positive control. Furthermore, the kit may comprise one or a plurality of standard compositions. A standard composition comprises anti-CXCR3 antibodies or C-X-C chemokines, preferably CXCL9, at a defined concentration. As outlined herein, determination of concentration of autoimmune-antibodies or chemokines may be performed using standard curves. These curves set out which concentration of antibodies in a sample or solution corresponds to what read-out value of the assay used, e.g. optical density or proportion of optical density at different wavelengths (e.g. 450nm/620nm). To this end the kits of the present invention may comprise one or more standard compositions having a defined concentration of the respective marker, preferably of the kind to be detected in the method. A standard composition of the kits according to this aspect of the present invention comprise anti-CXCR3 antibodies and/or C-X-C chemokines, preferably CXCL9, at concentrations selected from the group consisting of 40 units/ml, 20 units/ml, 10 units/ml, 5 units/ml, 2.5 units/ml and 1.25 units/ml. In one embodiment the kit comprises five standard compositions with the recited concentration. In another embodiment the kit comprises one standard composition with the highest concentration of the standard curve, e.g. 40 units/ml or 80 units/ml. The other concentrations may be produced at the side of the end user by further dilutions, e.g. in PBS. A dilution buffer may therefore also be comprised in the kits according to this aspect of the invention.

In a preferred embodiment the kit comprises a CXCR3, an immunogenic peptide or a functional analog thereof.

The immunological test kit according to this aspect of the invention comprises the CXCR3 or a functional analog thereof or immunogenic peptides or proteins of analogous function *per se.* The test kit of this aspect of the invention comprises at least one complete CXCR3 or functionally analogous peptides or proteins of said receptor, optionally bound to a solid phase. Furthermore, the test kit may also comprise buffers, specific conjugate together with an enzyme, wash solution, substrate solution to detect the immune reaction and/or a quenching solution. Using these substances a person skilled in the art will be able to perform, e.g. an ELISA to detect the anti-CXCR3 antibodies. The buffers, specific conjugate plus enzyme, wash solution, substrate solution to detect immune reaction and quenching solution are well known to those skilled in the art. For example, it would be sufficient to have the test comprise a freeze-dried CXCR3 or immunogenic peptides or proteins of CXCR3 analogous function and to add the buffers and other solutions immediately prior to testing the biological material. However, it is also possible to provide the test kit with the CXCR3 or its functionally analogous peptides of proteins bound to a solid phase. To detect the anti-CXCR3 antibodies the specific conjugate, wash solution, substrate solution and quenching solution, which can be components of the test kit, have to be added according to a mode well known to those skilled in the art.

The kit according to this aspect of the present invention preferably comprises means for detecting antibodies, preferably a secondary antibody binding the Fc portion of the anti-CXCR3 antibody to be detected.

In another advantageous embodiment of this aspect of the invention, it is envisioned that the test kit is a test strip comprising the CXCR3 or immunogenic peptides or its functionally analogous peptides or proteins immobilized on a solid phase. For example, the test strip can be immersed in serum or other patient samples and incubated. Using a specific biochemical reaction on the test strip after formation of the anti-CXCR3 antibody complex, a specific color reaction can be triggered by means of which the anti-CXCR3 antibody can be detected.

The test system of the invention permits quantification of the anti-CXCR3 antibodies directly in a sample, e.g. in plasma of patients. The detection method according to this aspect of the invention is time saving and cost efficient. Large amounts of the samples can be tested and, owing to the low amount of the equipment required, routine laboratories can be used.

Chemokine receptor CXCR3 (Gene ID: 2833) is a Gαᵢ protein-coupled receptor in the CXC chemokine receptor family. Other names for CXCR3 are G protein-coupled receptor 9 (GPR9) and CD183, C-X-C chemokine receptor type 3, CXC-R3, CXCR-3, G protein-coupled receptor 9, IP-10 receptor, IP10 receptor, Mig receptor, chemokine (C-X-C) receptor 3, and interferon-inducible protein 10 receptor. There are two variants of CXCR3: CXCR3-A binds to the CXC chemokines CXCL9 (a.k.a. Monokine induced by Gamma-Interferon (MIG)), CXCL10 (Interferon gamma-induced protein 10 (IP-10)), and CXCL11 (Interferon-inducible T-cell alpha chemoattractant (I-TAC)) (Clark-Lewis I, Mattioli I, Gong JH, Loetscher P (2003). "Structure-function relationship between the human chemokine receptor CXCR3 and its ligands". J. Biol. Chem. 278 (1): 289-295), whereas CXCR3-B can also bind to CXCL4 (a.k.a. Platelet factor 4 (PF4)) in addition to CXCL9, CXCL10, and CXCL11 (Lasagni L, Francalanci M, Annunziato F, Lazzeri E, Giannini S, Cosmi L et al. (2003). "An Alternatively Spliced Variant of CXCR3 Mediates the Inhibition of Endothelial Cell Growth Induced by IP-10, Mig, and I-TAC, and Acts as Functional Receptor for Platelet Factor 4". J. Exp. Med. 197 (11): 1537-1549). CXCR3 is expressed primarily on activated T lymphocytes and NK cells (Qin S, Rottman JB, Myers P, Kassam N, Weinblatt M, Loetscher M et al. (1998).

Sequence information for the C-X-C chemokines is available from the NCBI RefSeq database: CXCL4 (NCBI-Database as of September 16, 2016: NP_002610.1) [SEQ ID NO:7],
CXCL9 (NCBI-Database as of March 3, 2016: NP002407.1) [SEQ ID NO:8],
CXCL10 (NCBI-Database as of September 1, 2016: NP001556.2) [SEQ ID NO:9],
CXCL11 (isoform 1: NCBI-Database as of September 1, 2016: NP005400.1 [SEQ ID NO:10]; isoform 2 NCBI-Database as of September 11, 2016: NP_001289052.1) [SEQ ID NO:11].
These sequences relate to the precursor sequences of these proteins comprising e.g. signal sequences (first 31 residues for CXCL4, first 22 residues for CXCL9 and first 21 residues for CXCL10 and CXCL11); see the above NCBI databank entries.

### Items of the Invention

The invention particularly relates to the following items:
1. A method for diagnosis an autoimmune disease in a subject wherein, presence or absence of an anti-CXC chemokine receptor (CXCR) antibody is detected in a sample from the subject diagnosed, and wherein the presence of an anti-CXCR antibody is indicative of the disease.
2. The method according to item 1, wherein the presence or absence of the anti-CXCR antibody is detected by the following steps:
   (i) determining the level of anti-CXCR antibodies in a sample of the subject to be diagnosed; and
   (ii) comparing the determined level to an anti-CXCR antibody control level derived from a subject without an autoimmune disease;
   wherein an increased level of anti-CXCR antibody in the sample of the subject to be diagnosed as compared to the anti-CXCR antibody control level denotes the presence of an anti-CXCR antibody.
3. The method according to item 2, wherein a level in the sample of the subject to be diagnosed of more than 1.1 fold as compared to the control level from subjects without an autoimmune disease is indicative of the presence of an autoimmune disease in the subject to be diagnosed, preferably a level in the sample of the subject to be diagnosed of more than 1.8 fold as compared to the control level from subjects without an autoimmune disease is indicative of the presence of an autoimmune disease in the subject to be diagnosed, more preferably a level in the sample of the subject to be diagnosed of more than 2 fold as compared to the control level from subjects without an autoimmune disease is indicative of the presence of an autoimmune disease in the subject to be diagnosed.
4. A method for determining disease activity of an autoimmune disease comprising the steps of:
   (i) determining the level of anti-CXCR antibodies in a sample from a subject to be diagnosed,
   (ii) comparing the determined level in the sample to either one or both of a first and second anti-CXCR antibody control level,
      a) wherein the first anti-CXCR antibody control level is derived from subjects suffering an inactive autoimmune disease; and
      b) wherein the second anti-CXCR antibody control level derived from subjects suffering an active autoimmune disease;
   wherein an increased level in the sample from the subject to be diagnosed as compared to the first anti-CXCR antibody control level and/or an equal level as compared to the second anti-CXCR antibody control level is indicative of the presence of an active autoimmune disease in the subject to be diagnosed, and
   wherein an decreased level in the sample from the subject to be diagnosed as compared to the second anti-CXCR antibody control level and/or an equal level as compared to the first anti-CXCR antibody control level is indicative of the presence of an inactive autoimmune disease.
5. The method according to any one of items 1 to 4, wherein the anti-CXCR antibody is an anti-CXCR3 or anti-CXCR4 antibody.
6. The method according to any one of items 1 to 5, wherein the autoimmune disease is an autoimmune disease of connective tissue, preferably selected from the group of systemic lupus erythematosus (SLE), rheumatoid arthritis, scleroderma, Sjögren's syndrome, mixed connective tissue disease, systemic sclerosis (SSc); dermatomyositis (DM), polymyositis (PM), GvHD, cGvHD, anti-synthetase syndrome, and psoriatic arthritis, more preferably systemic lupus erythematosus.
7. The method according to any one of the preceding items, wherein the anti-CXCR antibody is detected using an immunoassay comprising the steps of
   (a) contacting the sample with a CXC chemokine receptor (CXCR) or an immunogenic peptide fragment thereof under conditions allowing for the formation of a complex between anti-CXCR antibodies with CXCR or the antigenic peptide fragment thereof;
   (b) detecting the complex.
8. An immunoassay method for detecting an anti-CXC chemokine receptor (CXCR) antibody in a sample from a subject, comprising the steps of
   (a) contacting the sample suspected of comprising an anti-CXCR antibody with a CXC chemokine receptor (CXCR) or an immunogenic peptide fragment thereof under conditions allowing for the formation of a complex between the anti-CXCR antibody with CXCR or the peptide fragment thereof;
   (b) detecting the complex.
9. The method of item 7 or 8, wherein the CXC chemokine receptor (CXCR) or the peptide fragment thereof is immobilized on a surface.
10. The method according to any one of items 7 to 9, wherein the CXC chemokine receptor (CXCR) is selected from the group consisting of CXCR3 and CXCR4, preferably the CXC chemokine receptor (CXCR) has a sequence selected from the group consisting of SEQ ID NO:1 (CXCR3-A), SEQ ID NO:2 (CXCR3-B), SEQ ID NO:3 (CXCR4a) and SEQ ID NO:4 (CXCR4b), or wherein said immunogenic peptide comprises a sequence selected from the group consisting of SEQ ID NO:5 and SEQ ID NO:6.
11. The method according to any one of items 7 to 10, wherein the complex is detected using a secondary antibody against the Fc portion of the anti-CXCR antibody.
12. The method according to item 11, wherein the anti-CXCR antibody is an IgG-antibody and the secondary antibody is an anti-IgG antibody.
13. The method according to item 11 or 12, wherein the secondary antibody is labeled with a detectable marker.
14. The method according to any one of items 7 to 13, wherein the immunoassay is selected from the group of immunoprecipitation, enzyme immunoassay (EIA), radioimmunoassay (RIA) or fluorescencent immunoassay, a chemilumineszent assay, an agglutination assay, nephelometric assay, turbidimetric assay, a Western blot, a competitive immunoassay, a noncompetitive immunoassay, a homogeneous immunoassay a heterogeneous immunoassay, a bioassay and a reporter-assay such as a Luciferase-Assay.
15. The method according to item 14, wherein the immunoassay is an ELISA.
16. Use of a CXCR or an immunogenic peptide thereof for the diagnosis of an autoimmune disease.
17. Use of a kit in for the diagnosis of an autoimmune disease, wherein the kit comprises a CXCR or an immunogenic peptide thereof.
18. The use according to item 16 or 17, wherein the CXC chemokine receptor (CXCR) is selected from the group consisting of CXCR3 and CXCR4, preferably the CXC chemokine receptor (CXCR) has a sequence selected from the group consisting of SEQ ID NO:1 (CXCR3-A), SEQ ID NO:2 (CXCR3-B) and SEQ ID NO:3 (CXCR4).
19. The use according to any one of items 16 to 18, wherein the autoimmune disease is an autoimmune disease of connective tissue, preferably selected from the group of systemic lupus erythematosus (SLE), rheumatoid arthritis, scleroderma, Sjögren's syndrome, mixed connective tissue disease, GvHD, cGvHD, systemic sclerosis (SSc); dermatomyositis (DM), polymyositis (PM), anti-synthetase syndrome, and psoriatic arthritis, more preferably systemic lupus erythematosus.
20. A method for the removal of anti-CXCR antibodies from isolated blood,
   (i) wherein in a first step the presence or absence of an anti-CXCR antibody is determined in a blood sample from a subject to be diagnosed for an autoimmune disease.
   (ii) wherein upon determining the presence of an anti-CXCR antibody the antibody is removed from isolated blood of the subject.
21. A kit for detecting an anti-CXCR antibody comprising a CXCR or an immunogenic peptide thereof.
22. The kit according to item 23, wherein the kit comprises means for detecting antibodies, preferably a secondary antibody binding the Fc portion of the anti-CXCR antibody to be detected.
23. The kit according to item 21 or 22, wherein the CXC chemokine receptor (CXCR) is selected from the group consisting of CXCR3 and CXCR4, preferably the CXC chemokine receptor (CXCR) has a sequence selected from the group consisting of SEQ ID NO:1 (CXCR3-A), SEQ ID NO:2 (CXCR3-B) and SEQ ID NO:3 (CXCR4)
24. The kit according to any one of items 21 to 23, wherein the kit further comprises means for handling and/or processing a blood sample.
25. A method for treating an autoimmune disease in a subject, wherein the subject is treated upon diagnosing an autoimmune disease or an autoimmune disease with an active autoimmune disease with a method according to any one of items 1 to 15.
26. An immunosuppressant for use in the treatment in an autoimmune disease, wherein the subject is treated upon determination of the presence and/or increased levels of an anti-CXCR antibody in a sample of said subject; preferably upon diagnosing an autoimmune disease or an autoimmune disease with an active autoimmune disease with a method according to any one of items 1 to 15.
27. The immunosuppressant for use according to item 26, wherein the immunosuppressant is selected from the group consisting of cyclophosphamides, glucocorticoids, and Rituximab.
28. The immunosuppressant for use according to item 26 or 27, wherein the autoimmune disease is an autoimmune disease of connective tissue, preferably selected from the group of systemic lupus erythematosus (SLE), rheumatoid arthritis, scleroderma, Sjögren's syndrome, mixed connective tissue disease, GvHD, cGvHD, systemic sclerosis (SSc); dermatomyositis (DM), polymyositis (PM), anti-synthetase syndrome, and psoriatic arthritis, more preferably systemic lupus erythematosus.
29. A method for the prognosis of acute graft-versus host-disease (GVHD) in a subject comprising the determination of the level of an antibody against the C-X-C chemokine receptor type 3 (CXCR3) and/or the level of a C-X-C chemokine selected from the group consisting of CXCL4, CXCL9, CXCL10 and CXCL11, preferably CXCL9, in a sample of a bodily fluid of said subject.
30. A method for the prognosis of acute graft-versus host-disease (GVHD) in a subject, wherein said method comprises
   (a)
      (i) determining the level of an antibody against the C-X-C chemokine receptor type 3 (CXCR3) in a sample of a bodily fluid of said subject and
      (ii) comparing the determined level of the anti-CXCR3 antibody to a control level of anti-CXCR3 antibody, and/or
   (b)
      (i) determining the level of a C-X-C chemokine selected from the group consisting of CXCL4, CXCL9, CXCL10 and CXCL11, preferably CXCL9, in a sample of a bodily fluid of said subject and
      (ii) comparing the determined level of said C-X-C chemokine to a control level of said C-X-C chemokine,
   wherein a decreased level of said anti-CXCR3 antibody with respect to the anti-CXCR3 antibody control level and/or an increased level of said C-X-C chemokine with respect to the C-X-C chemokine control level in said sample is indicative for an adverse outcome for said subject.
31. The method of item 29, wherein the adverse outcome is a high mortality rate.
32. The method of item 29 to 31, wherein the subject suffers from high grade acute GVHD.
33. The method of item 30 to 32, wherein said control level(s) is/are derived from a subject or a group of subjects not having acute GVHD, preferably a subject or a group of subjects not having high grade acute GVHD.
34. The method of item 30 to 32, wherein the control level of the anti-CXCR3 antibody and/or the control level of the C-X-C chemokine is/are
   a) a level derived from subjects prior to allogeneic stem cell transplantation, or
   b) a level on day 50 after allogeneic stem cell transplantation.
35. The method according to items 30 to 32, wherein a level of said anti-CXCR3 antibody in the sample of said subject of less than 0.9-fold, preferably less than 0.8-fold, more preferably less than 0.5-fold, as compared to the control level is indicative of a negative outcome.
36. The method according to items 30 to 32, wherein a level of said C-X-C chemokine, preferably CXCL9, in the sample of said subject of more than 1.5-fold, preferably more than 1.8-fold, more preferably more than 2.0-fold, as compared to the control level is indicative of a negative outcome.
37. The method according to any one of the preceding items, wherein the anti-CXCR3 antibody is detected using an immunoassay comprising the steps of
   (a) contacting the sample with CXC3R or an immunogenic peptide fragment thereof under conditions allowing for the formation of a complex between the anti-CXCR3 with CXCR3 or the antigenic peptide fragment thereof;
   (b) detecting the complex.
38. The method of item 37, wherein the CXCR3 or the peptide fragment thereof is immobilized on a surface.
39. The method according to item 37 or 38, wherein the CXCR3 has a sequence selected from the group consisting of SEQ ID NO:1 (CXCR3-A) and SEQ ID NO:2 (CXCR3-B), or wherein said immunogenic peptide comprises a sequence according to SEQ ID NO:5.
40. The method according to any one of items 37 to 39, wherein the complex is detected using a secondary antibody against the Fc portion of the anti-CXCR3 antibody.
41. The method according to item 40, wherein the anti-CXCR3 antibody is an IgG-antibody and the secondary antibody is an anti-IgG antibody.
42. The method according to any one of the preceding items, wherein the C-X-C chemokine, preferably CXCL9, is detected using an immunoassay comprising the steps of
   (a) contacting the sample with an anti-C-X-C chemokine antibody, preferably an anti-CXCL9 antibody, under conditions allowing for the formation of a complex between said antibody with the C-X-C chemokine, preferably CXCL9;
   (b) detecting the complex.
43. The method of item 42, wherein the anti-C-X-C chemokine antibody, preferably the anti-CXCL9 antibody, is immobilized on a surface.
44. The method according to any one of items 42 or 43, wherein the complex is detected using a secondary antibody against the C-X-C chemokine, preferably CXCL9.
45. The method according to item 40, 41 or 44, wherein the secondary antibody is labeled with a detectable marker.
46. The method according to any one of items 37 to 45, wherein the immunoassay is selected from the group of immunoprecipitation, enzyme immunoassay (EIA), radioimmunoassay (RIA) or fluorescencent immunoassay, a chemiluminescent assay, an agglutination assay, nephelometric assay, turbidimetric assay, a Western blot, a competitive immunoassay, a noncompetitive immunoassay, a homogeneous immunoassay a heterogeneous immunoassay, a bioassay and a reporter-assay such as a Luciferase-Assay.
47. The method according to item 46, wherein the immunoassay is an ELISA.
48. Use of CXCR3 or an immunogenic peptide thereof for the prognosis of acute GVHD, preferably high grade acute GVHD.
49. Use of an anti-C-X-C chemokine antibody, preferably an anti-CXCL9 antibody, for the prognosis of acute GVHD, preferably high grade acute GVHD.
50. Use of a kit for the prognosis of acute GVHD, preferably high grade acute GVHD, wherein the kit comprises CXCR3 or an immunogenic peptide thereof.
51. Use of a kit for the prognosis of acute GVHD, preferably high grade acute GVHD, wherein the kit comprises an anti-C-X-C chemokine antibody, preferably an anti-CXCL9 antibody.
52. A method for treating acute GVHD, preferably high grade GVHD, in a subject, wherein the subject is treated upon detecting a decreased level of anti-CXCR3 antibody with respect to an anti-CXCR3 antibody control level and/or an increased level of a C-X-C chemokine, preferably CXCL9, with respect to a C-X-C chemokine control level, preferably a CXCL9 control level, in a sample of a bodily fluid of said subject.
53. The method for treating acute GVHD, preferably high grade GVHD according to item 52, wherein the subject is treated with an immunosuppressant and/or a statin, preferably wherein the immunosuppressant is prednisolone, mycofenolate mofetil or a calcineurin inhibitor such as ciclosporin or tacrolimus.
54. The method for treating acute GVHD, preferably high grade GVHD according to item 52, wherein the subject is treated with a CXCR3 antagonist, preferably an anti-CXCR3 antibody.
55. An immunosuppressant for use in the treatment of acute GVHD, preferably high grade acute GVHD, wherein the subject is treated upon detecting a decreased level of anti-CXCR3 antibody with respect to an anti-CXCR3 antibody control level and/or an increased level of a C-X-C chemokine, preferably CXCL9, with respect to a C-X-C chemokine control level, preferably a CXCL9 control level, in a sample of a bodily fluid of said subject.
56. The immunosuppressant for use according to item 55, wherein the immunosuppressant is prednisolone, mycofenolate mofetil or a calcineurin inhibitor such as ciclosporin or tacrolimus.
57. The immunosuppressant for use according to item 55 or 56, wherein the immunosuppressant is combined with a statin such as pravastatin.
58. A CXCR3 antagonist, preferably an anti-CXCR3 antibody, for use in the treatment of acute GVHD, preferably high grade acute GVHD, wherein the subject is treated upon detecting a decreased level of anti-CXCR3 antibody with respect to an anti-CXCR3 antibody control level and/or an increased level of a C-X-C chemokine, preferably CXCL9, with respect to a C-X-C chemokine control level, preferably a CXCL9 control level, in a sample of a bodily fluid of said subject.
59. A C-X-C chemokine antagonist, preferably an CXCL9 antagonist, or an anti-C-X-C chemokine antibody, preferably an anti-CXCL9 antibody, for use in the treatment of acute GVHD, preferably high grade acute GVHD, wherein the subject is treated upon detecting a decreased level of anti-CXCR3 antibody with respect to an anti-CXCR3 antibody control level and/or an increased level of a C-X-C chemokine, preferably CXCL9, with respect to a C-X-C chemokine control level, preferably a CXCL9 control level, in a sample of a bodily fluid of said subject.

In the following, the invention is illustrated with references to Examples and Figures which are not intended to limit the scope of the invention.

### EXAMPLES

### Example 1:

Anti-CXCR3 and anti-CXCR4 autoantibodies in serum samples were measured using a sandwich ELISA kit. To this end, microtiter 96-well polystyrene plates were coated with a polypeptide of the sequence of SEQ ID NO:5 (anti-CXCR3 ELISA) or of SEQ ID NO:6 (anti-CXCR4 ELISA) using common techniques (e.g. see for AT₁-Receptor ELISA Giral M, Foucher Y, Dufay A, Van Huyen JP, Renaudin K, Moreau A, Philippe A, Hegner B, Dechend R, Heidecke H, Brouard S, Cesbron A, Castagnet S, Devys A, Soulillou JP, Dragun D. Pretransplant sensitization against angiotensin II type 1 receptor is a risk factor for acute rejection and graft loss. Am J Transplant. 2013 Oct;13(10):2567-76). Conformational epitopes of the receptor were maintained by addition of 1 mM calcium chloride to every buffer.

In order to obtain a standard curve, plates were incubated with test sera from an anti-CXCR3 and anti-CXCR4 autoantibody positive index patient suffering from systemic sclerosis. The ELISA was validated according to the FDA's "Guidance for industry: Bioanalytical method validation". A serum sample from a patient with a systemic sclerosis was used for the standard curve. A 1:100 dilution of the serum sample is defined as 40 units/ml anti-C5a-receptor-antibodies. A 1:100 dilution of a healthy donor (not having an autoimmune disease) served as a negative control. To set a standard for the concentrations of the autoimmune antibodies, two standard curves were generated for the anti-CXCR3 and for the anti-CXCR4. In detail, a serum sample of systemic sclerosis serum sample was diluted (a) 1:50 for standard point 40 Units/ml, (b) 1:100 for standard point 20 Units/ml, (c) 1:200 for standard point 10 Units/ml, (d) 1:400 for standard point 5 Units/ml, (e) 1:800 for standard point 2.5Units/ml, (f) 1:1600 for standard point 1.25 Units/ml. Then the ratio of optical density at 450 nm and 620 nm was determined using the kit and method of above. Each standard point was performed in duplicates. Results are shown in Figure 1 for the anti-CXCR3 ELISA and in Figure 2 for the anti-CXCR4 ELISA.

To maintain the conformational epitopes of the protein/fragment, 1 mM calcium chloride was added to every buffer. Duplicates of a 1:100 dilution of all serum samples were incubated at 4°C for 2 hours. After washing steps using, plates were incubated for 60 minutes with a 1:20.000 dilution of horseradish-peroxidase-labeled goat anti-human-IgG (Cat-No.: 109035008, Jackson, USA) used for detection.

### Example 2:

Anti-C5a-receptor antibody levels in serum samples from healthy subjects and SLE patients.

### 2.1 Cross-section analysis for CXCR3 and CXCR4 antibody

In the following cross-sectional analysis a characterization of each patient was precisely included in the investigation. In the presence of several characterizations the earliest one was the selected.

### 2.2 Baseline Characteristics

The population studied for the cross-sectional analysis included 74 patients with systemic lupus erythematosus. An overview is given in Table 2.

**Table 2: Baseline Characteristics SLE CXCR3-, CXCR4-AB**

| **Characterization** | |
|---|---|
| Gender, N (%) | |
| - Female | 65 (87.8) |
| - Male | 9 (12.2) |
| - Total | 74(100) |
| Age (Years), mean±SD, (range) | 38.20±12.30 (20-69) |
| Disease duration (Years), mean±SD, (range) | 10.07±7.45 (0-28) |
| Ethnic groups N (%) | |
| - Caucasian | 66 (89.2) |
| - Asian | 8(10.8) |
| SLEDAI, mean±SD (range) | 6.55±5.76 (0-21) |
| mSLEDAI-2K, mean±SD (range) | 4.50±4.94 (0-17) |
| BILAG-2004, mean±SD (range) | 8.80±8.60 (0-39) |

### 2.3 Distribution of CXCR3 antibody titer values

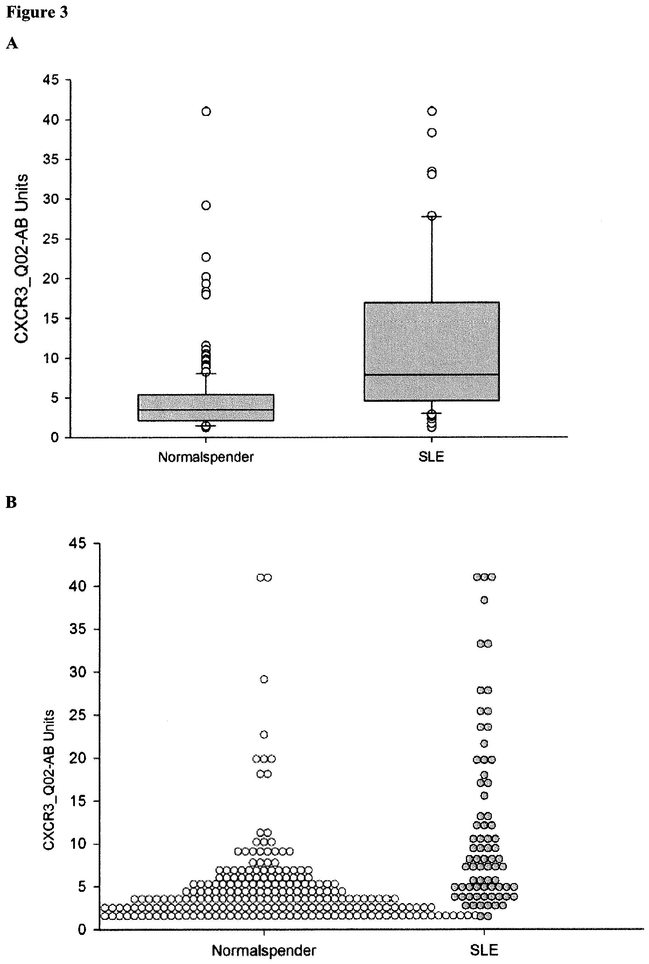

There was no normal distribution of the CXCR3 antibody titers in the studied patient population, as well as in the normal donors group. (Kolmogorov-Smirnov test p = <0.001, Shapiro-Wilk test <0.001). The distribution of titers compared to normal donors is shown in Figure 3. The median was 7.89 Units/ml, the percentile is shown in Table 3.

**Table 3 CXCR3-AB percentile in SLE**

| **Percentile** | **5** | **10** | **25** | **50** | **75** | **90** | **95** |
|---|---|---|---|---|---|---|---|
| CXCR3-AB (units/ml) | 2.56 | 2.99 | 4.60 | 7.89 | 16.92 | 27.81 | 38.98 |

The CXCR3 antibody titers in the examined SLE collective were highly significant increased compared with the healthy controls (median 7.89 vs. 3.45 Units / ml, p <0.001, n = 308). In addition, there was a highly significant association between CXCR3-AB titers of SLE patients and normal donors above the 95^{th} percentile (chi-squared test p <0.001). 11 of 234 healthy controls (4.7%) compared with 26 of 74 patients suffering from SLE patients (35.1%) showed such a higher titer.

### 2.4 Distribution of CXCR4 antibody titer values

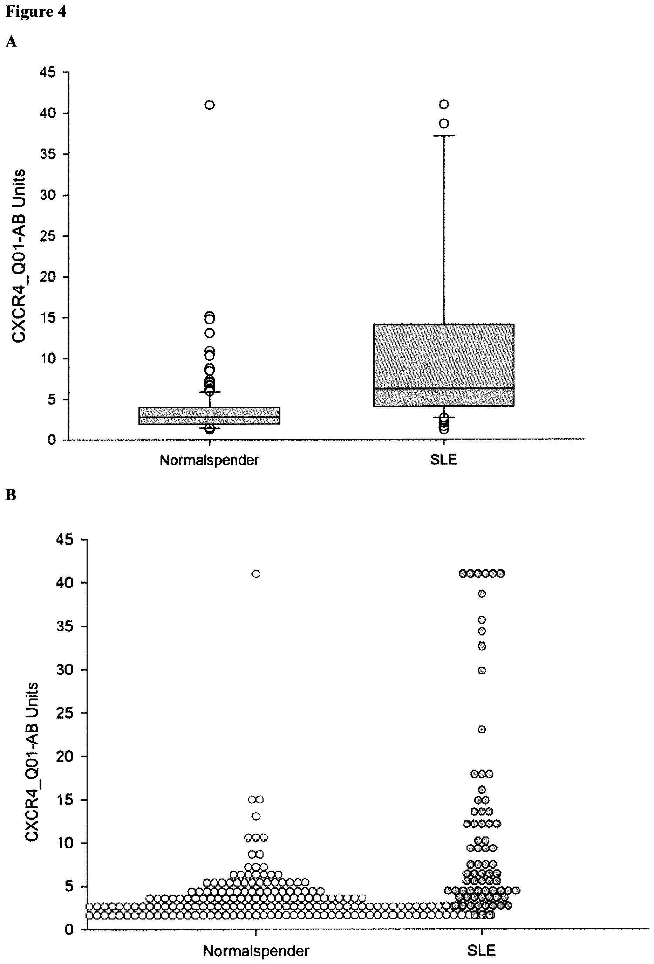

There was no normal distribution of the CXCR4 antibody titers in the studied patient population, as well as in the normal donors group. (Kolmogorov-Smirnov test p = <0.001, Shapiro-Wilk test <0.001). The distribution of titers compared to normal donors is shown in Figure 4. The median was 6.29 Units/ml, the percentile is shown in Table 4.

**Table 4: CXCR4-AB percentile in SLE**

| **Percentile** | **5** | **10** | **25** | **50** | **75** | **90** | **95** |
|---|---|---|---|---|---|---|---|
| CXCR4-AB (units/ml) | 2.20 | 2.67 | 4.12 | 6.29 | 14.10 | 37.16 | > 40.00 |

The anti-CXCR4 antibody titers in the examined SLE collective were highly significant increased compared with the healthy controls (median 6.29 vs. 2.74 Units / ml, p <0.001, n = 272). In addition, there was a highly significant association between anti-CXCR4 titers of SLE patients and normal donors above the 95th percentile (chi-squared test p <0.001). 11 of 234 healthy controls (4.7%) compared with 35 of 74 patients suffering from SLE patients (47%) showed such a higher titer.

### 2.5 Correlation with clinical and laboratory parameters

The following Table 5 provides an overview of cross-correlations with clinical and laboratory parameters examined in the SLE panel for anti-CXCR3 and anti-CXCR4 antibodies. For both antibodies highly significant correlations can be identified. Further evidencing the diagnostic impact of these antibodies

**Table 5: Correlation with clinical and laboratory parameters**

| | | | **CXCR3-AB** | **CXCR4-AB** |
|---|---|---|---|---|
| **General** | | | | |
| | **Age** | SRT-r | -0.142 | -0.108 |
| | | P-Wert | 0.114 | 0.180 |
| | | n | 74 | 74 |
| | **Disease duration** | SRT-r | -0.084 | -0.057 |
| | | P-Wert | 0.241 | 0.318 |
| | | n | 72 | 72 |

| **Blood count, CRP and proteinuria** | | | | |
|---|---|---|---|---|
| | **BSG** | SRT-r | **0.249** | 0.203 |
| | | P-Wert | **0.038** | 0.075 |
| | | n | **52** | 52 |
| | **Hb** | SRT-r | -0.094 | -0.080 |
| | | P-Wert | 0.215 | 0.251 |
| | | n | 73 | 73 |
| | **Leukocytes** | SRT-r | -0.092 | -0.104 |
| | | P-Wert | 0.219 | 0.192 |
| | | n | 73 | 73 |
| | **Neutrophiles** | SRT-r | **-0.406** | -0.335 |
| | | P-Wert | **0.024** | 0.055 |
| | | n | **24** | 24 |
| | **Lymphocytes** | SRT-r | -0.113 | -0.088 |
| | | P-Wert | 0.189 | 0.246 |
| | | n | 63 | 63 |
| | **Monocytes** | SRT-r | 0.191 | **0.234** |
| | | P-Wert | 0.067 | **0.032** |
| | | n | 63 | **63** |
| | **Thrombocytes** | SRT-r | 0.006 | 0.014 |
| | | P-Wert | 0.480 | 0.452 |
| | | n | 72 | 72 |
| | **CRP** | SRT-r | 0.077 | 0.040 |
| | | P-Wert | 0.260 | 0.369 |
| | | n | 72 | 72 |
| | **Proteinuria** | SRT-r | 0.131 | 0.137 |
| | | P-Wert | 0.175 | 0.164 |
| | | n | 53 | 53 |

| **Complement** | | | | |
|---|---|---|---|---|
| | **C3C** | SRT-r | -0.043 | -0.70 |
| | | P-Wert | 0.358 | 0.278 |
| | | n | 74 | 74 |
| | **C1q** | SRT-r | **0.244** | **0.292** |
| | | P-Wert | **0.018** | **0.006** |
| | | n | **74** | **74** |
| | **C4** | SRT-r | -0.157 | -0.154 |
| | | P-Wert | 0.090 | 0.095 |
| | | n | 74 | 74 |

| **Interferon** | | | | |
|---|---|---|---|---|
| | **IFN-α** | SRT-r | 0.183 | **0.204** |
| | | P-Wert | 0.059 | **0.040** |
| | | n | 74 | **74** |
| | **IP10** | SRT-r | **0.283** | **0.305** |
| | | P-Wert | **0.007** | **0.004** |
| | | n | **74** | **74** |
| | **SIGLEC-1** | SRT-r | **0.320** | **0.324** |
| | | P-Wert | **0.003** | **0.002** |
| | | n | **74** | **74** |

| **Auto-Antibody** | | | | |
|---|---|---|---|---|
| | **dsDNA** | SRT-r | **0.480** | **0.490** |
| | | P-Wert | **<0.001** | **<0.001** |
| | | n | **74** | **74** |
| | **dsDNA-RIA** | SRT-r | **0.305** | **0.308** |
| | | P-Wert | **0.004** | **0.004** |
| | | n | **74** | **74** |
| | **dsDNA-NcX** | SRT-r | **0.316** | **0.284** |
| | | P-Wert | **0.003** | **0.007** |
| | | n | **74** | **74** |
| | **CIC** | SRT-r | **0.694** | **0.673** |
| | | P-Wert | **<0.001** | **<0.001** |
| | | n | **26** | **26** |
| | **SSA** | SRT-r | 0.005 | -0.042 |
| | | P-Wert | 0.482 | 0.363 |
| | | n | 74 | 74 |
| | **SSB** | SRT-r | 0.082 | 0.018 |
| | | P-Wert | 0.244 | 0.440 |
| | | n | 74 | 74 |
| | **Ro52** | SRT-r | -0.091 | -0.145 |
| | | P-Wert | 0.221 | 0.109 |
| | | n | 74 | 74 |
| | **NuK** | SRT-r | **0.195** | 0.166 |
| | | P-Wert | **0.048** | 0.079 |
| | | n | **74** | 74 |
| | **Sm** | SRT-r | **0.361** | **0.431** |
| | | P-Wert | **0.001** | **<0.001** |
| | | n | **74** | **74** |
| | **nRNP-SM** | SRT-r | **0.348** | **0.397** |
| | | P-Wert | **0.001** | **<0.001** |
| | | n | **74** | **74** |
| | **RibN** | SRT-r | **0.365** | **0.453** |
| | | P-Wert | **0.001** | **<0.001** |
| | | n | **74** | **74** |
| | **RibP0** | SRT-r | **0.194** | **0.244** |
| | | P-Wert | **0.049** | **0.018** |
| | | n | **74** | **74** |
| | **RibP1** | SRT-r | **0.352** | **0.442** |
| | | P-Wert | **0.001** | **<0.001** |
| | | n | **74** | **74** |
| | **RibP2** | SRT-r | 0.066 | 0.143 |
| | | P-Wert | 0.288 | 0.113 |
| | | n | 74 | 74 |

### 2.6 Correlation with the disease activity

The disease activity was assessed by the SLEDAI, the mSLEDAI and BILAG-2004 scores.

**Table 6 shows a cross-sectional correlation of scores evaluated with the CXCR3 and CXCR4 antibodies:**

| | | **CXCR3-AB** | **CXCR4-AB** |
|---|---|---|---|
| **SLEDAI** | SRT-r | **0.361** | **0.356** |
| | P-value | **0.001** | **0.001** |
| | n | **74** | **74** |
| **mSLEDAI** | SRT-r | **0.303** | **0.313** |
| | P-value | **0.004** | **0.003** |
| | n | **74** | **74** |
| **BILAG-2004** | SRT-r | **0.227** | **0.245** |
| | P-value | **0.026** | **0.018** |
| | n | **74** | **74** |

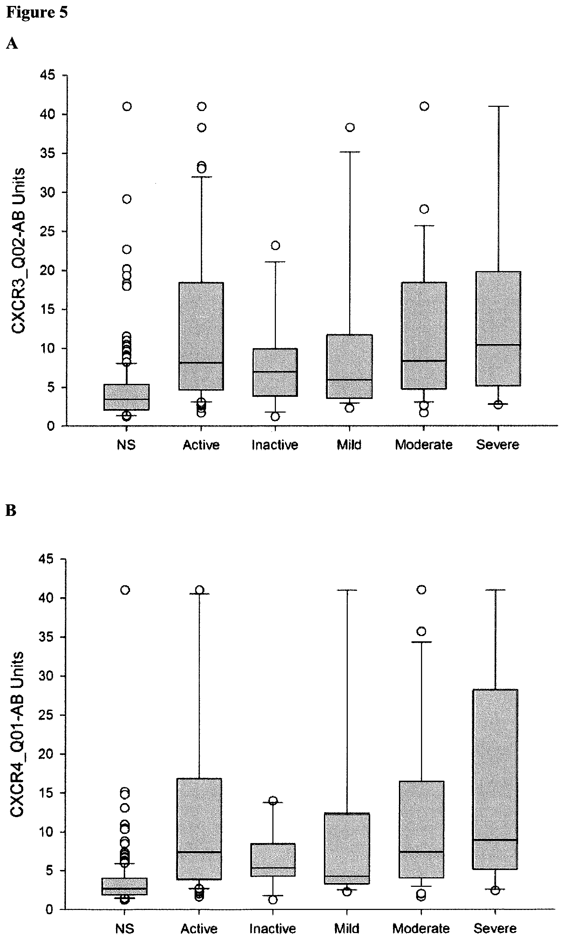

The correlation of antibody levels with BELAG scoring is depicted in Figure 5 for anti-CXCR3 (A) and anti-CXCR4 (B) antibodies.

### 2.7 Summary

The data clearly show that anti-CXCR antibody titers in patient samples are a potent tool for diagnosing an autoimmune disease. Further, they show that the activity of the autoimmune disease correlates with the level of the antibodies.

### Example 3:

### Patients

Patients were eligible for this study if they had undergone alloSCT at University Hospital Heidelberg between 2002 and 2013 and had blood samples available for anti-CXCR3/MIG measurement (collected directly before start of conditioning chemotherapy prior to alloSCT and at onset of acute GVHD grade 3-4). Written informed consent to sample and data collection according to the declaration of Helsinki was obtained in all eligible patients and sample and data collection was approved by our local Ethics Committee.

### GVHD prophylaxis, treatment and supportive care

GVHD prophylaxis was performed as previously described^{1, 2} and remained unchanged over the whole study period. GVHD was clinically and histologically diagnosed and graded using standard criteria. Treatment of histologically confirmed GVHD was performed as reported previously¹⁻³.

### Statistical analysis

Categorical and continuous variables of patient characteristics were compared using χ² test and the Mann-Whitney test, respectively. Overall survival (OS) and incidences of relapse and NRM were calculated from date of alloSCT to the appropriate endpoint. NRM was calculated as the time from alloSCT to death in the absence of relapse considering recurrence of the underlying malignancy as competing event. NRM after GVHD onset was calculated as the time from GVHD onset to death in the absence of relapse, again, considering recurrence of the underlying malignancy as competing event. NRM of different groups of patients was compared with the Grays test⁴⁻⁶. Cox proportional hazard regression models were performed for multivariable analysis of predictors of refractory GVHD and multivariable competing risk proportional subdistribution hazards regression models were set up to adjust for the impact of potential confounders of the effect of CXCL9 and anti-CXCR3 on NRM incidence, relapse incidence and overall survival (OS) after alloSCT^{4,6}

Calculations were done using the statistical software environment R. version 3.2.3 BiocStyle_1.6.0, maxstat_0.7-23, knitr_1.12.3, survplot_0.0.7, cmprsk_2.2-7, survival_2.38-3. All statistical tests were two-sided. Hazard ratios (HR) were estimated with 95% confidence interval (95%CI). Results with P values smaller than 5% were considered to be statistically significant.

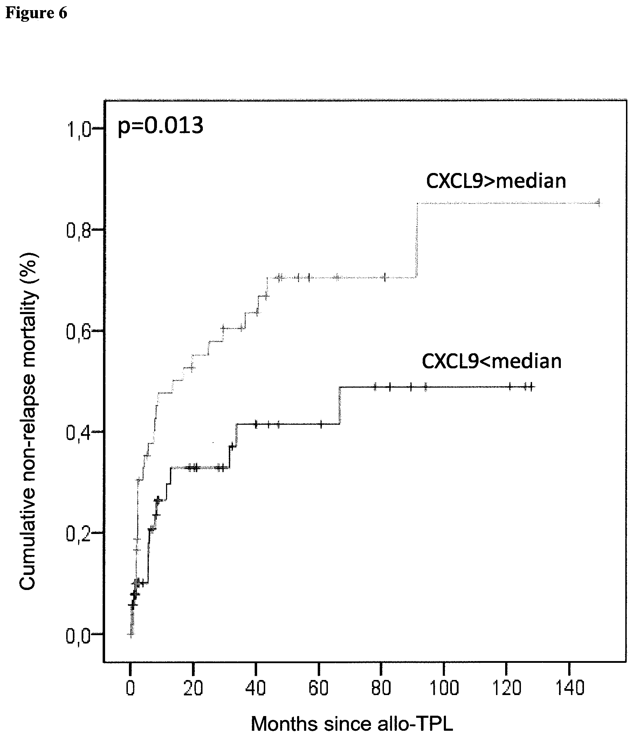

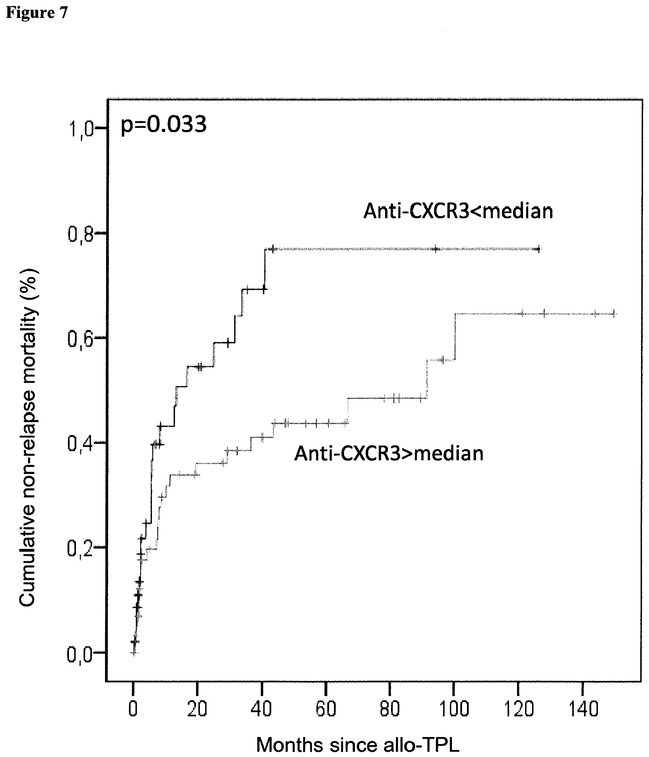

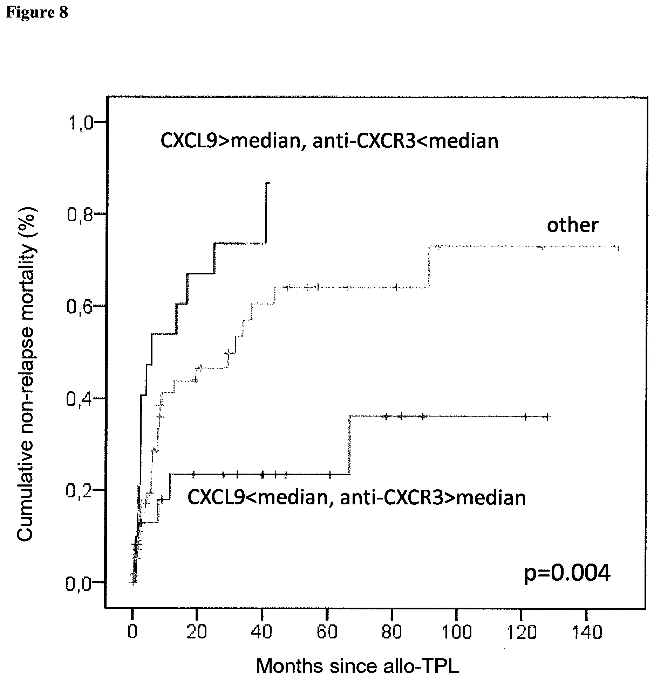

Results are shown in Figures 6 to 8.

### References for Example 3

1. Dietrich S, Falk CS, Benner A, Karamustafa S, Hahn E, Andrulis M et al. Endothelial vulnerability and endothelial damage are associated with risk of graft-versus-host disease and response to steroid treatment. Biol Blood Marrow Transplant 2013; 19(1): 22-27. e-pub ahead of print 2012/10/09; doi: 10.1016/j.bbmt.2012.09.018 S1083-8791(12)00396-5 [pii]
2. Luft T, Dietrich S, Falk C, Conzelmann M, Hess M, Benner A et al. Steroid-refractory GVHD: T-cell attack within a vulnerable endothelial system. Blood 2011; 118(6): 1685-1692. e-pub ahead of print 2011/06/04; doi: 10.1182/blood-2011-02-334821 blood-2011-02-334821 [pii]
3. Dietrich S, Okun JG, Schmidt K, Falk CS, Wagner AH, Karamustafa S et al. High pretransplant serum nitrate levels predict risk of acute steroid-refractory graft-versus-host disease in the absence of statins. Haematologica 2013. doi: 10.3324/haematol.2013.090209
4. Gray R. A Class of K-Sample Tests for Comparing the Cumulative Incidence of a Competing Risk. The Annals of Statistics. 1988; 16(3): 1141-1154.;
5. Scrucca L, Santucci A, Aversa F. Competing risk analysis using R: an easy guide for clinicians. Bone Marrow Transplant 2007; 40(4): 381-387. e-pub ahead of print 2007/06/15; doi: 1705727 [pii] 10.1038/sj.bmt.1705727
6. Scrucca L, Santucci A, Aversa F. Regression modeling of competing risk using R: an in depth guide for clinicians. Bone Marrow Transplant 2010; 45(9): 1388-1395. e-pub ahead of print 2010/01/12; doi: bmt2009359 [pii] 10.1038/bmt.2009.359

### Disclosed Sequences:

SEQ ID NO: 1: CXCR3-A (also referred to as CXCR3 isoform 1)
(NCBI-Database as of February 13, 2015:
NP_001136269)

1 MVLEVSDHQV LNDAEVAALL ENFSSSYDYG ENESDSCCTS PPCPQDFSLN
51 FDRAFLPALY SLLFLLGLLG NGAVAAVLLS RRTALSSTDT FLLHLAVADT
101 LLVLTLPLWA VDAAVQWVFG SGLCKVAGAL FNINFYAGAL LLACISFDRY
151 LNIVHATQLY RRGPPARVTL TCLAVWGLCL LFALPDFIFL SAHHDERLNA
201 THCQYNFPQV GRTALRVLQL VAGFLLPLLV MAYCYAHILA VLLVSRGQRR
251 LRAMRLVVVV VVAFALCWTP YHLVVLVDIL MDLGALARNC GRESRVDVAK
301 SVTSGLGYMH CCLNPLLYAF VGVKFRERMW MLLLRLGCPN QRGLQRQPSS
351 SRRDSSWSET SEASYSGL

SEQ ID NO: 2: CXCR3-B (also referred to as CXCR3 isoform 2)
(NCBI-Database as of February 13, 2015: NP_001495)

1 MELRKYGPGR LAGTVIGGAA QSKSQTKSDS ITKEFLPGLY TAPSSPFPPS
51 QVSDHQVLND AEVAALLENF SSSYDYGENE SDSCCTSPPC PQDFSLNFDR
101 AFLPALYSLL FLLGLLGNGA VAAVLLSRRT ALSSTDTFLL HLAVADTLLV
151 LTLPLWAVDA AVQWVFGSGL CKVAGALFNI NFYAGALLLA CISFDRYLNI
201 VHATQLYRRG PPARVTLTCL AVWGLCLLFA LPDFIFLSAH HDERLNATHC
251 QYNFPQVGRT ALRVLQLVAG FLLPLLVMAY CYAHILAVLL VSRGQRRLRA
301 MRLVVVVVVA FALCWTPYHL VVLVDILMDL GALARNCGRE SRVDVAKSVT
351 SGLGYMHCCL NPLLYAFVGV KFRERMWMLL LRLGCPNQRG LQRQPSSSRR
401 DSSWSETSEA SYSGL

SEQ ID NO: 3: CXCR4a (isoform a; NCBI-Database as of February
13, 2015: NP_001008540)

1 MSIPLPLLQI YTSDNYTEEM GSGDYDSMKE PCFREENANF NKIFLPTIYS
51 IIFLTGIVGN GLVILVMGYQ KKLRSMTDKY RLHLSVADLL FVITLPFWAV
101 DAVANWYFGN FLCKAVHVIY TVNLYSSVLI LAFISLDRYL AIVHATNSQR
151 PRKLLAEKVV YVGVWIPALL LTIPDFIFAN VSEADDRYIC DRFYPNDLWV
201 VVFQFQHIMV GLILPGIVIL SCYCIIISKL SHSKGHQKRK ALKTTVILIL
251 AFFACWLPYY IGISIDSFIL LEIIKQGCEF ENTVHKWISI TEALAFFHCC
301 LNPILYAFLG AKFKTSAQHA LTSVSRGSSL KILSKGKRGG HSSVSTESES
351 SSFHSS

SEQ ID NO: 4: CXCR4b (isoform b; NCBI-Database as of January 5,
2016: NP_003458)

1 MEGISIYTSD NYTEEMGSGD YDSMKEPCFR EENANFNKIF LPTIYSIIFL
51 TGIVGNGLVI LVMGYQKKLR SMTDKYRLHL SVADLLFVIT LPFWAVDAVA
101 NWYFGNFLCK AVHVIYTVNL YSSVLILAFI SLDRYLAIVH ATNSQRPRKL
151 LAEKVVYVGV WIPALLLTIP DFIFANVSEA DDRYICDRFY PNDLWVVVFQ
201 FQHIMVGLIL PGIVILSCYC IIISKLSHSK GHQKRKALKT TVILILAFFA
251 CWLPYYIGIS IDSFILLEII KQGCEFENTV HKWISITEAL AFFHCCLNPI
301 LYAFLGAKFK TSAQHALTSV SRGSSLKILS KGKRGGHSSV STESESSSFH
351 SS

SEQ ID NO:5: preferred antigenic fragment of CXCR3-A
(SEQ ID NO:1)
1 SGLCKVAGAL FNINFYAGAL LLACISFDRY LNIVHATQLY RRGPPARVTL
51 TCLAVBGLCL LFALPDFIFL SAHHDERLNA THCQYNFPQV GRTALRVLQL

**SEQ ID NO:6: preferred antigenic fragment of CXCR4b (SEQ ID NO:4)**
1 MEGISIYTSD NYTEEMGSGD YDSMKEPCFR EENANFNKIF LPTIYS

SEQ ID NO:7: CXCL4
1 MSSAAGFCAS RPGLLFLGLL LLPLVVAFAS AEAEEDGDLQ CLCVKTTSQV
51 RPRHITSLEV IKAGPHCPTA QLIATLKNGR KICLDLQAPL YKKIIKKLLE
101 S

SEQ ID NO:8: CXCL9
1 MKKSGVLFLL GIILLVLIGV QGTPVVRKGR CSCISTNQGT IHLQSLKDLK
51 QFAPSPSCEK IEIIATLKNG VQTCLNPDSA DVKELIKKWE KQVSQKKKQK
101 NGKKHQKKKV LKVRKSQRSR QKKTT

SEQ ID NO:9: CXCL10
1 MNQTAILICC LIFLTLSGIQ GVPLSRTVRC TCISISNQPV NPRSLEKLEI
51 IPASQFCPRV EIIATMKKKG EKRCLNPESK AIKNLLKAVS KERSKRSP

SEQ ID NO:10: CXCL11 (isoform 1)
1 MSVKGMAIAL AVILCATVVQ GFPMFKRGRC LCIGPGVKAV KVADIEKASI
51 MYPSNNCDKI EVIITLKENK GQRCLNPKSK QARLIIKKVE RKNF

SEQ ID NO:11: CXCL11 (isoform 2)
1 MSVKGMAIAL AVILCATVVQ GFPMFKRGRC LCIGPGVKAV KVADIEKASI
51 MYPSNNCDKI EVIITLKENK GQRCLNPKSK QARLIIKLKE RIFKNIKTYE
101 VLEKSI

All references cited herein are hereby incorporated by reference in their entirety.

## Claims

1. A method for the prognosis of acute graft-versus host-disease (GVHD) in a subject comprising the determination of the level of an antibody against the C-X-C chemokine receptor type 3 (CXCR3) and/or the level of a C-X-C chemokine selected from the group consisting of CXCL4, CXCL9, CXCL10 and CXCL11, preferably CXCL9, in a sample of a bodily fluid of said subject.

2. A method for the prognosis of acute graft-versus host-disease (GVHD) in a subject, wherein said method comprises
(a)
(i) determining the level of an antibody against the C-X-C chemokine receptor type 3 (CXCR3) in a sample of a bodily fluid of said subject and
(ii) comparing the determined level of the anti-CXCR3 antibody to a control level of anti-CXCR3 antibody, and/or
(b)
(i) determining the level of a C-X-C chemokine selected from the group consisting of CXCL4, CXCL9, CXCL10 and CXCL11, preferably CXCL9, in a sample of a bodily fluid of said subject and
(ii) comparing the determined level of said C-X-C chemokine to a control level of said C-X-C chemokine,
wherein a decreased level of said anti-CXCR3 antibody with respect to the anti-CXCR3 antibody control level and/or an increased level of said C-X-C chemokine with respect to the C-X-C chemokine control level in said sample is indicative for an adverse outcome for said subject.

3. The method of claim 1, wherein the adverse outcome is a high mortality rate.

4. The method of claim 1 to 3, wherein the subject suffers from high grade acute GVHD

5. The method of claim 2 to 4, wherein said control level(s) is/are derived from a subject or a group of subjects not having acute GVHD, preferably a subject or a group of subjects not having high grade acute GVHD

6. The method of claim 2 to 4, wherein the control level of the anti-CXCR3 antibody and/or the control level of the C-X-C chemokine is/are
a) a level derived from subjects prior to allogeneic stem cell transplantation, or
b) a level on day 50 after allogeneic stem cell transplantation.

7. The method according to claims 2 to 4, wherein a level of said anti-CXCR3 antibody in the sample of said subject of less than 0.9-fold, preferably less than 0.8-fold, more preferably less than 0.5-fold, as compared to the control level is indicative of a negative outcome.

8. The method according to claims 2 to 4, wherein a level of said C-X-C chemokine, preferably CXCL9, in the sample of said subject of more than 1.5-fold, preferably more than 1.8-fold, more preferably more than 2.0-fold, as compared to the control level is indicative of a negative outcome.

9. Use of CXCR3 or an immunogenic peptide thereof for the prognosis of acute GVHD, preferably high grade acute GVHD

10. Use of an anti-C-X-C chemokine antibody, preferably an anti-CXCL9 antibody, for the prognosis of acute GVHD, preferably high grade acute GVHD

11. An immunosuppressant for use in the treatment of acute GVHD, preferably high grade acute GVHD, wherein the subject is treated upon detecting a decreased level of anti-CXCR3 antibody with respect to an anti-CXCR3 antibody control level and/or an increased level of a C-X-C chemokine, preferably CXCL9, with respect to a C-X-C chemokine control level, preferably a CXCL9 control level, in a sample of a bodily fluid of said subject.

12. The immunosuppressant for use according to claim 11, wherein the immunosuppressant is prednisolone, mycofenolate mofetil or a calcineurin inhibitor such as ciclosporin or tacrolimus.

13. The immunosuppressant for use according to claim 11 or 12, wherein the immunosuppressant is combined with a statin such as pravastatin.

14. A CXCR3 antagonist, preferably an anti-CXCR3 antibody, for use in the treatment of acute GVHD, preferably high grade acute GVHD, wherein the subject is treated upon detecting a decreased level of anti-CXCR3 antibody with respect to an anti-CXCR3 antibody control level and/or an increased level of a C-X-C chemokine, preferably CXCL9, with respect to a C-X-C chemokine control level, preferably a CXCL9 control level, in a sample of a bodily fluid of said subject.

15. A C-X-C chemokine antagonist, preferably an CXCL9 antagonist, or an anti-C-X-C chemokine antibody, preferably an anti-CXCL9 antibody, for use in the treatment of acute GVHD, preferably high grade acute GVHD, wherein the subject is treated upon detecting a decreased level of anti-CXCR3 antibody with respect to an anti-CXCR3 antibody control level and/or an increased level of a C-X-C chemokine, preferably CXCL9, with respect to a C-X-C chemokine control level, preferably a CXCL9 control level, in a sample of a bodily fluid of said subject.
